# EUROPÄISCHE PATENTSCHRIFT

(11) **EP 0 949 695 B1**
(45) Veröffentlichungstag und Bekanntmachung des Hinweises auf die Patenterteilung: **17.06.2009**
(21) Anmeldenummer: 99104958.6
(22) Anmeldetag: 12.03.1999
(51) Int. Cl.: H01L 51/54, C08G 61/12

(54) **Elektrolumineszierende Anordnungen unter Verwendung von Blendsystemen**
Electroluminescent structure using blend systems
Structure électroluminescente utilisant des systèmes de mélanges

(30) Priorität: 20.03.1998 DE 19812258
(43) Veröffentlichungstag der Anmeldung: 13.10.1999
(73) Patentinhaber: H.C. Starck GmbH, 38642 Goslar (DE)
(72) Erfinder: Wehrmann, Rolf, Dr., 47800 Krefeld (DE); Heuer, Helmut Werner, Dr., 47829 Krefeld (DE); Jonas, Friedrich, Dr., 52006 Aachen (DE); Elschner, Andreas, Dr., 45479 Mülheim (DE); Mayer, Andrea, Dr., 70197 Stuttgart (DE); Hüppauff, Martin, Dr., 70563 Stuttgart (DE); Andries, Hartwig, 9150 Rupelmonde (BE)
(74) Vertreter: Clauswitz, Kai-Uwe Wolfram

(56) Entgegenhaltungen:
- EP-A- 0 440 957
- EP-A- 0 686 662
- WO-A-98/02018
- DE-A- 19 627 070
- DE-A- 19 627 071
- ADACHI C. ET AL: "CONFINEMENT OF CHARGE CARRIERS AND MOLECULAR EXCITONS WITHIN 5-NM-THICK EMITTER LAYER IN ORGANIC ELECTROLUMINESCENT DEVICES WITH A DOUBLE HETEROSTRUCTURE" APPLIED PHYSICS LETTERS, Bd. 57, 6. August 1990 (1990-08-06), Seiten 531-533,

## Beschreibung

Eine elektrolumineszierende (EL) Anordnung ist dadurch charakterisiert, daß sie unter Anlegung einer elektrischen Spannung unter Stromfluß Licht aussendet. Derartige Anordnungen sind unter der Bezeichnung "Leuchtdioden" (LEDs = light emitting diodes) seit langem in der Technik bekannt. Die Emission von Licht kommt dadurch zustande, daß positive Ladungen ("Löcher", holes) und negative Ladungen ("Elektronen", electrons) unter Aussendung von Licht rekombinieren.

Bei der Entwicklung lichtemittierender Bauteile für Elektronik oder Photonik kommen heute hauptsächlich anorganische Halbleiter, wie Galliumarsenid, zum Einsatz.
Auf Basis derartiger Substanzen können punktförmige Anzeigeelemente hergestellt werden. Großflächige Anordnungen sind nicht möglich.

Neben den Halbleiterleuchtdioden sind elektrolumineszierende Anordnungen auf Basis aufgedampfter niedermolekularer organischer Verbindungen bekannt (US-A 4 539 507, US-A 4 769 262, US-A 5 077 142, EP-A 406 762, EP-A 278 758, EP-A 278 757).

Weiterhin werden Polymere, wie Poly-(p-phenylene) und Poly-(p-phenylenvinylene (PPV)) als elektrolumineszierende Polymere beschrieben: G. Leising et al., Adv. Mater. 4 (1992) No. 1; Friend et al., J. Chem. Soc., Chem. Commun. 32 (1992); Saito et al., Polymer, 1990, Vol. 31, 1137; Friend et al., Physical Review B, Vol. 42, No. 18, 11670 oder WO-A 90/13148. Weitere Beispiele für PPV in Elektrolumineszenzanzeigen werden in EP-A 443 861, WO-A 92/03490 und WO-A 92/003491 beschrieben.

EP-A 0 294 061 stellt einen optischen Modulator auf Basis von Polyacetylen vor.

Zur Herstellung flexibler Polymer-LEDs haben Heeger at al. lösliche konjugierte PPV-Derivate vorgeschlagen (WO-A 92/16023).

Polymerblends unterschiedlicher Zusammensetzung sind ebenfalls bekannt: M. Stolka et al., Pure & Appt. Chem., Vol. 67, No. 1, pp 175-182, 1995; H. Bässler et al., Adv. Mater. 1995, 7, No. 6, 551; K. Nagai et al., Appl. Phys. Lett. 67 (16), 1995, 2281; EP-A 532 798.

Die organischen EL-Anordnungen enthalten in der Regel eine oder mehrere Schichten aus organischen Ladungstransportverbindungen. Der prinzipielle Aufbau in der Reihenfolge der Schichten ist wie folgt:
- 1: Träger, Substrat
- 2: Basiselektrode / Anode
- 3: Löcher-injizierende Schicht
- 4: Löcher-transportierende Schicht
- 5: Licht-emittierende Schicht
- 6: Elektronen-transportierende Schicht
- 7: Elektronen-injizierende Schicht
- 8: Topelektrode / Kathode
- 9: Kontakte
- 10: Umhüllung, Verkapselung.

Die Schichten 3 bis 7 stellen das elektrolumineszierende Element dar.

Dieser Aufbau stellt den allgemeinsten Fall dar und kann vereinfacht werden, indem einzelne Schichten weggelassen werden, so daß eine Schicht mehrere Aufgaben übernimmt. Im einfachsten Fall besteht eine EL-Anordung aus zwei Elektroden, zwischen denen sich eine organische Schicht befindet, die alle Funktionen - inklusive der der Emission von Licht - erfüllt. Derartige Systeme sind z.B. in der Anmeldung WO-A 90/13148 auf der Basis von Poly-(p-phenylenvinylen) beschrieben.

DE 19627070 beschreibt elektrolumineszierende Anordnungen, aufgebaut aus einem Substrat, einer Anode, einem elektrolumineszierenden Element und einer Kathode, wobei wenigstens eine der beiden Elektroden im sichtbaren Spektralbereich transparent ist, und das elektrolumineszierende Element eine oder mehrere Zonen aus der Gruppe der lochinjizierenden Zone, der lochtransportierenden Zone, der elektrolumineszierenden Zone, der elektronentransportierenden Zone und der elektroneninjizierenden Zone in der genannten Reihenfolge enthält, wobei jede der vorhandenen Zonen auch die Funktion der anderen Zonen übernehmen kann. Die lochtransportierende Zone stellt eine gegebenenfalls substituierte Tris-1,3,5-(aminophenyl)verbindung A dar. Auch in der EP 0686662 werden solche elektrolumineszierende Anordnungen offenbart, welche als lochtransportierende Zone ebenfalls eine einzige aromatische Verbindung enthalten.

Der Aufbau von Mehrschichtsystemen kann durch Aufdampfverfahren, bei denen die Schichten sukzessive aus der Gasphase aufgebracht werden oder durch Gießverfahren erfolgen. Gießverfahren sind aufgrund der höheren Prozeßgeschwindigkeiten bevorzugt. Allerdings kann der Anlöseprozeß einer bereits aufgebrachten Schicht beim Überschichten mit der nächsten Schicht in bestimmten Fällen eine Schwierigkeit darstellen.

Die Aufgabe der vorliegenden Erfindung ist die Bereitstellung von elektrolumineszierenden Anordnungen mit hoher Leuchtdichte, wobei die aufzubringende Mischung gießbar aufgebracht werden kann.

Es wurde gefunden, daß elektrolumineszierende Anordnungen, die untengenanntes Blendsystem enthalten, diese Anforderungen erfüllen. Im folgenden ist der Begriff Zone auch mit Schicht gleichzusetzen.

Gegenstand der vorliegenden Erfindung sind daher elektrolumineszierende Anordnungen, enthaltend ein Substrat, eine Anode, ein elektrolumineszierendes Element und eine Kathode, wobei wenigstens eine der beiden Elektroden im sichtbaren Spektralbereich transparent bzw. semitransparent ist und das elektrolumineszierende Element eine lochinjizierende Zone, eine lochtransportierende Zone, eine elektrolumineszierende Zone, und gegebenenfalls eine elektronentransportierende Zone und/oder eine elektroneninjizierende Zone in der genannten Reihenfolge enthält, dadurch gekennzeichnet, daß die lochinjizierende Zone ein neutrales oder kationisches Polythiophen der Formeln (Ia) und/oder (Ib) enthält 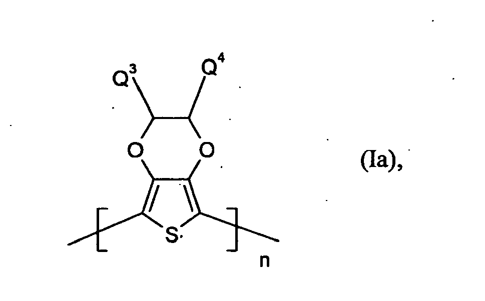 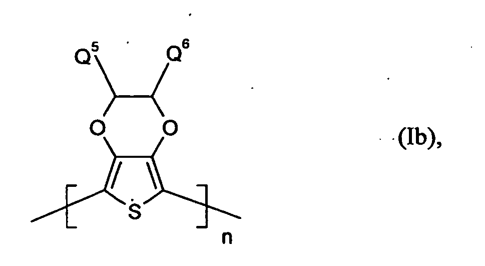 worin
Q³ und Q⁴ unabhängig voneinander für Wasserstoff, gegebenenfalls substituiertes (C₁-C₁₈)-Alkyl, vorzugsweise (C₁-C₁₀)-, insbesondere (C₁-C₆)-Alkyl, (C₂-C₁₂)-Alkenyl, vorzugsweise (C₂-C₈)-Alkenyl, (C₃-C₇)-Cycloalkyl, vorzugsweise Cyclopentyl, Cyclohexyl, (C₇-C₁₅)-Aralkyl, vorzugsweise Phenyl-(C₁-weise Cyclopentyl, Cyclohexyl, (C₇-C₁₅)-Aralkyl, vorzugsweise Phenyl-(C₁-C₄)-alkyl, (C₆-C₁₀)-Aryl, vorzugsweise Phenyl, Naphthyl, (C₁-C₁₈)-Alkoxy, vorzugsweise (C₁-C₁₀)-Alkoxy, beispielsweise Methoxy, Ethoxy, n-oder iso-Propoxy oder (C₂-C₁₈)-Alkyloxyester steht und
Q⁵ und Q⁶ unabhängig voneinander für Wasserstoff, jedoch nicht beide gleichzeitig, mit jeweils mindestens einer Sulfonatgruppe substituiertes (C₁-C₁₈)-Alkyl, vorzugsweise (C₁-C₁₀)-, insbesondere (C₁-C₆)-Alkyl, (C₂-C₁₂)-Alkenyl, vorzugsweise (C₂-C₈)-Alkenyl, (C₃-C₇)-Cycloalkyl, vorzugsweise Cyclopentyl, Cyclohexyl, (C₇-C₁₅)-Aralkyl, vorzugsweise Phenyl-(C₁-C₄)-allyl, (C₆-C₁₀)-Aryl, vorzugweise Phenyl, Naphthyl, (C₁-C₁₈)-Alkoxy, vorzugsweise (C₁-C₁₀)-Alkoxy, beispielsweise Methoxy, Ethoxy, n-oder iso-Propoxy oder (C₂-C₁₈)-Alkyloxyester steht, wobei falls Q⁵ für Wasserstoff steht, Q⁶ verschieden von Wasserstoff ist und umgekehrt,
- n: für eine ganze Zahl von 2 bis 10 000, vorzugsweise 5 bis 5 000 steht,
und die an die lochinjizierende Zone angrenzende lochleitende Zone mehrere aromatische Aminverbindungen enthält, wobei als aromatische Aminverbindung mindestens eine Aminverbindung A ausgewählt aus der allgemeinen Formel (II) enthalten ist 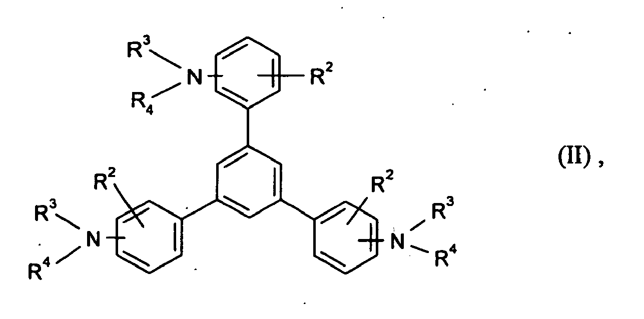
- R²: für Wasserstoff gegebenenfalls substituiertes Alkyl oder Halogen steht,
- R³: und R⁴ unabhängig voneinander für gegebenenfalls substituiertes (C₁-C₁₀)-Alkyl, Alkoxycarbonyl-substituiertes (C₁-C₁₀)-Alkyl, jeweils gegebenenfalls substituiertes Aryl, Aralkyl oder Cycloalkyl stehen.

R³ und R⁴ stehen unabhängig voneinander bevorzugt für (C₁-C₆)-Alkyl, insbesondere Methyl, Ethyl, n- oder iso-Propyl, n-, iso-, sec.- oder tert.-Butyl, (C₁-C₄)-Alkoxycarbonyl-(C₁-C₆)alkyl, wie beispielsweise Methoxy-, Ethoxy-, Propoxy-, Butoxycarbonyl-(C₁-C₄)alkyl, jeweils gegebenenfalls durch (C₁-C₄)-Alkyl und/oder (C₁-C₄)-Alkoxy substituiertes Phenyl(C₁-C₄)-alkyl, Naphthyl-(C₁-C₄)-alkyl, Cyclopentyl, Cyclohexyl, Phenyl, Naphthyl oder Anthracyl.

Besonders bevorzugt stehen R³ und R⁴ unabhängig voneinander für unsubstituiertes Phenyl oder Naphthyl oder jeweils einfach bis dreifach durch Methyl, Ethyl, n-, iso-Propyl, Methoxy, Ethoxy, n- und/oder iso-Propoxy substituiertes Phenyl oder Naphthyl.

R² steht vorzugsweise für Wasserstoff, (C₁-C₆)-Alkyl, wie beispielsweise Methyl, Ethyl, n- oder iso-Propyl, n-, iso-, sec.- oder tert.-Butyl, oder Halogen.

Derartige Verbindungen und deren Herstellung sind in US-A 4 923 774 für den Einsatz in der Elektrophotographie beschrieben, welche hiermit ausdrücklich als Bestandteil der Beschreibung aufgenommen wird ("incorporated by reference"). Die Tris-Nitrophenyl-Verbindung kann beispielsweise durch allgemein bekannte katalytische Hydrierung beispielweise in Gegenwart von Raney-Nickel in die Trisaminophenyl-Verbindung überführt werden (Houben-Weyl 4/1C, 14-102, UMmann (4) 13, 135-148). Die Aminoverbindung wird in allgemein bekannter Weise mit substituierten Halogenbenzolen umgesetzt.

Beispielhaft seien die folgenden Verbindungen genannt, wobei die Substitution am Phenylring sowohl in ortho, meta und/oder para zum Aminstickstoff erfolgen kann:

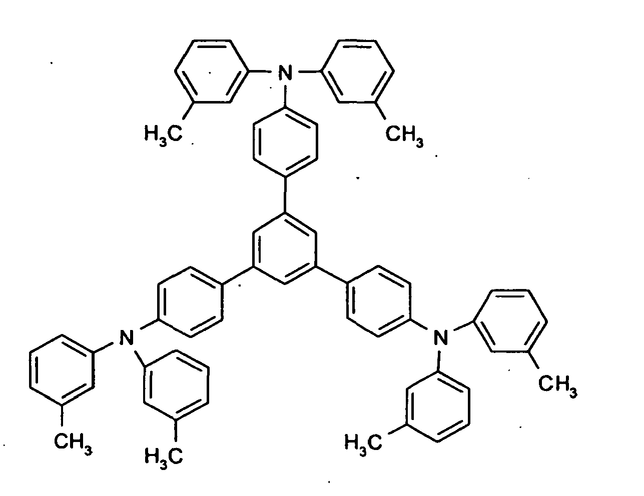

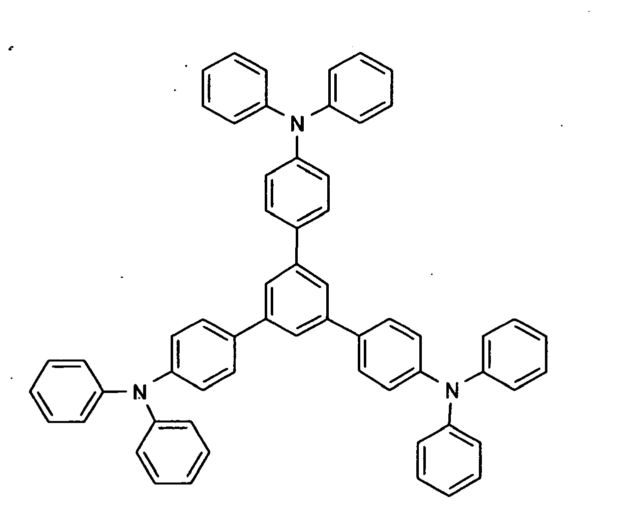

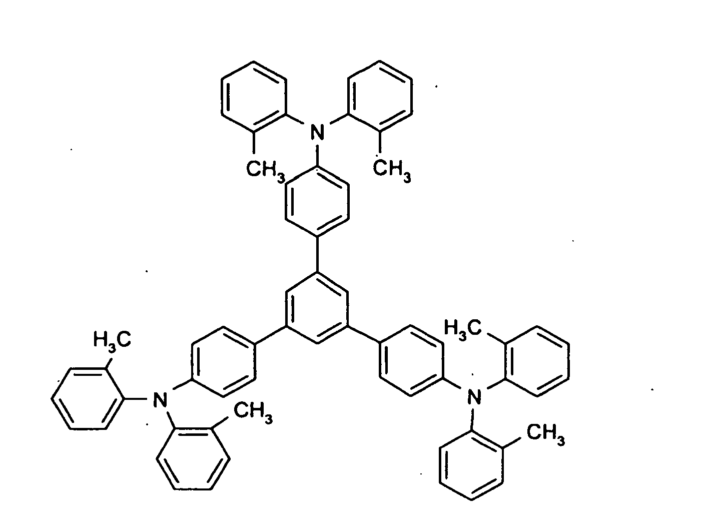

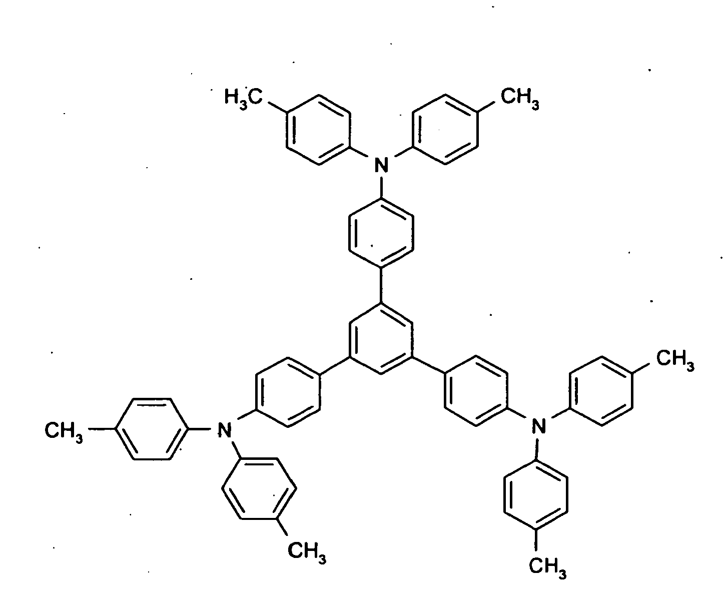

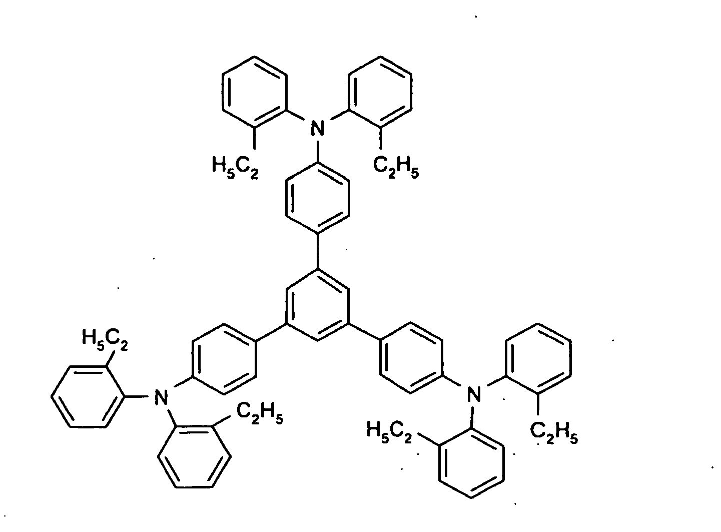

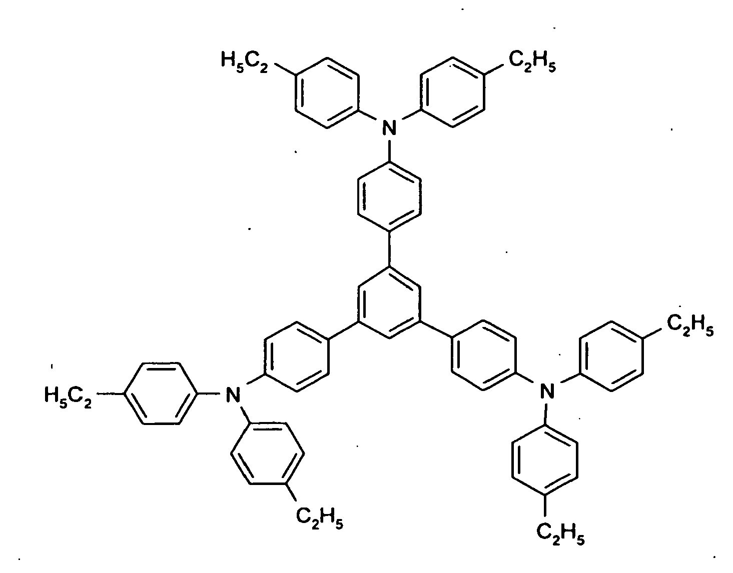

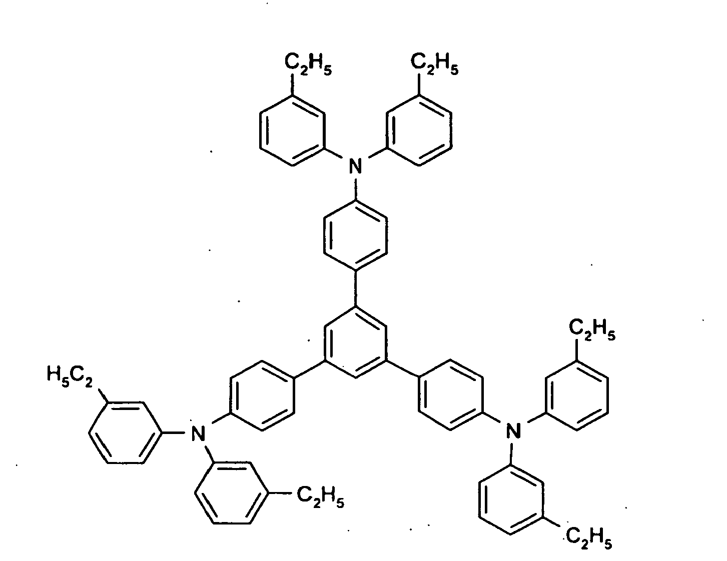

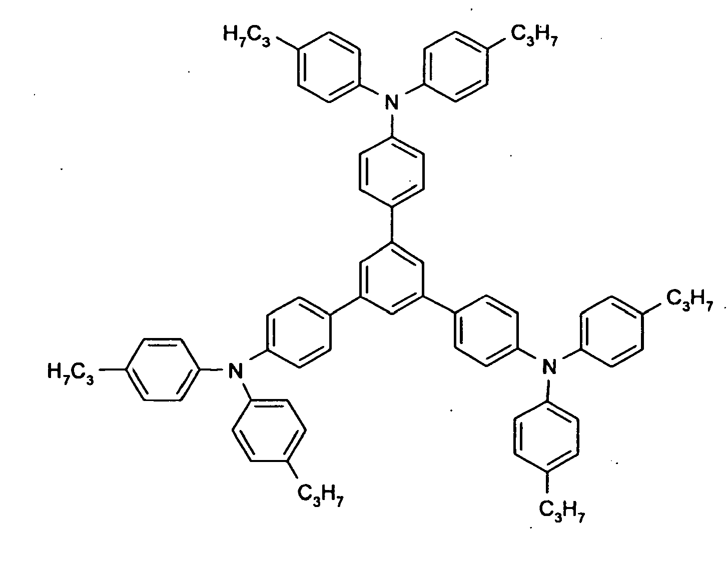

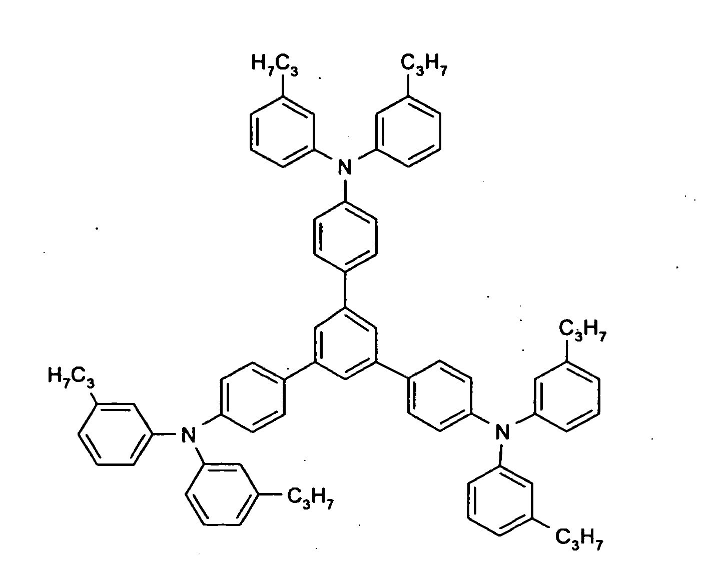

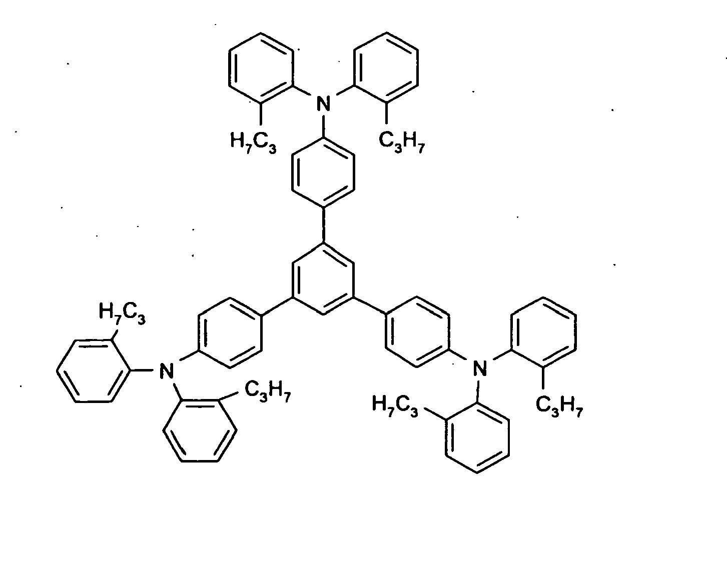

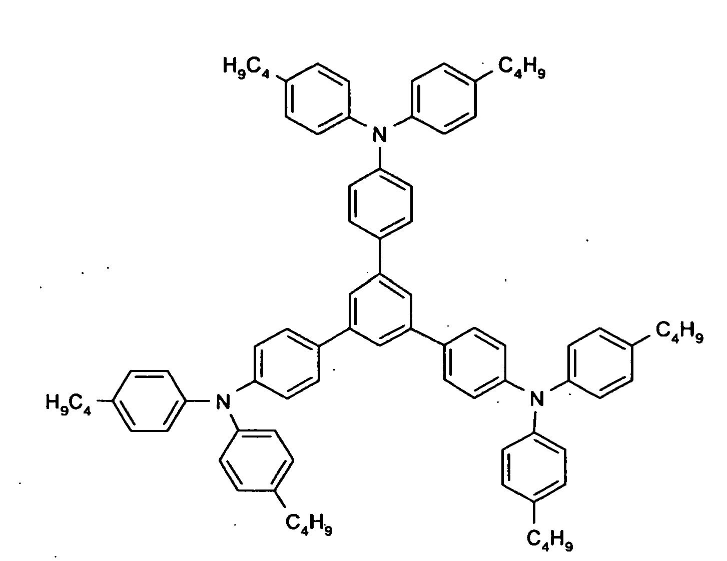

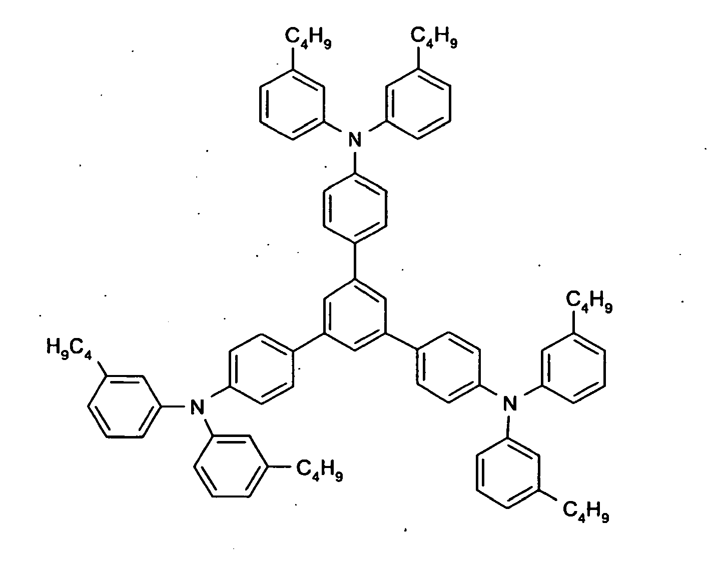

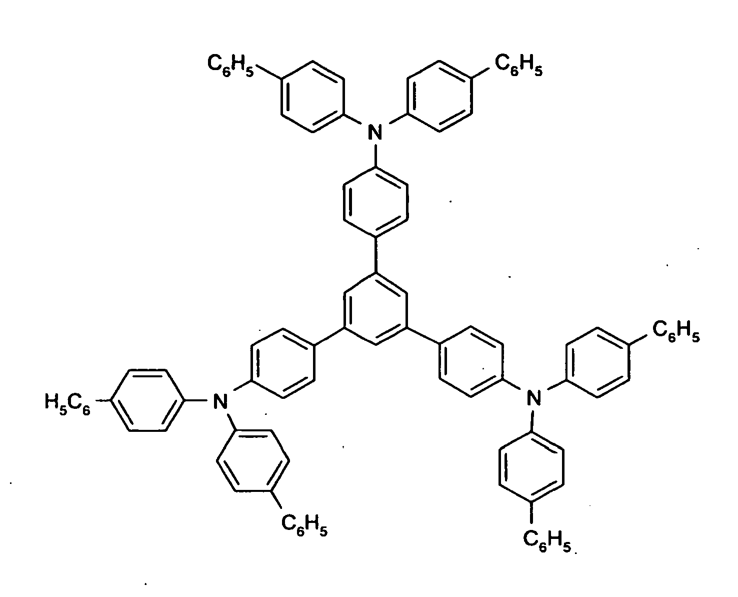

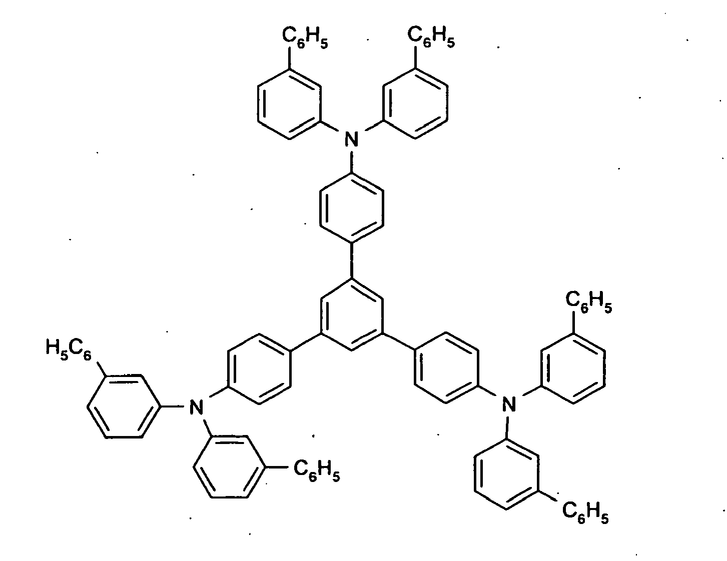

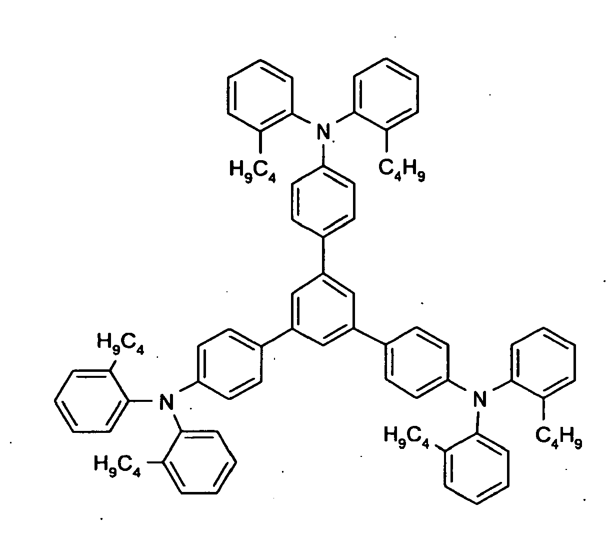

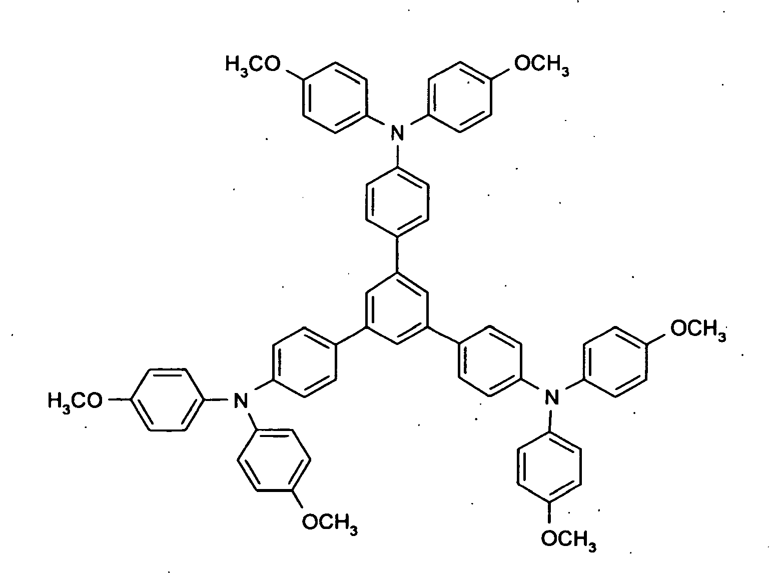

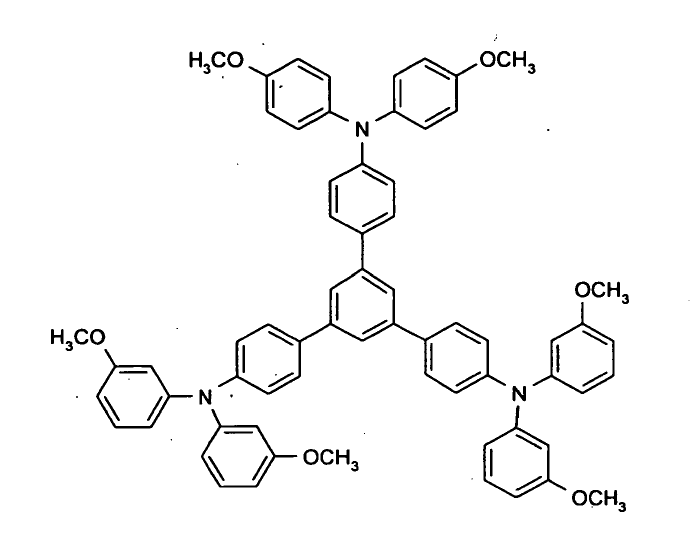

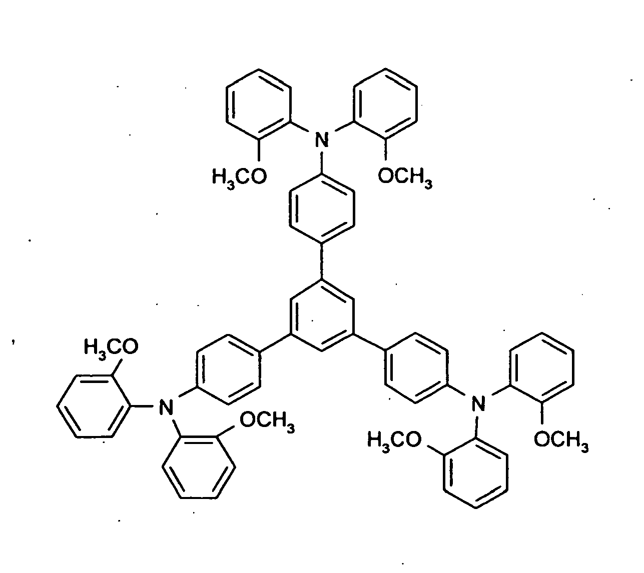

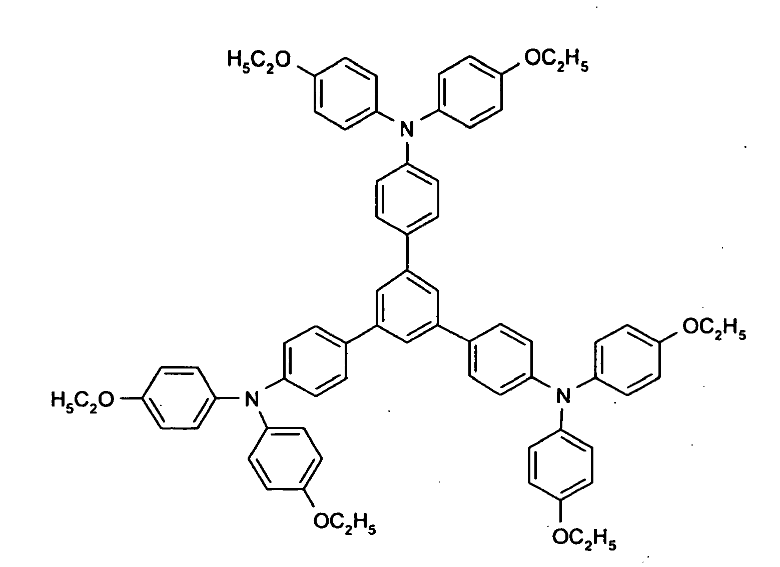

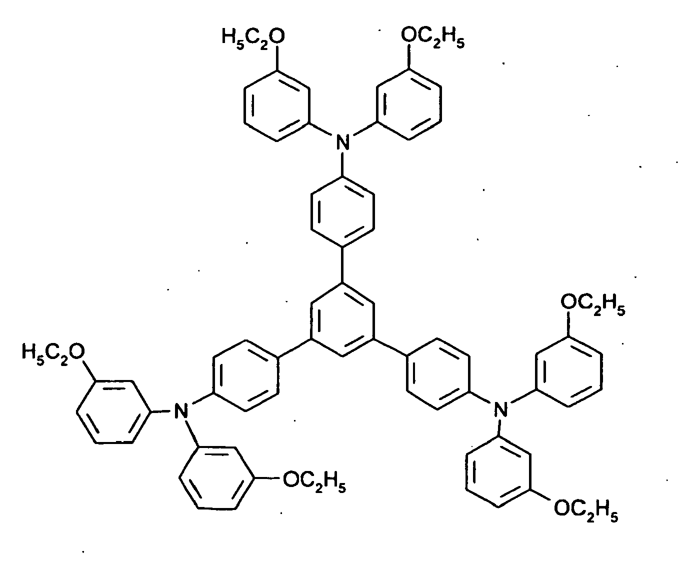

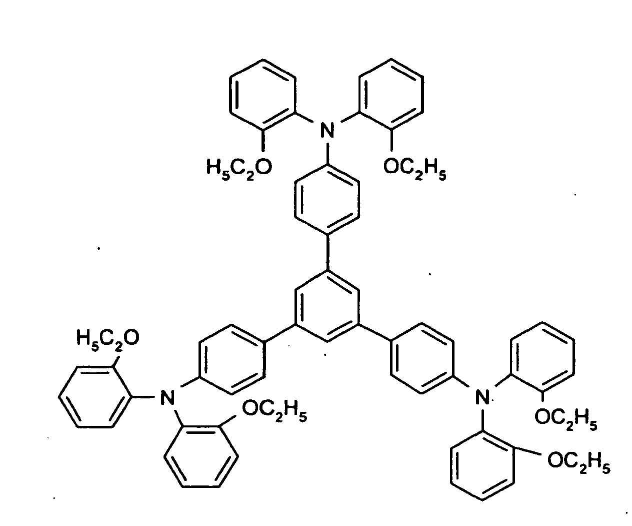

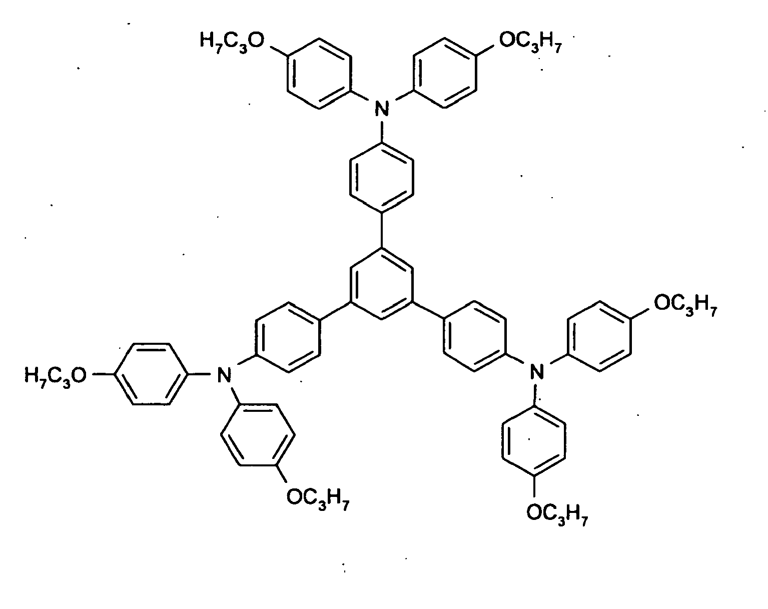

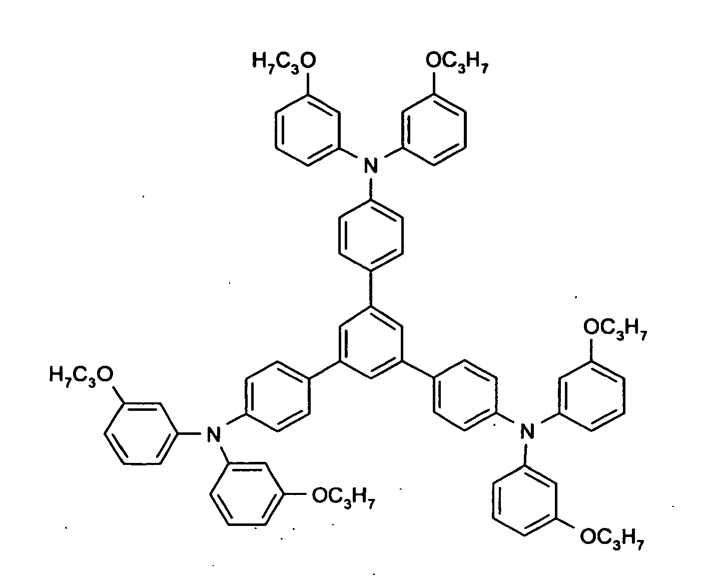

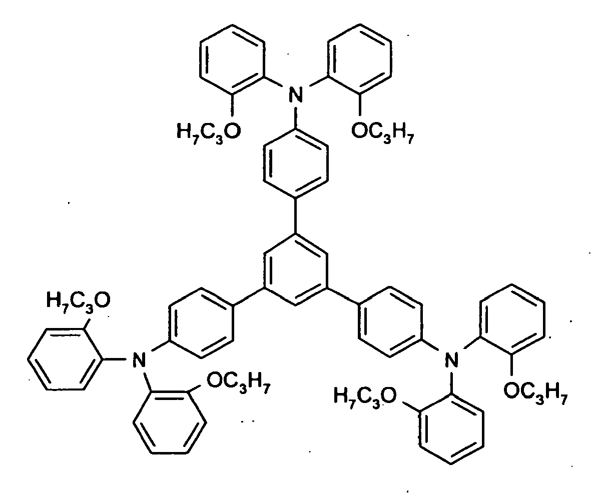

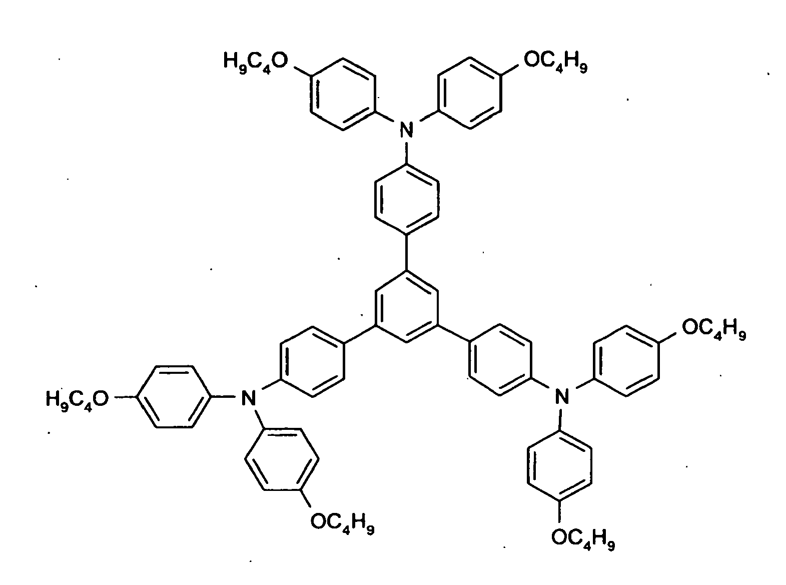

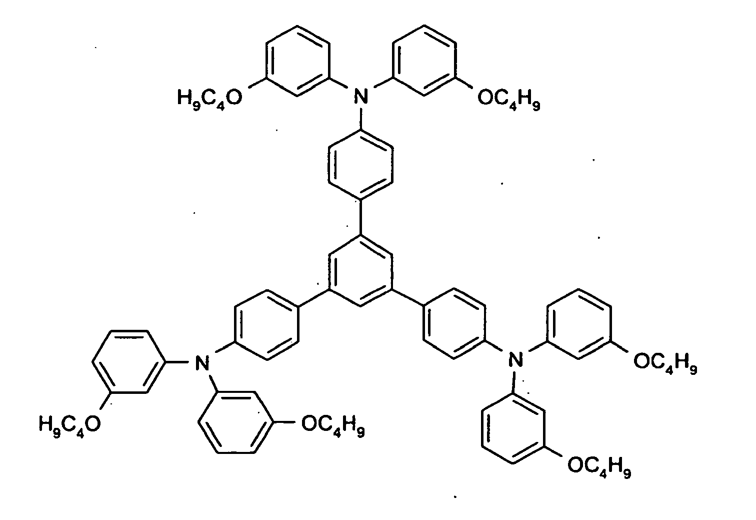

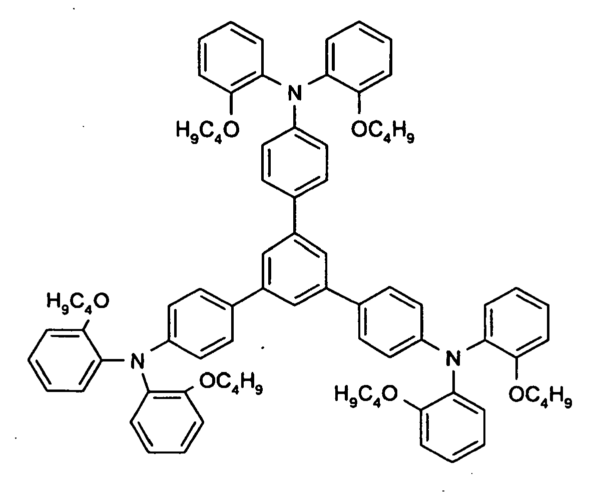

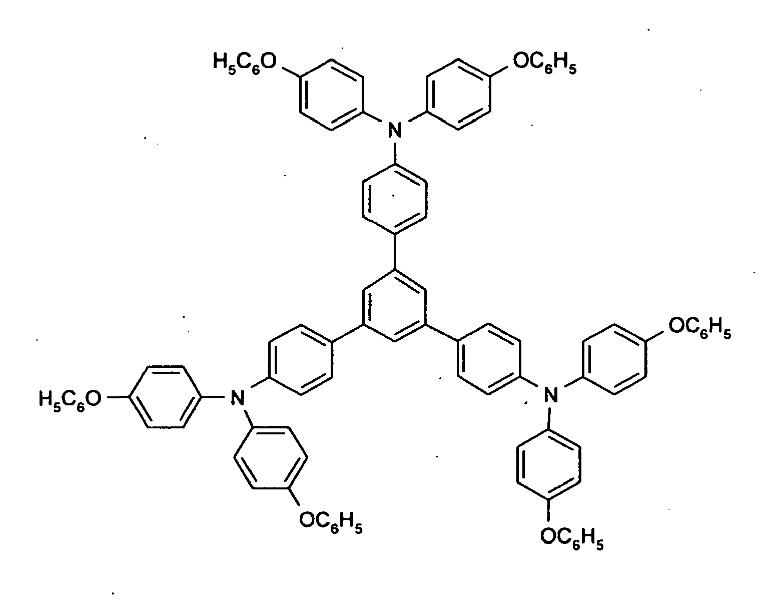

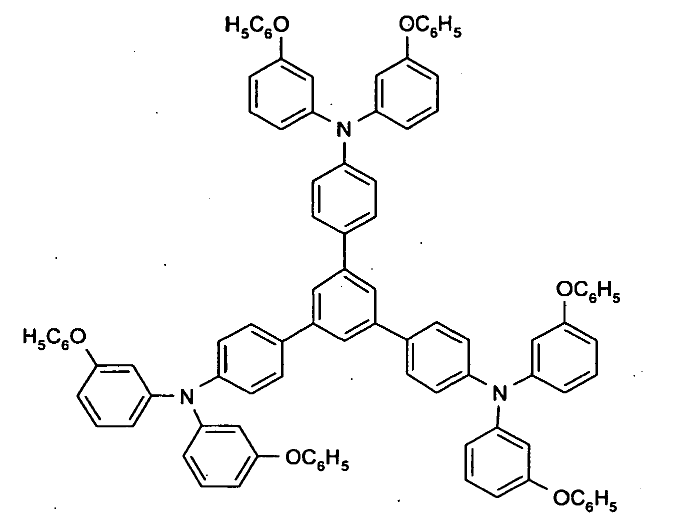

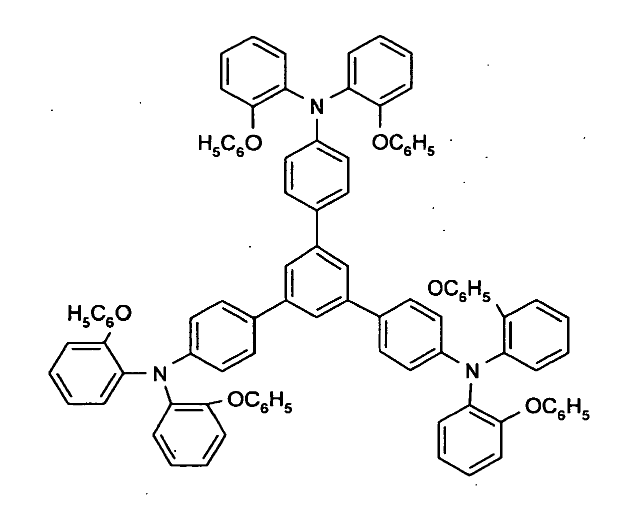

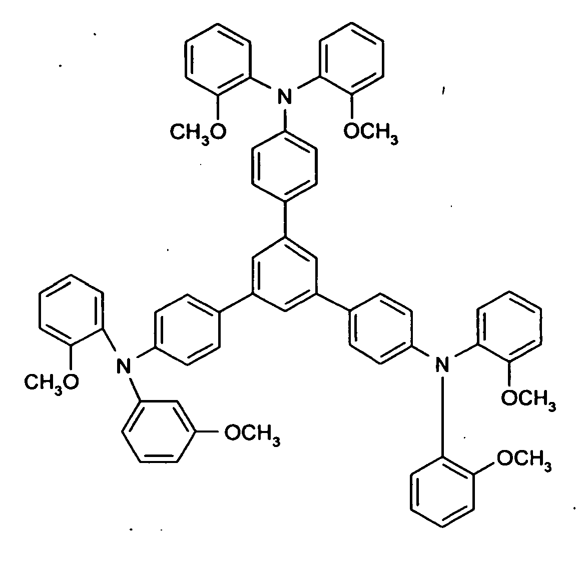

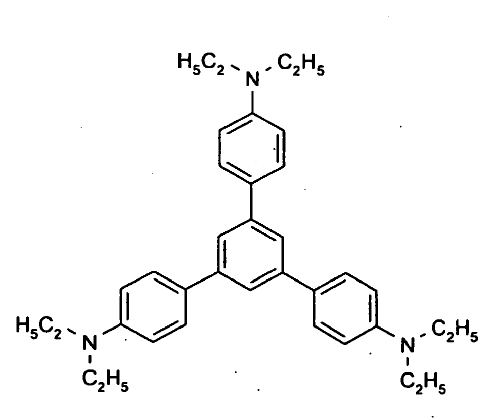

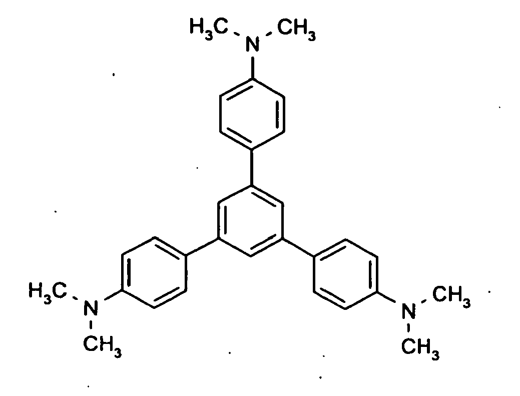

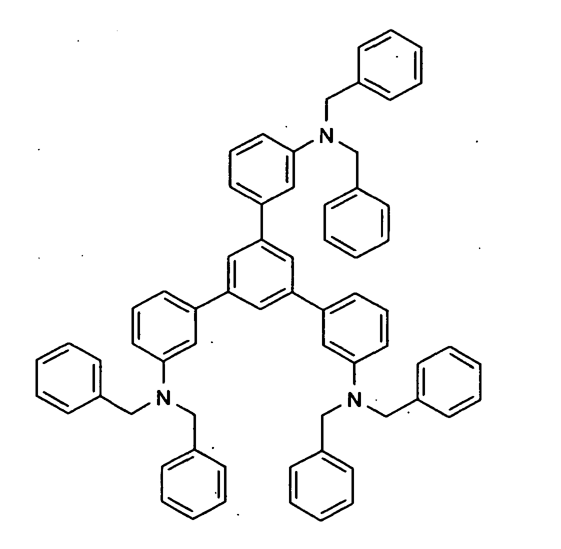

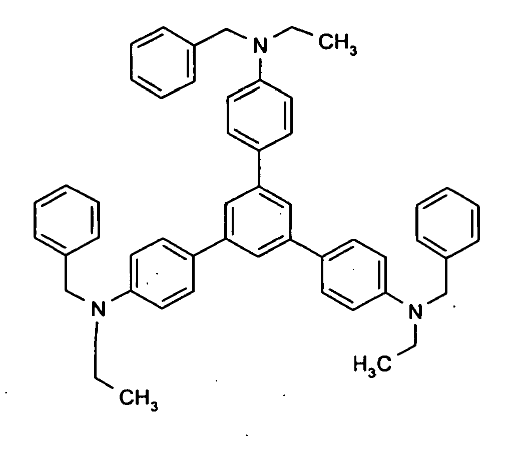

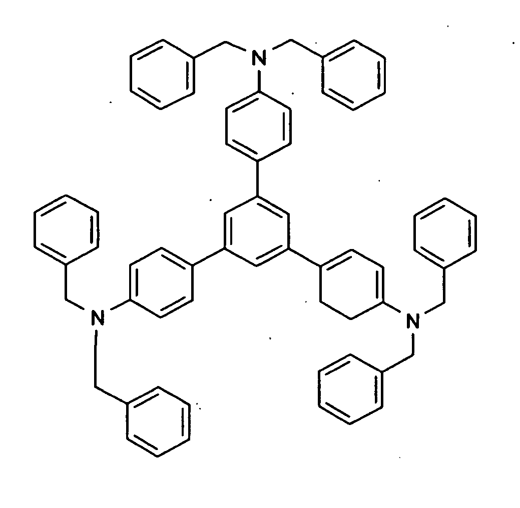

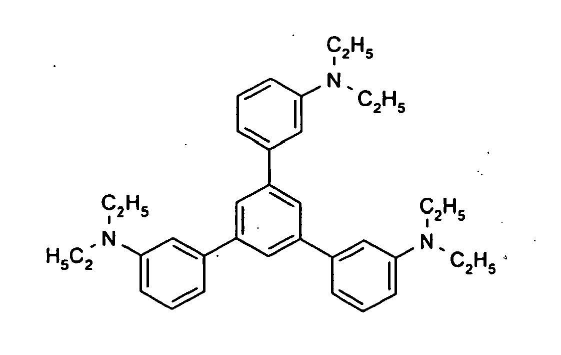

Bei den Mischungen aus den Verbindungen der Formel (II), kann es sich einerseits um Gemische von Isomeren handeln, andererseits um Mischungen aus Verbindungen der allgemeinen Formel (II) -mit verschiedener Struktur handeln.

Materialien, die lochleitende Eigenschaften aufweisen und als Mischpartner zur Komponente A eingesetzt werden können, sind beispielsweise die folgenden Verbindungen, wobei X¹ bis X⁶ unabhängig voneinander für H, Halogen, Alkyl, Aryl, Alkoxy, Aryloxy stehen. 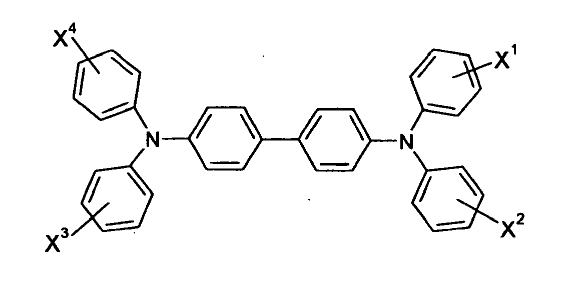 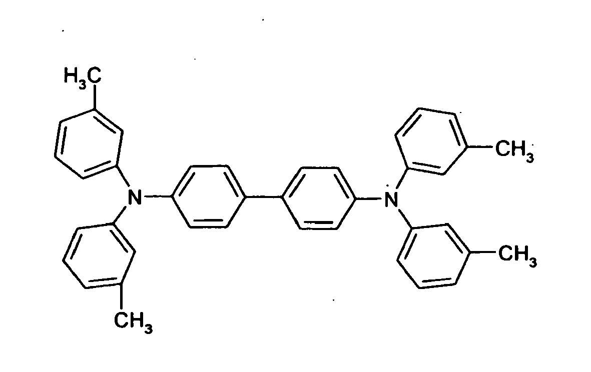 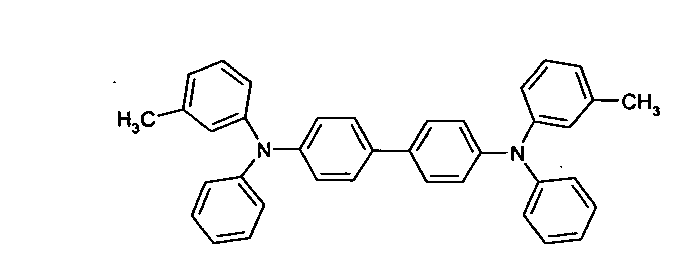 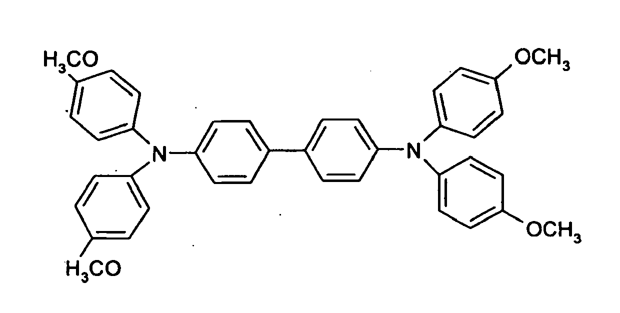 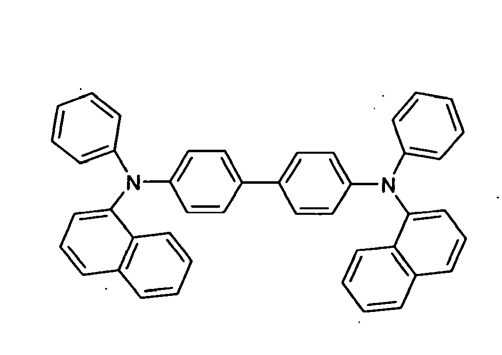 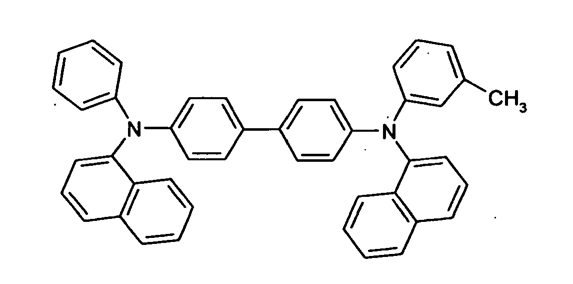 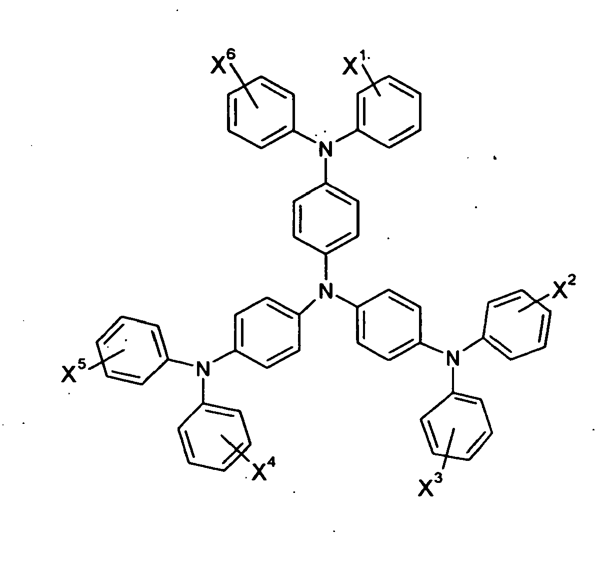 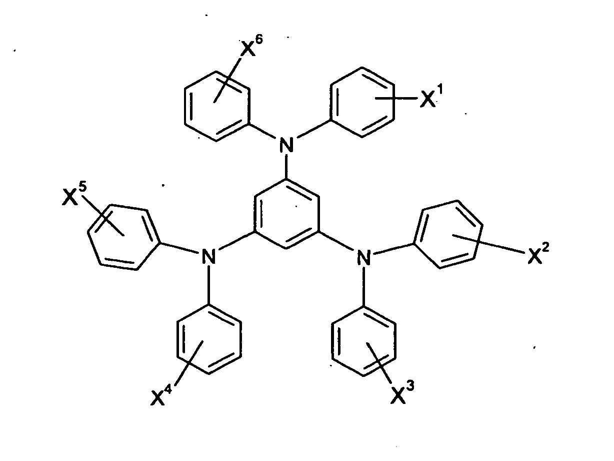 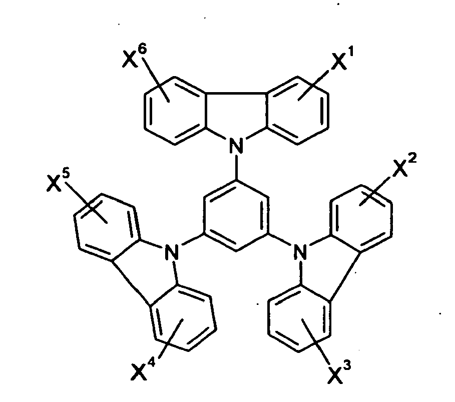 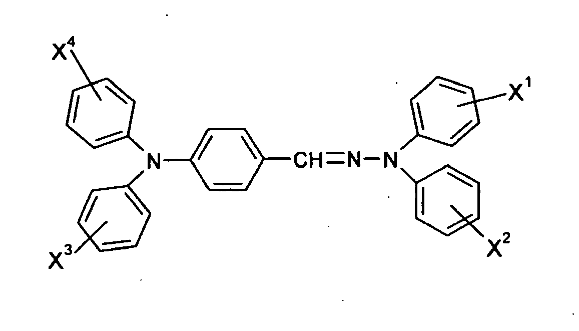 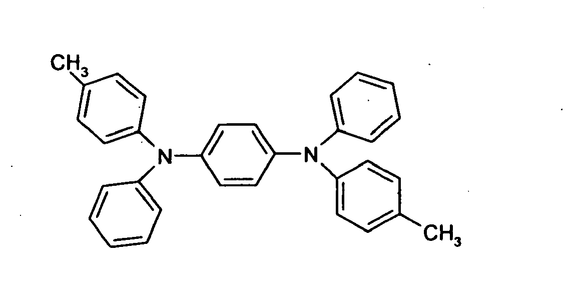 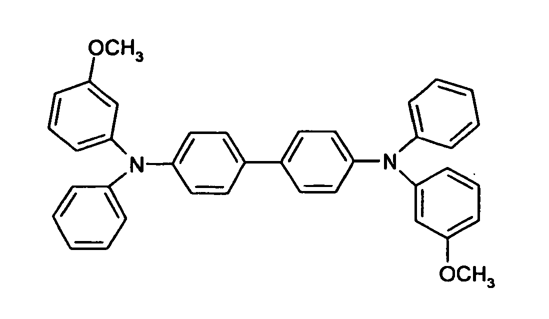 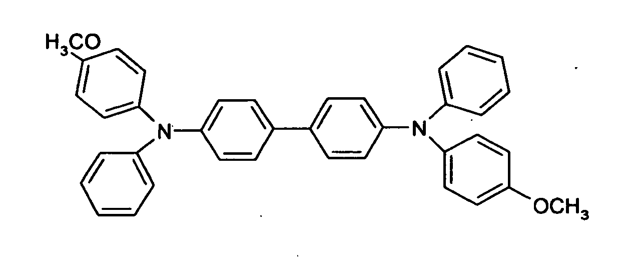 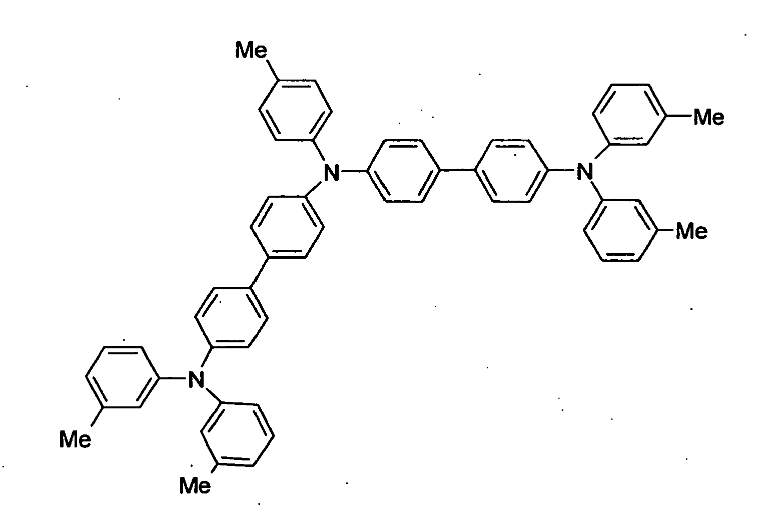 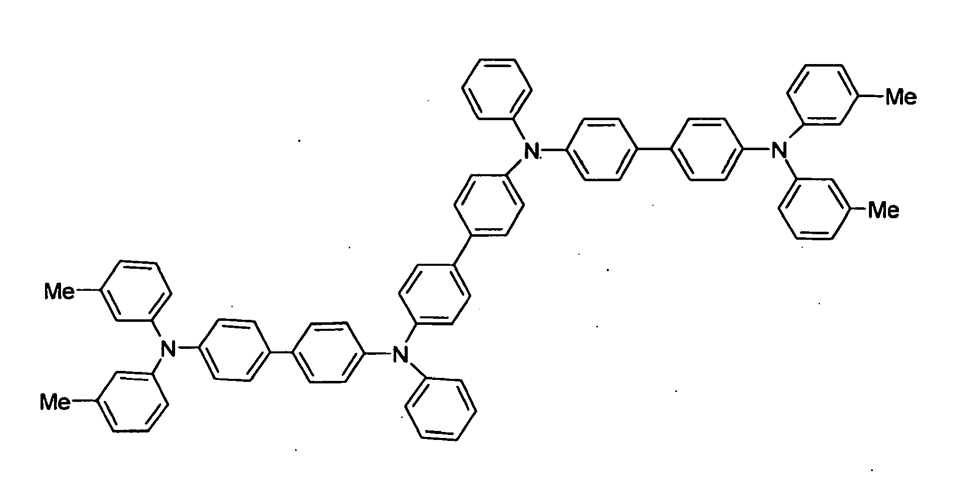 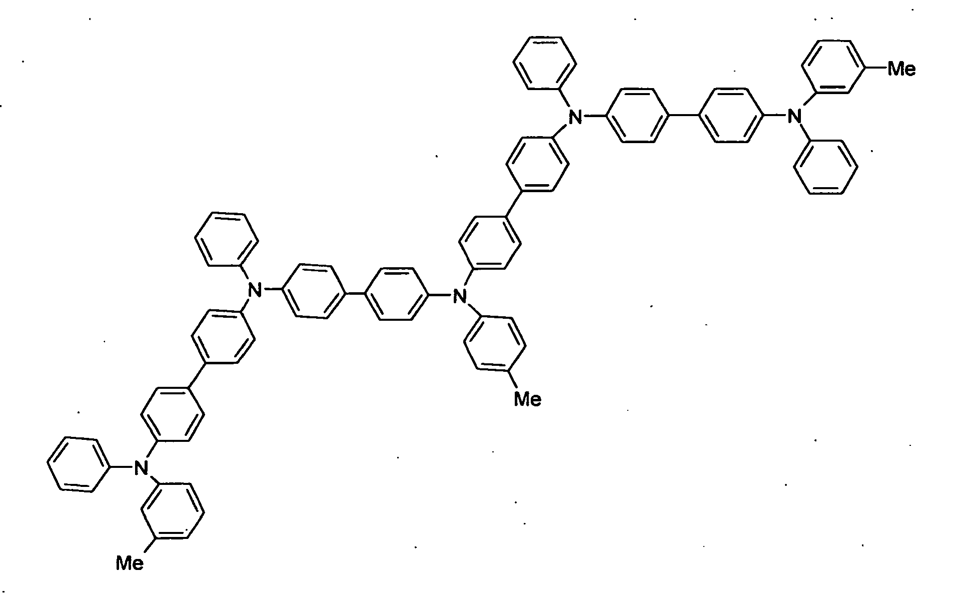 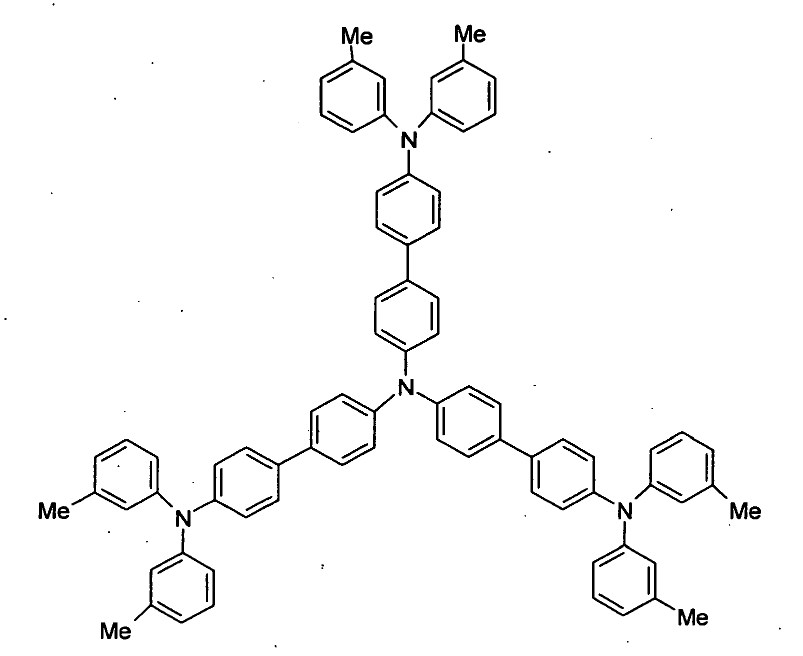 Me = Methyl
Diese und weitere Beispiele sind beschrieben in J. Phys. Chem. 1993, 97, 6240-6248 und Appl. Phys. Lett., Vol. 66, No. 20,2679-2681.

X¹ bis X⁶ stehen unabhängig voneinander vorzugsweise für Wasserstoff, Fluor, Chlor, Brom, (C₁-C₁₀)-, insbesondere (C₁-C₄)-Alkyl oder -Alkoxy, Phenyl, Naphthyl, Phenoxy und/oder Naphthyloxy. Die aromatischen Ringe können ein-, zwei, drei- oder vierfach, gleich oder verschieden durch mindestens einen Rest X¹ bis X⁶ substituiert sein.

Bei den Mischungen der aromatischen Amine können die Verbindungen in einem beliebigen Verhältnis eingesetzt werden.

Die Polythiophene der wiederkehrenden Struktureinheit der Formeln (Ia) und (Ib) sind bekannt (vgl. EP-A 440 958 und 339 340). Die Herstellung der erfindungsgemäßen Dispersionen bzw. Lösungen ist in EP-A 440 957 und DE-A 4 211 459 beschrieben.

Die Polythiophene werden in der Dispersion bzw. Lösung bevorzugt in kationischer Form, wie sie z.B. durch Behandlung der neutralen Thiophene mit Oxidationsmitteln erhalten werden, eingesetzt. Übliche Oxidationsmittel wie Kaliumperoxodisulfat werden für die Oxidation verwendet. Durch die Oxidation erhalten die Polythiophene positive Ladungen, die in den Formeln nicht dargestellt sind, da ihre Zahl und ihre Position nicht einwandfrei feststellbar sind. Gemäß den Angaben in EP-A 339 340 können sie direkt auf Trägern hergestellt werden.

Die sich zwischen lochinjizierender Zone und Kathode befindlichen Zonen oder Zone können auch mehrere Funktionen übernehmen, d.h. daß eine Zone z.B. lochtransportierende, elektrolumineszierende, elektronentransportierende, elektroneninjizierende und/oder Substanzen enthalten kann.

Das elektrolumineszierende Element kann ferner einen oder mehrere transparente polymere Binder B enthalten.

Besonders bevorzugt sind kationische bzw. neutrale Polythiophene der Formeln (Ia-1) und (Ib-1) 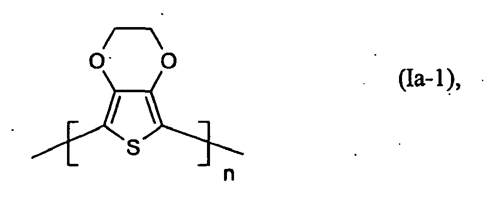 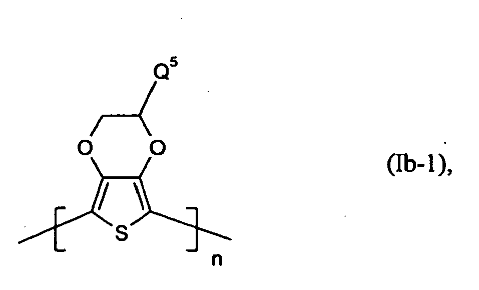 worin
Q⁵ und n die oben angegebene Bedeutung haben.

Als Polyanionen dienen die Anionen von polymeren Carbonsäuren, wie Polyacrylsäuren, Polymethacrylsäure oder Polymaleinsäuren und polymeren Sulfonsäuren, wie Polystyrolsulfonsäuren und Polyvinylsulfonsäuren. Diese Polycarbon- und -sulfonsäuren können auch Copolymere von Vinylcarbon- und Vinylsulfonsäuren mit anderen polymerisierbaren Monomeren, wie Acrylsäureestern und Styrol, sein.

Als Gegenion wird bevorzugt das Anion der Polystyrolsulfonsäure oder ein Erdalkalisalz davon eingesetzt, besonders bevorzugt als Gegenion ist das Anion der Polystyrolsulfonsäure.

Das Molekulargewicht der die Polyanionen liefernden Polysäuren beträgt vorzugsweise 1 000 bis 2 000 000, besonders bevorzugt 2 000 bis 500 000. Die Polysäuren oder ihre Alkalisalze sind im Handel erhältlich, z.B. Polystyrolsulfonsäuren und Polyacrylsäuren, oder aber nach bekannten Verfahren herstellbar (siehe z.B. Houben-Weyl, Methoden der organischen Chemie, Bd. E 20 Makromolekulare Stoffe, Teil 2 (1987), S. 1141 f).

Anstelle der für die Bildung der Dispersionen aus Polydioxythiophenen und Polyanionen erforderlichen freien Polysäuren, kann man auch Gemische aus Alkalisalzen der Polysäuren und entsprechenden Mengen an Monosäuren einsetzen.

Im Falle der Formel (Ib) und (Ib-1) tragen die Polydioxythiophene positive und negative Ladung in der Monomereinheit selbst.

Das elektrolumineszierende Element kann gegebenenfalls eine weitere funktionalisierte Verbindung aus der Gruppe der lochinjizierenden und/oder lochtransportierenden Materialien, ein lumineszierendes Material C und gegebenenfalls Elektronentransportmaterialien enthalten, wobei die lochtransportierende Zone neben der Komponente A eine oder mehrere weitere lochtransportierende, elektrolumineszierende, elektronentransportierende und/oder elektroneninjizierende Verbindungen enthalten kann, wobei mindestens eine Zone vorhanden ist, einzelne Zonen weggelassen werden können und die vorhandene(n) Zone(n) mehrere Aufgaben übernehmen kann.

Als lumineszierendes Material (Komponente C) können solche Substanzen eingesetzt werden, die Photolumineszenz zeigen, d.h. Fluoreszenz- und Laserfarbstoffe, aber auch Metallkomplexe und Chelate oder anorganische Nanoteilchen.

Beispiele für Fluoreszenz- und Laserfarbstoffe sind Stilbene, Distilbene, Methinfarbstoffe, Cumarine, Naphthalimide, Perylene, Rubren, Chinacridone, Phenanthrene, Anthracene, Phthalocyamine usw. Weitere Beispiele sind in EP-A 532 798 beschrieben.

Bei den Metallkomplexen können generell ein-, zwei- oder dreiwertige Metalle benutzt werden, von denen bekannt ist, daß sie Chelate bilden.

Das Metall kann ein ein-, zwei- oder dreiwertiges Metall sein, beispielsweise Lithium, Natrium, Kalium, Magnesium, Calcium, Bor, Aluminium, Gallium, Indium, Seltende Erden. Als anorganische Nanoteilchen eignen sich beispielsweise Halbleiter wie CdS, CdSe, ZnS oder ZnO.

Geeignete Beispiele für die Komponente C) sind die Oxin-Komplexe (8-Hydroxychinolin-Komplexe) von Al³⁺, Mg²⁺, In³⁺, Ga³⁺, Zn²⁺, Be²⁺, Li⁺, Ca²⁺, Na⁺ oder Aluminiumtris(5-methyloxin) und Galliumtris(5-chloro-chinolin). Auch Komplexe mit Seltenenerd-Metallen sind einsetzbar.

Beispiele für Metallkomplexe sind
Alq₃ q = 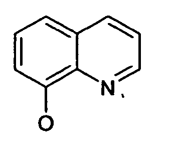 Inq₃ , Gaq₃, Znq₂, Beq₂, Mgq₂,
oder Al(qa)₃, Ga(qa)₃, In(qa)₃, Zn(qa)₂, Be(qa)₂, Mg(qa)₂ wobei
(qa) für 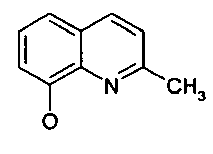 steht.

Es können auch Metallkomplexe eingesetzt werden, die über unterschiedliche Reste verfügen. Beispiele für derartige Verbindungen sind Alq₂ OR, Gaq₂ OR, Al(qa)₂ OR, Ga(qa)₂ OR, Alqa₂ OCOR, Ga(qa)₂ OCOR, Alq₂X und Gaq₂X sowie Al(qa)₂X und Ga(qa)₂X wobei X für Halogen steht und wobei R für jeweils gegebenenfalls substituiertes Alkyl, Aryl, Arylalkyl und Cycloalkyl stehen, vorzugsweise für jeweils gegebenenfalls durch Halogen und/oder Cyano substituiertes (C₁-C₁₂)-Alkyl, insbesondere (C₁-C₈)-Alkyl, (C₄-C₈)-Cycloalkyl, insbesondere Cyclopentyl oder Cyclohexyl, Aryl mit 6 bis 20 C-Atomen, insbesondere Phenyl, Naphthyl, Phenyl-(C₁-C₄)-alkyl, wobei die cyclischen und aromatischen Reste auch (C₁-C₄)-alkylsubstituiert sein können.

Die Kohlenstoffketten sind jeweils geradkettig oder verzweigt.

Eine Zusammenstellung geeigneter Metallkomplexe und elektronenleitender Verbindungen ist in EP-A 525 739, EP-A 579 151 und EP-A 757 088 wiedergegeben. Herstellungsverfahren sind beispielsweise in US-A 4 769 292 beschrieben.

Es können auch Mischungen unterschiedlicher Metallkomplexe eingesetzt werden.

Als Binder B) können Polymere und/oder Copolymere wie z.B. Polycarbonate, Polyestercarbonate, Polystyrol, Poly-α-methylstyrol, Copolymere des Styrols wie SAN oder Styrolacrylate, Polysulfone, Polymerisate auf Basis von Vinylgruppen-haltigen Monomeren wie z.B. Poly(meth)acrylate, Polyvinylpyrrolidon, Polyvinylcarbazol, Vinylacetat- und Vinylalkoholpolymere und -copolymere, Polyolefine, cyclische Olelfincopolymere, Phenoxyharze usw. eingesetzt werden. Es können auch Mischungen verschiedener Polymere eingesetzt werden. Die polymeren Binder B) weisen Molekulargewichte von 1 000 bis 200 000 g/mol auf, sind löslich und filmbildend und sind im sichtbaren Spektralbereich transparent. Sie sind z.B. beschrieben in Encyclopedia of Polymer Science and Engineering, 2nd Ed. bei A. Wiley-Intersciencepublication.

Die Komponenten können aber auch in separaten Schichten angeordnet sein.

Die einzelnen Zonen des elektrolumineszierenden Elements können entweder aus einer Lösung oder durch Deposition aus der Gasphase oder einer Kombination beider Verfahren abgeschieden werden.

Zur Herstellung des Schichtaufbaus werden beispielsweise in einem geeigneten Lösemittel gelöst und durch Gießen, Rakeln, Drucken oder spincoating auf eine geeignete Unterlage aufgebracht. Dabei kann es sich z.B. um Glas oder ein Kunststoffmaterial handeln, das mit einer gegebenenfalls transparenten Elektrode versehen ist. Als Kunststoffmaterial kann z.B. eine Folie aus Polycarbonat, Polyester wie Polyethylenterephthalat oder Polyethylennaphthalat, Polysulfon oder Polyimid eingesetzt werden.

Als transparente und semitransparente Elektroden sind geeignet
a) Metalloxide, z.B. Indium-Zinn-Oxid (ITO), Zinnoxid (NESA), Zinkoxid, dotiertes Zinnoxid, dotiertes Zinkoxid, etc.,
b) semi-transparente Metallfime, z.B. Au, Pt, Ag, Cu etc.,
c) leitfähige Polymerfilme wie Polyaniline, Polythiophene, etc.

Die Metalloxid- und die semitransparenten Metallfilmelektroden werden durch Techniken wie Aufdampfen, Aufsputtern, Platinierung, etc., in dünner Schicht aufgebracht. Die leitfähigen Polymerfilme werden durch Techniken wie Spincoaten, Casting, Rakeln, Drucken etc. aus der Lösung aufgebracht.

Die Dicke der transparenten bzw. semitransparenten Elektrode beträgt 3 nm bis etwa mehrere µm, vorzugsweise 10 nm bis 500 nm.

In einer bevorzugten Anordnung wird das elektrolumineszierende Element direkt auf die Anode aufgebracht. In einer alternativen Ausführungsform kann das elektrolumineszierende Element auf den mit einer Kathode versehenen Träger aufgebracht werden.

Die Dicke des elektrolumineszierenden Elements beträgt im allgemeinen 10 nm bis 5 µm, vorzugsweise 20 nmbis 1 µm, besonders bevorzugt 50 nm bis 600nm.

Eine Zusammenstellung von geeigneten ladungstransportierenden Zwischenschichten, bei denen es sich um loch- und/oder elektronenleitenden Materialien handeln kann, die in polymerer oder niedermolekularer Form gegebenenfalls als Blend vorliegen können, ist in EP-A 532 798 aufgeführt.

Der Gehalt an niedermolekularen Verbindungen - lochinjizierende, lochtransportierende, elektrolumineszierende, elektronenleitende und elektroneninjizierende Substanzen - in einem polymeren Binder ist im allgemeinen im Bereich von 2 bis 97 Gew.-% variierbar; bevorzugt beträgt der Gehalt 5 bis 95 Gew.-%, besonders bevorzugt 10 bis 90 Gew.-%, insbesondere 10 bis 85 Gew.-%.

Filmbildende niedermolekulare Verbindungen können auch in reiner Form (100 %ig) eingesetzt werden.

Blends, die ausschließlich aus niedermolekularen Verbindungen bestehen, können durch Deposition aus der Gasphase abgeschieden werden; lösliche und filmbildende Blends, die neben niedermolekularen Verbindungen auch einen Binder B) enthalten können (nicht notwendigerweise), können aus einer Lösung z.B. mittels SpinCoating, Gießen, Rakeln, Drucken deponiert werden.

Es ist auch möglich, emittierende und/oder elektronenleitende Substanzen in einer separaten Schicht auf die lochleitende Schicht mit der Komponente A aufzubringen. Dabei kann eine emittierende Substanz auch der die Verbindung A enthaltenden Schicht zudotiert ("Dopant") und zusätzlich eine elektronenleitende Substanz aufgebracht werden. Eine elektrolumineszierende Substanz kann auch der elektroneninjizierenden bzw. elektronenleitenden Schicht zugesetzt werden.

Der Gehalt an niedermolekularen Elektronenleitern im polymeren Binder ist im Bereich von 2 bis 95 Gew.-% variierbar; bevorzugt beträgt der Gehalt 5 bis 90 Gew.-%, besonders bevorzugt 10 bis 85 Gew.-%. Filmbildende Elektronenleiter können auch in reiner Form (100 %ig) eingesetzt werden.

Die Gegenelektrode zu der sich auf dem Träger befindlichen Elektrode besteht aus einer leitfähigen Substanz, die gegebenenfalls transparent ist, enthält vorzugsweise Metalle, z.B. Ca, Al, Au, Ag, Mg, In, etc. oder Legierungen und Metalloxide, leitfähige Polymere die durch Techniken wie z. B. Aufdampfen, Aufsputtern, Platinierung, Drucken, Gießen oder Rakeln aufgebracht werden können.

Die erfindungsgemäße Anordnung wird durch zwei elektrische Zuführungen (z.B. Metalldrähte) mit den beiden Elektroden in Kontakt gebracht.

Die Anordnungen emittieren beim Anlegen einer Gleichspannung im Bereich von 0,1 bis 100 Volt Licht der Wellenlänge von 200 bis 2 000 nm.

Die erfindungsgemäßen Anordnungen sind zur Herstellung von Einheiten zur Beleuchtung und zur Informationsdarstellung geeignet.

### Beispiele

### Beispiel 1

Beim erfindungsgemäßen Aufbau einer organischen Leuchtdiode (OLED) wird folgendermaßen vorgegangen:
1. Reinigung des ITO-Substrats
   ITO-beschichtetes Glas (Merck Balzers AG, Fürstentum Lichtenstein, Part.No. 253 674 XO) wird in 50 mm x 50 mm große Stücke (Substrate) geschnitten. Die Substrate werden anschließend in 3 %iger wässriger Mukasollösung im Ultraschalbad 15 min lang gereinigt. Danach werden die Substrate mit destilliertem Wasser gespült und in einer Zentrifuge trocken geschleudert. Dieser Spül- und Trockenvorgang wird 10 mal wiederholt.
2. Aufbringen der Baytron^{®} P-Schicht auf das ITO
   Etwa 10 ml der ca. 1,2 %igen Poly(ethylendioxythiophen)Polysulphonsäure-Lösung (BAYER AG, Leverkusen, Deutschland, Baytron^{®} P) werden filtriert (Millipore HV, 0,45 µm). Das Substrat wird anschließend auf eine Lackschleuder gelegt und die filtrierte Lösung wird auf der ITO-beschichteten Seite des Substrats verteilt. Anschließend wird die überstehende Lösung durch Rotation des Tellers bei 500 U/min über den Zeitraum von 3 min abgeschleudert. Danach wird das so beschichtete Substrat 5 min lang bei 110°C auf einer Heizplatte getrocknet. Die Schichtdicke beträgt 60 nm (Tencor, Alphastep 200).
3. Aufbringen der lochleitenden Schicht
   5 ml einer 1,5 %igen Dichlorethanlösung aus 1 Gew.-Teil Polyvinylcarbazol (PVK) (BASF, Ludwigshafen, Deutschland, Luvican) und 2 Gew.-Teile des Amins A werden filtriert (Millipore HV, 0,45 µm) und auf der getrockneten Baytron P Schicht verteilt. Anschließend wird die überstehende Lösung durch Rotation des Tellers bei 800 U/min 50 sec. lang abgeschleudert. Danach wird das so beschichtete Substrat 5 min lang bei 110°C auf einer Heizplatte getrocknet. Die Gesamtschichtdicke beträgt 150 nm.
4. Aufbringen der lichtemittierenden/elektroneninjizierenden Schicht
   5 ml einer 1,5 %igen Methanollösung aus dem Metallkomplex 1 werden filtriert (Millipore HV, 0,45 µm) und auf der getrockneten lochleitenden Schicht verteilt. Anschließend wird die überstehende Lösung durch Rotation des Tellers bei 400U/min 30 sec. lang abgeschleudert. Danach wird das so beschichtete Substrat 5 min lang bei 110°C auf einer Heizplatte getrocknet. Die Gesamtschichtdicke beträgt 200 nm.
5. Aufdampfen der Metallkathode
   Auf das organische Schichtsystem wird eine Metallelektrode gedampft. Dazu wird das Substrat mit dem organischen Schichtsystem nach unten auf eine Lochmaske (Lochdurchmesser 5 mm) gelegt. Aus zwei Aufdampfschiffchen werden bei einem Druck von 10⁻³ Pa parallel die Elemente Mg und Ag verdampft. Die Aufdampfraten betragen für Mg: 28 Å/sec und für Ag: 2 Å/sec. Die Dicke der aufgedampften Metallkontakte beträgt 500 nm.

Die beiden Elektroden der organischen LED werden über elektrische Zuführungen mit einer Spannungsquelle verbunden. Der positive Pol ist mit der ITO-Elektrode, der negative Pol ist mit der MgAg-Elektrode verbunden.

Bereits ab einer Spannung von 3 Volt läßt sich mit einer Photodiode (EG & G C30809E) Elektrolumineszenz nachweisen. Bei einer Spannung von 10 Volt fließt ein Flächenstrom von 35 mA/cm² und die Elektrolumineszenz ist gut sichtgar. Die Farbe der Elektrolumineszenz ist grün-blau.

### Beispiel 2

Vorgehen beim erfindungsgemäßen Aufbau einer OLED wie in Beispiel 1, mit folgendem Unterschied:
- 5 ml einer 1,0 %igen Methanollösung bestehend aus 1 Gew.-Teil Metallkomplex 1 und 0,02 Gew.-Teile Fluoreszenzfarbstoff F werden filtriert (Millipore HV, 0,45 µm) und auf der getrockneten lochleitenden Schicht verteilt. Anschließend wird die überstehende Lösung durch Rotation des Tellers bei 400 U/min 30 sec lang abgeschleudert. Danach wird das so beschichtete Substrat 5 min lang bei 110°C auf einer Heizplatte getrocknet.

Ab einer Spannung von 3 Volt läßt sich mit einer Photodiode (EG & G C30809E) Elektrolumineszenz nachweisen. Bei einer Spannung von 10 Volt fließt ein Flächenstrom von 180 mA/cm² und die Elektrolumineszenz ist gut sichtbar. Die Farbe der Elektrolumineszenz ist grün-blau.

### Beispiel 3

Vorgehen beim erfindungsgemäßen Aufbau einer OLED wie in Beispiel 1, mit folgendem Unterschied:
- 5 ml einer 1,0 %igen Methanollösung Metallkomplex 2 werden filtriert (Millipore HV, 0,45 µm) und auf der getrockneten lochleitenden Schicht verteilt. Anschließend wird die überstehende Lösung durch Rotation des Tellers bei 250 U/min 40 sec lang abgeschleudert. Danach wird das so beschichtete Substrat 5 min lang bei 110°C auf einer Heizplatte getrocknet.

Ab einer Spannung von 3 Volt läßt sich mit einer Photodiode (EG & G C30809E) Elektrolumineszenz nachweisen. Bei einer Spannung von 10 Volt fließt ein Flächenstrom von 100 mA/cm² und die Elektrolumineszenz ist gut sichtbar. Die Farbe der Elektrolumineszenz ist grün-blau.

### Amin A

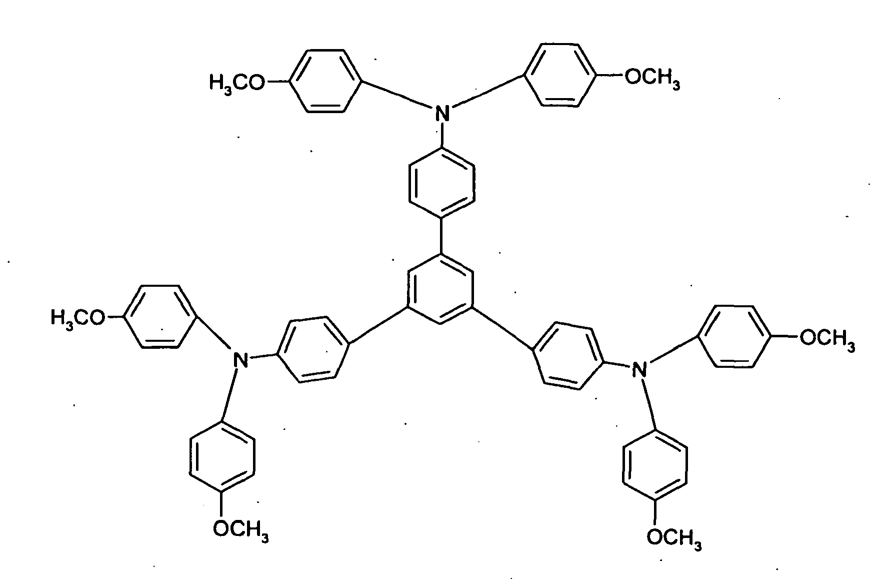

### Metallkomplex 1:

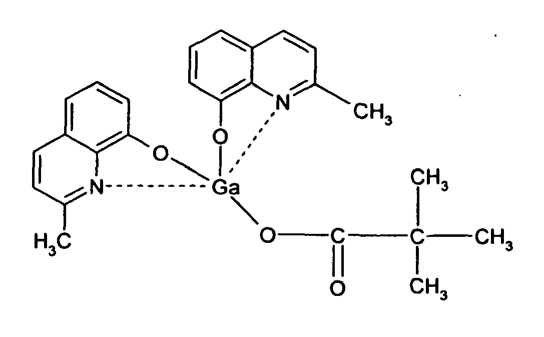

### Metallkomplex 2:

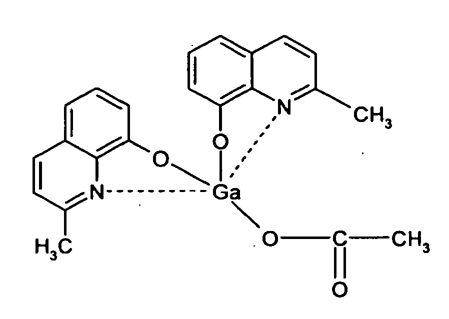

### Fluoreszenzfarbstoff F:

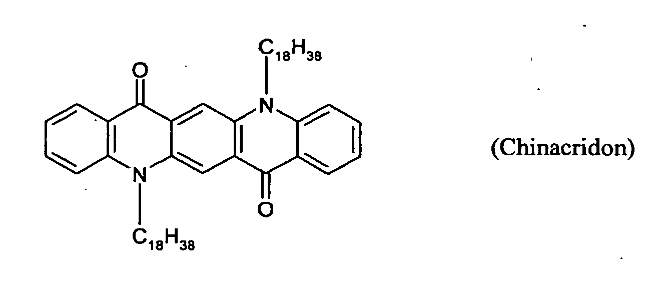

### Beispiel 4 (nicht Teil der Erfindung)

### Substrat: Baltracon 255 (Firma Balzers)

Lochinjizierende Schicht: Baytron^{®}P (Bayer AG, Leverkusen) ca. 1,2 %ige Lösung, Lösemittel H₂O, aufgeschleudert bei 800 U/min, Schichtdicke ca. 70 nm.
Lochleitende Schicht: Polystyrol von Aldrich, 89555 Steinheim, Deutschland, Artikel.-Nr. 18, 242-7) (PS) + Amin A (1:1) aus Lösungsmittel Dichlorethan, 1 %ige Lösung aufgeschleudert bei 800 U/min, Schichtdicke ca. 60 nm.
Elektroleitende Schicht: Alq₃ aufgedampft ca. 60 nm bei einem Druck von 10e-6 mbar. Polystyrol von Aldrich, 89555 Steinheim, Deutschland, Artikel.-Nr. 18, 242-7)
Kathode: MgAg (10:1) aufgedampft (Kodeposition), Schichtdicke ca. 200 nm.
Bei einer Spannung von 9 Volt fließt ein Strom von 21 mA/cm²; die Lichtintensität beträgt 290 Cd/m².

### Beispiel 5 (nicht Teil der Erfindung)

### Substrat: Baltracon 255 (Firma Balzers)

Lochinjizierende Schicht: Baytron^{®}P (Bayer AG, Leverkusen) ca. 1.2 %ige Lösung, Lösemittel H₂O, aufgeschleudeert bei 800 U/min, Schichtdicke ca. 70 nm.
Lochleitende Schicht: PS + Amin A (1:2) aus Lösungsmittelgemisch Cyclohexan/THF (Tetrahydrofuran) (1:10), 1 %ige Lösung aufgeschleudert bei 800 U/min, Schichtdicke ca. 60 nm.
Elektronenleitende Schicht: Metallkomplex 3, 1 %ig aus Methanol bei 300 U/min aufgeschleudert, Schichtdicke ca. 30 nm.
Kathode: MgAg (10:1) aufgedampft (Kodeposition), Schichtdicke ca. 200 nm.
Bei einer Spannung von 7 Volt fließt ein Strom von 17 mA/cm²; die Lichtintensität beträgt 210 Cd/m².
Lichtemission: blaugrün

### Beispiel 6

### Substrat: Baltracon 255 (Firma Balzers)

Lochinjizierende Schicht: Baytron^{®}P (Bayer AG, Leverkusen) ca. 1,2 %ige Lösung, Lösemittel H₂O, aufgeschleudert bei 800 U/min, Schichtdicke ca. 70 nm.
Lochleitende Schicht: PVK + Amin A (1:4) dotiert mit Rubren, 2,5 % bezogen auf Feststoffanteil aus Lösungsmittel Dichlorethan, 1 %ige Lösung aufgeschleudert bei 800 U/min, Schichtdicke ca. 60 nm. (PVK = Polyvinylcarbazol/Luvican EP, BASF AG, Ludwigshafen, Deutschland).
Elektroleitende Schicht: Alq₃ aufgedampft ca. 60 nm bei einem Druck von 10e-6 mbar.
Kathode: MgAg (10:1) aufgedampft (Kodeposition), Schichtdicke ca. 200 nm.
Lichtemission: gelb
Bei einer Spannung von 9 Volt fließt ein Strom von 54 mA/cm²; die Lichtintensität beträgt 1200 Cd/m².

### Beispiel 7 (nicht Teil der Erfindung)

### Substrat: Baltracon 255 (Firma Balzers)

Lochinjizierende Schicht: Baytron^{®}P (Bayer AG, Leverkusen) ca. 1,2 %ige Lösung, Lösemittel H₂O, aufgeschleudert bei 800 U/min, Schichtdicke ca. 70 nm.
Lochleitende Schicht: PS + Amin A (1:2) aus Lösungsmittelgemisch Cyclohexan/THF (1:10), 1 %ige Lösung aufgeschleudert bei 800 U/min, Schichtdicke ca. 60 nm.
Elektronenleitende Schicht: Metallkomplex 3, 1 %ig aus Methanol bei 300 U/min aufgeschleudert, Schichtdicke ca. 30 nm.
Kathode; MgAg (10:1) aufgedampft (Kodeposition), Schichtdicke ca. 200 nm.
Bei einer Spannung von 9 Volt fließt ein Strom von 21 mA/cm²; die Lichtintensität beträgt 212 Cd/m².
Lichtemission: blaugrün

### Metallkomplex 3:

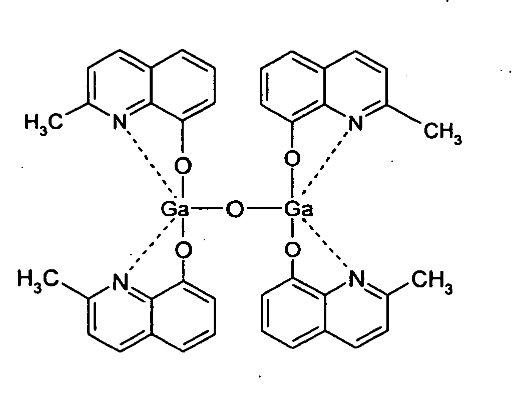

### Beispiel 8

### Substrat: Baltracon 255 (Firma Balzers)

Lochinjizierende Schicht: Baytron^{®}P (Bayer AG, Leverkusen) ca. 1 %ige Lösung, Lösemittel H₂O, aufgeschleudert bei 800 U/min, Schichtdicke ca. 70 nm.
Lochleitende Schicht: PVK + Amin A (1:2) aus Lösungsmittelgemisch Cyclohexan/THF (1:10), 1 %ige Lösung aufgeschleudert bei 800 U/min, Schichtdicke ca. 60 nm.
Elektronenleitende Schicht: Alq₃ aufgedampft ca. 60 nm bei einem Druck von 10e-6 mbar.
Kathode: MgAg (10:1) aufgedampft (Kodeposition), Schichtdicke ca, 200 nm.
Bei einer Spannung von 8 Volt fließt ein Strom von 30 mA/cm²; die Lichtintensität beträgt 500 Cd/m².

### Beispiel 9

### Substrat: Baltracon 255 (Firma Balzers)

Lochinjizierende Schicht: Baytron^{®}P (Bayer AG, Leverkusen) ca. 1,2 %ige Lösung, Lösemittel H₂O, aufgeschleudert bei 800 U/min, Schichtdicke ca. 70 nm.
Lochleitende Schicht: PVK + Amin B (1:1) aus Lösungsmittel Dichlorethan, 1 %ige Lösung, aufgeschleudert bei 800 U/min, Schichtdicke ca. 60 nm.
Elektronenleitende Schicht: Alq₃ aufgedampft ca. 60 nm bei einem Druck von 10e-6 mbar.
Kathode: MgAg (10:1) aufgedampft (Kodeposition), Schichtdicke ca. 200 nm.
Bei einer Spannung von 14 Volt fließt ein Strom von 55 mA/cm²; die Lichtintensität beträgt 521 Cd/m².

## Patentansprüche

1. Elektrolumineszierende Anordnungen, enthaltend ein Substrat, eine Anode, ein elektrolumineszierendes Element und eine Kathode, wobei wenigstens eine der beiden Elektroden im sichtbaren Spektralbereich transparent bzw. semitransparent ist und das elektrolumineszierende Element eine lochinjizierende Zone, eine lochtransportierende Zone, eine elektrolumineszierende Zone und gegebenenfalls eine elektronentransportierende Zone und/oder eine elektroneninjizierende Zone in der genannten Reihenfolge enthält, wobei jede der vorhandenen Zonen auch die Funktion der anderen Zonen übernehmen kann, **dadurch gekennzeichnet, daß** die lochinjizierende Zone ein neutrales oder kationisches Polythiophen der Formeln (Ia) und/oder (Ib)enthält, 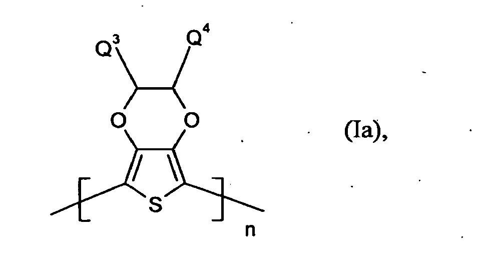 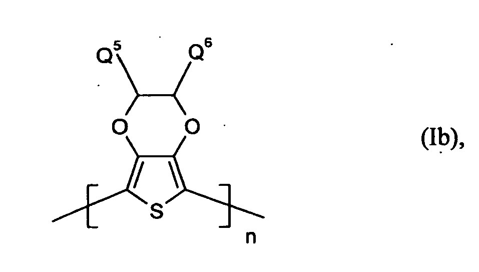 worin
Q³ und Q⁴ unabhängig voneinander für Wasserstoff, gegebenenfalls substituiertes (C₁-C₁₈)-Alkyl, (C₂-C₁₂)-Alkenyl, (C₃-C₇)-Cycloalkyl, (C₇-C₁₅)Aralkyl, (C₆-C₁₀)-Aryl, (C₁-C₁₈)-Alkoxy, oder (C₂-C₁₈)-Alkyloxyester steht und
Q⁵ und Q⁶ unabhängig voneinander für Wasserstoff, jedoch nicht beide gleichzeitig, mit jeweils mindestens einer Sulfonatgruppe substituiertes (C₁-C₁₈)-Alkyl, (C₂-C₁₂)-Alkenyl, (C₃-C₇)-Cycloalkyl, (C₇-C₁₅)-Aralkyl, (C₆-C₁₀)-Aryl, (C₁-C₁₈)-Alkoxy, oder (C₂-C₁₈)-Alkyloxyester steht,
n für eine ganze Zahl von 2 bis 10 000 steht.
und die an die lochinjizierende Zone angrenzende lochleitende Zone mehrere aromatische Aminverbindungen enthält, wobei als aromatische Aminverbindung mindestens eine Aminverbindung A ausgewählt aus der allgemeinen Formel (II) enthalten ist 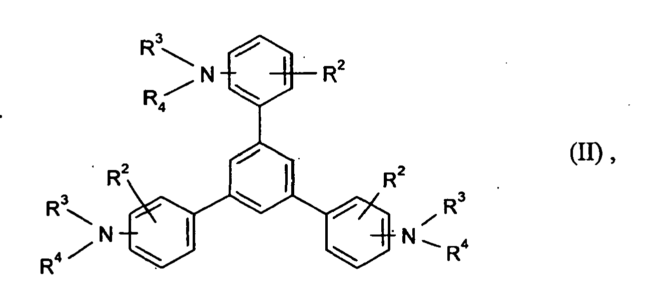 worin
R² für Wasserstoff, gegebenenfalls substituiertes Alkyl oder Halogen steht,
R³ und R⁴ unabhängig voneinander für gegebenenfalls substituiertes (C₁-C₁₀)-Alkyl, Alkoxycarbonyl-substituiertes (C₁-C₁₀)-Alkyl, jeweils gegebenenfalls substituiertes Aryl, Aralkyl oder Cycloalkyl stehen.

2. Elektrolumineszierende Anordnungen gemäß Anspruch 1, **dadurch gekennzeichnet, daß** ein oder mehrere transparente polymere Binder B enthalten sind.

3. Elektrolumineszierende Anordnungen gemäß Anspruch 1, **dadurch gekennzeichnet, daß** in Formel (II)
R³ und R⁴ unabhängig voneinander für (C₁-C₆)-Alkyl, (C₁-C₄)-Alkoxycarbonyl-(C₁-C₆)-alkyl, jeweils gegebenenfalls durch (C₁-C₄)-Alkyl und/oder (C₁-C₄)-Alkoxy substituiertes Phenyl-(C₁-C₄)-alkyl, Naphthyl-(C₁-C₄)-alkyl, Cyclopentyl, Cyclohexyl, Phenyl, Naphthyl oder Anthracyl stehen und
R² für Wasserstoff, (C₁-C₆)-Alkyl oder Halogen steht.

4. Elektrolumineszierende Anordnungen gemäß Anspruch 3, **dadurch gekennzeichnet, daß** die aromatische Aminverbindung A ausgewählt ist aus folgenden Verbindungen:

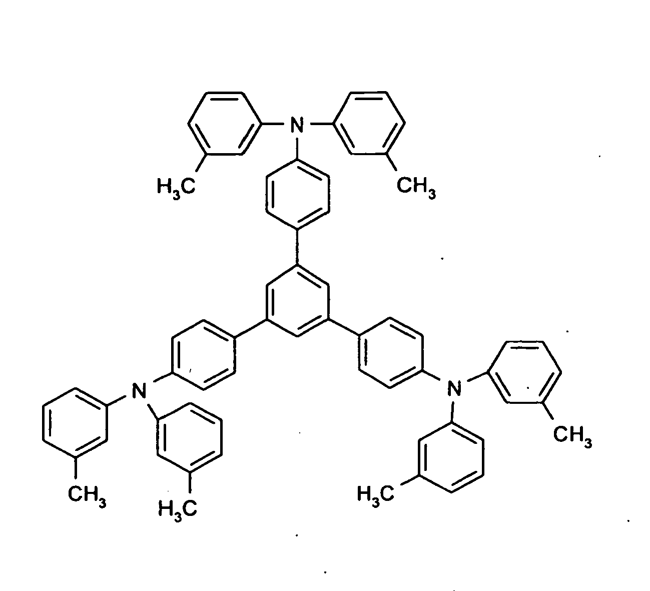

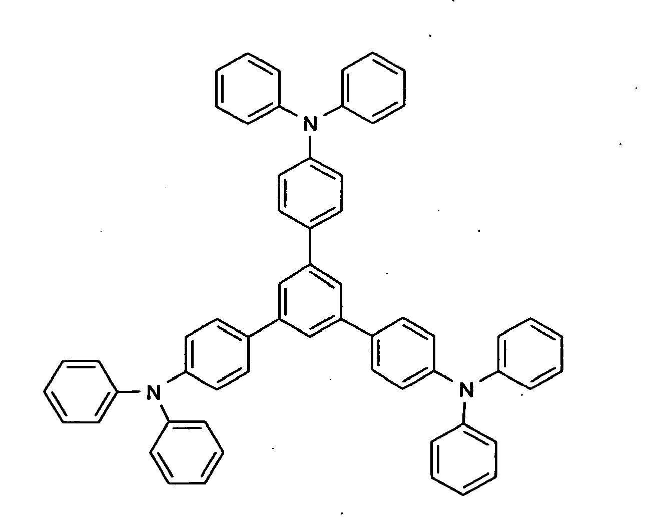

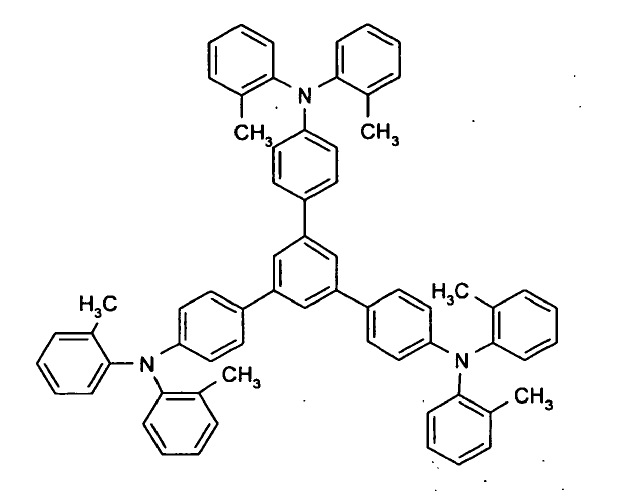

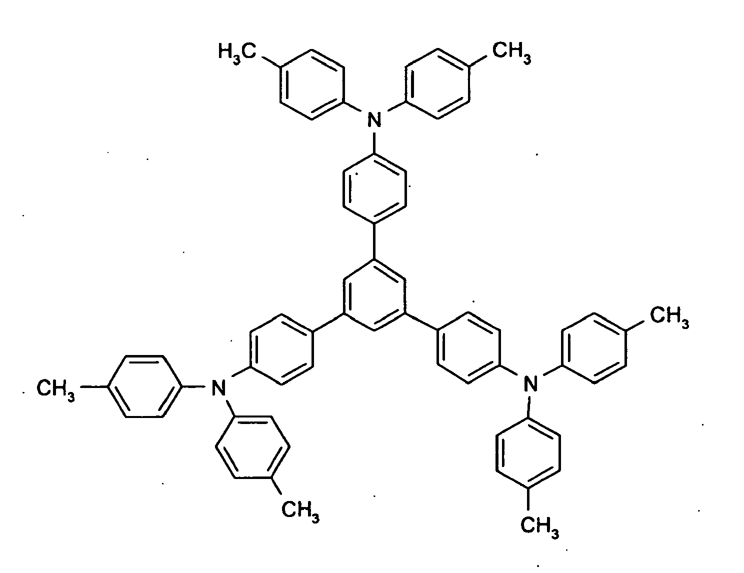

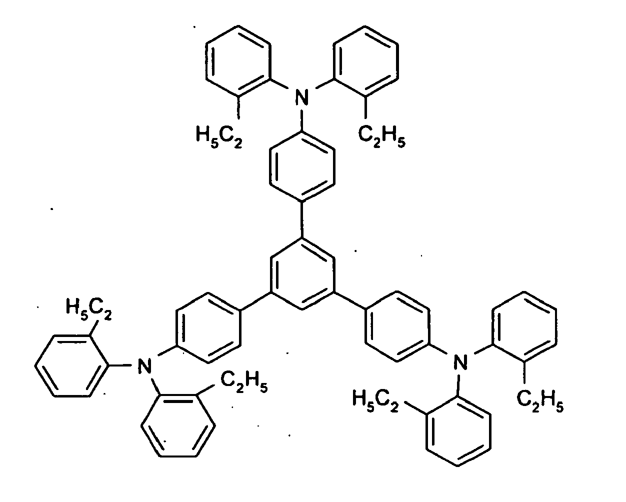

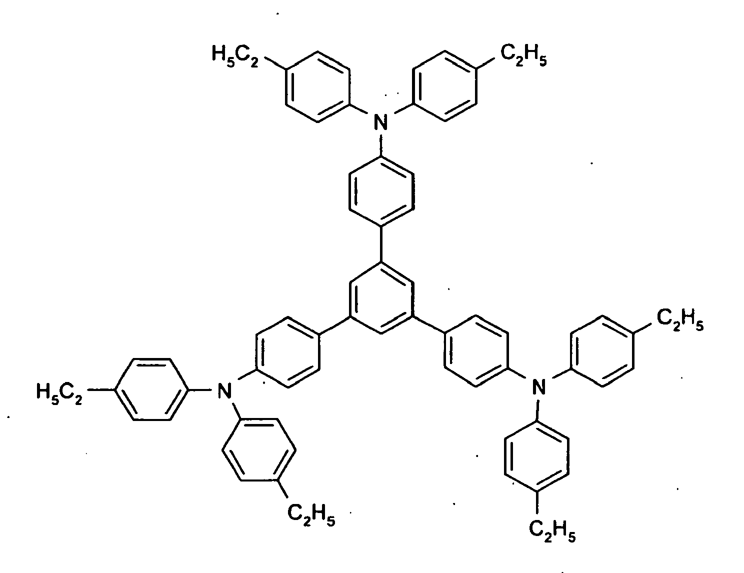

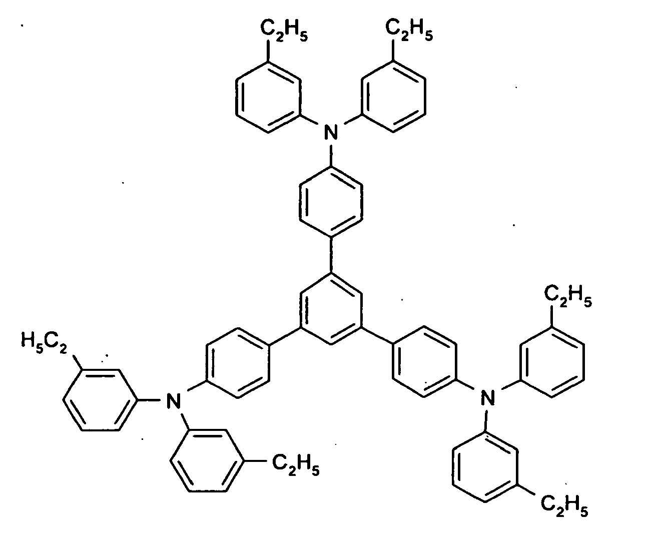

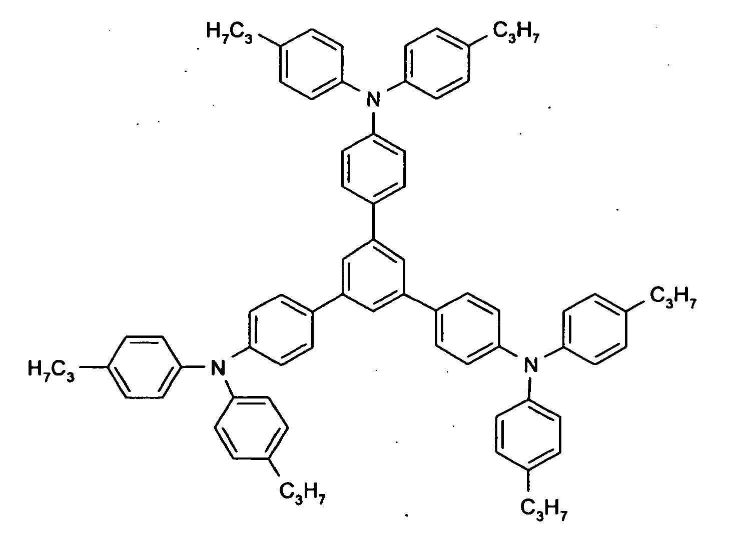

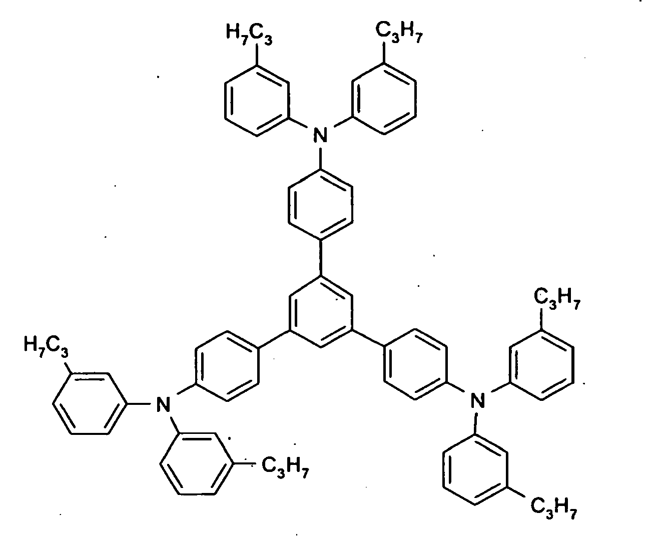

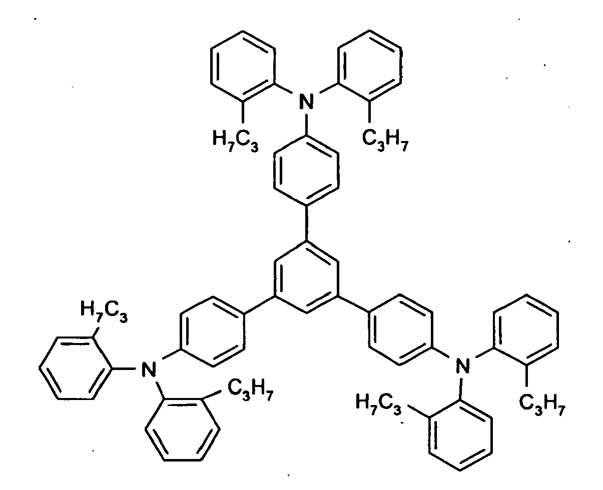

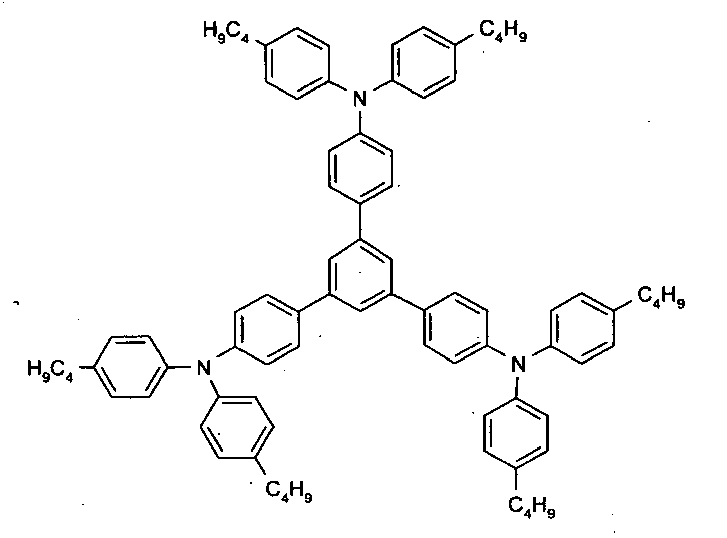

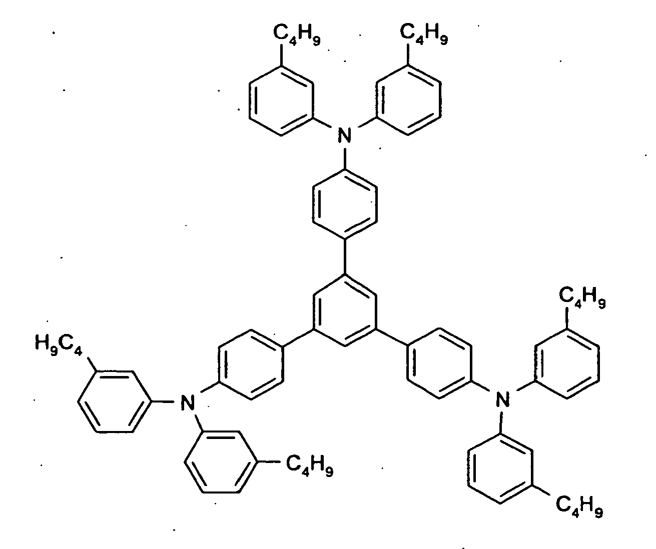

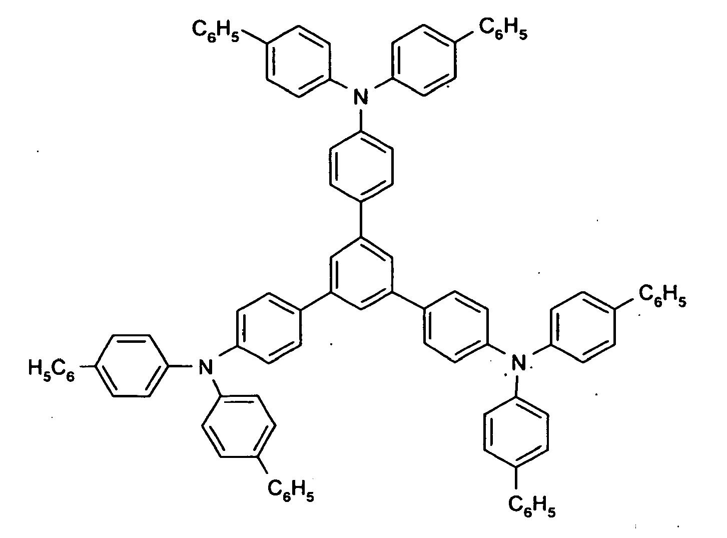

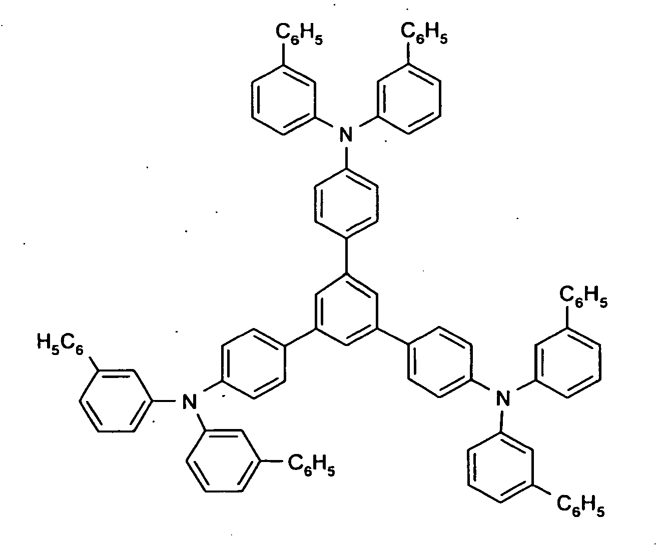

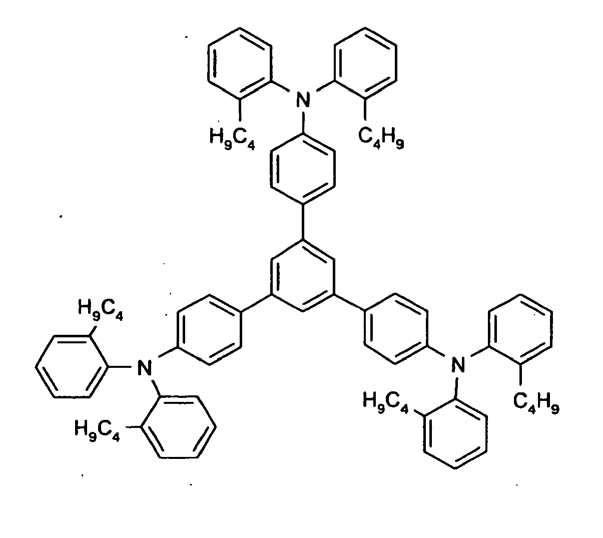

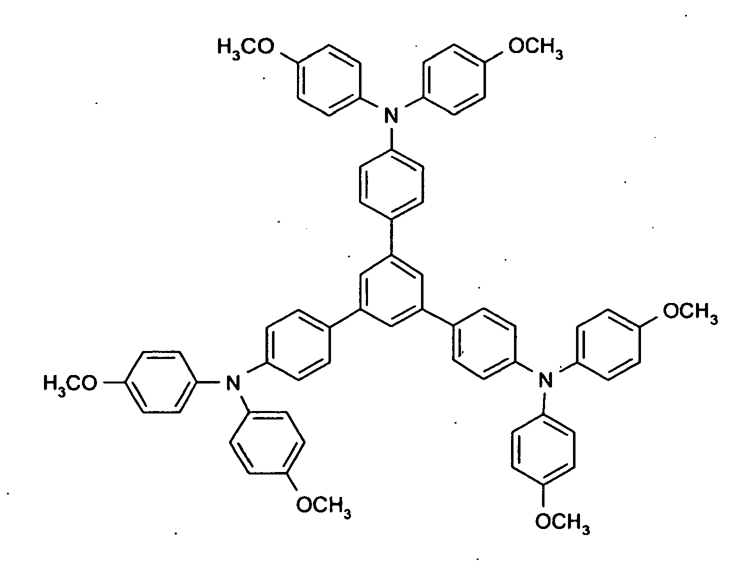

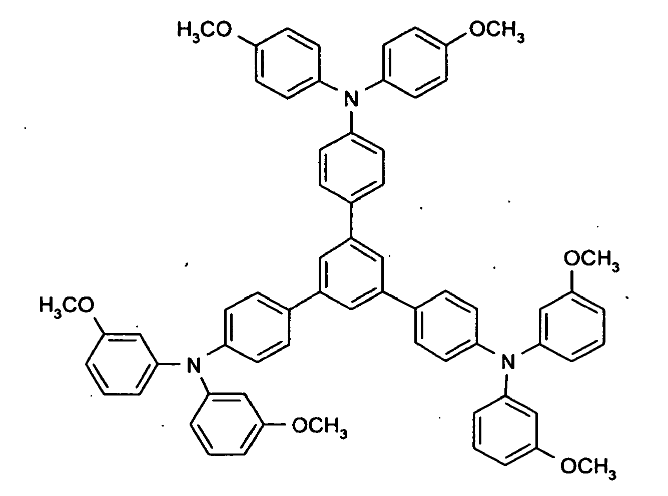

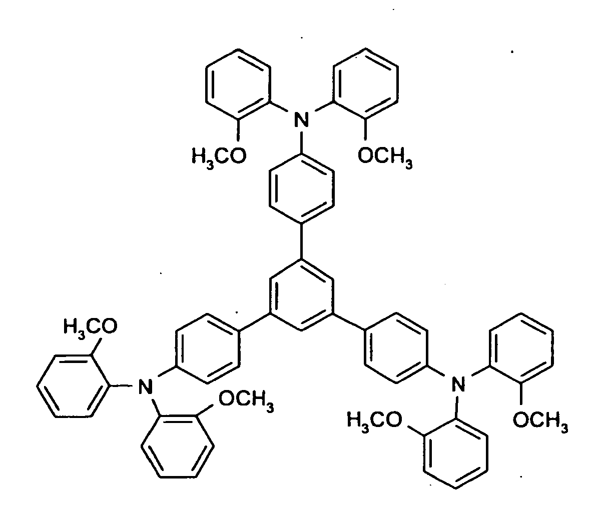

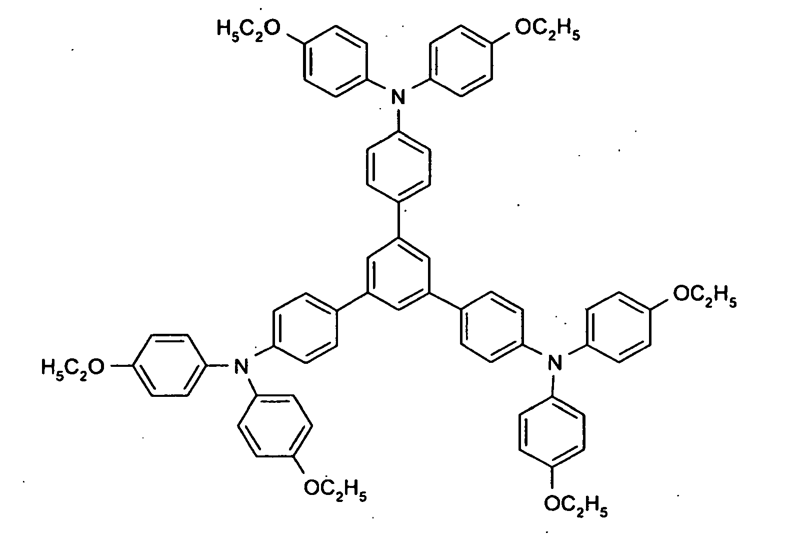

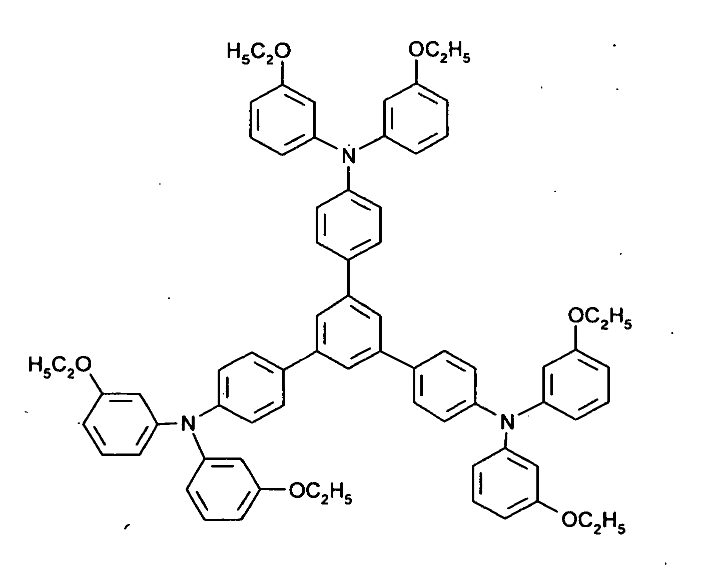

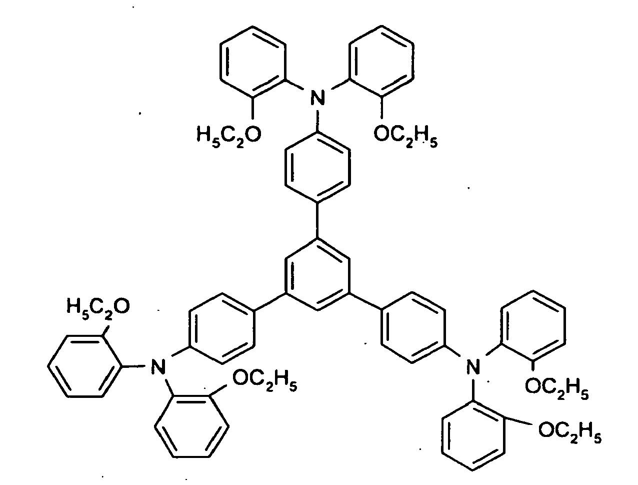

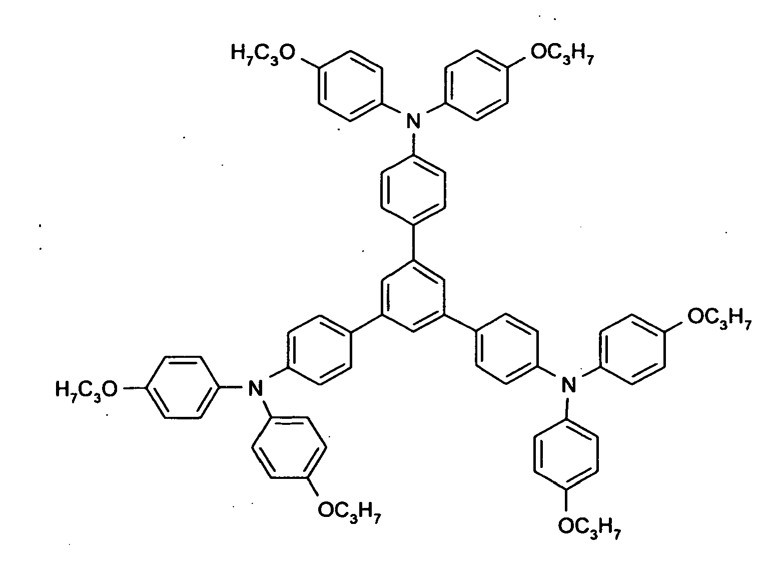

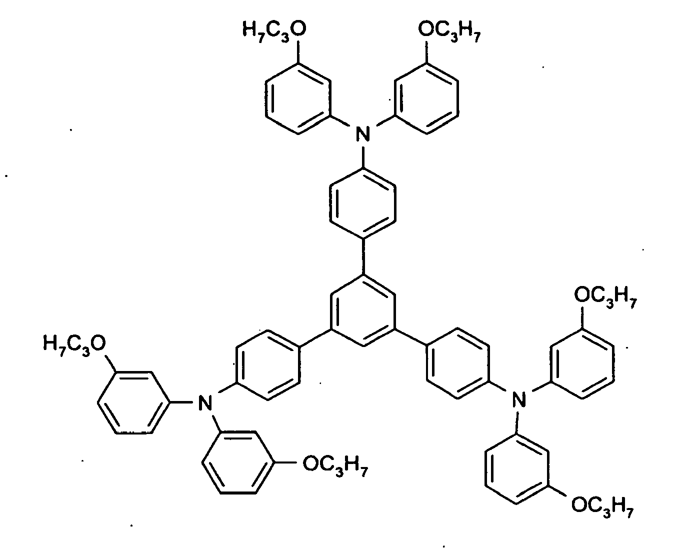

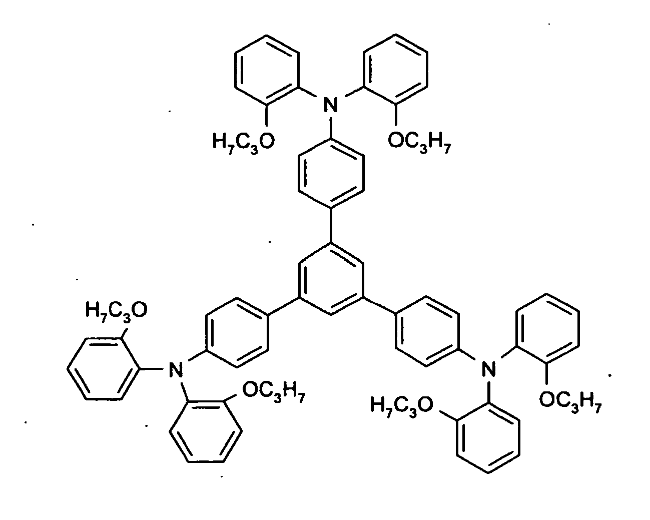

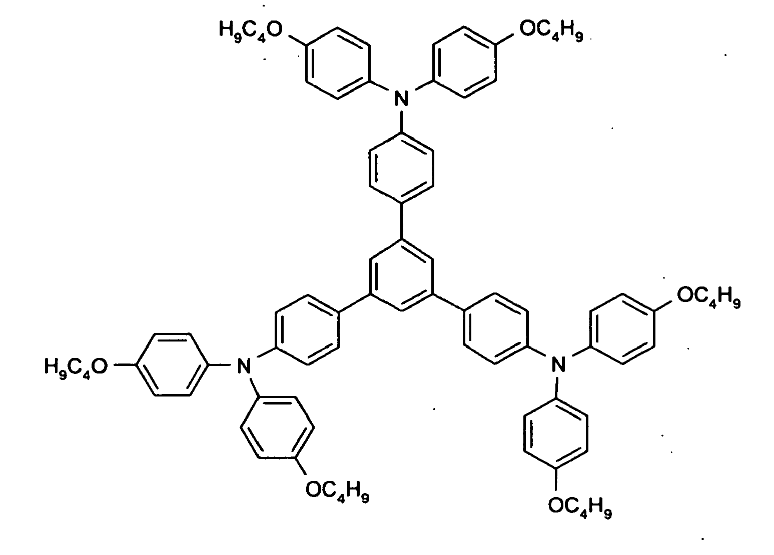

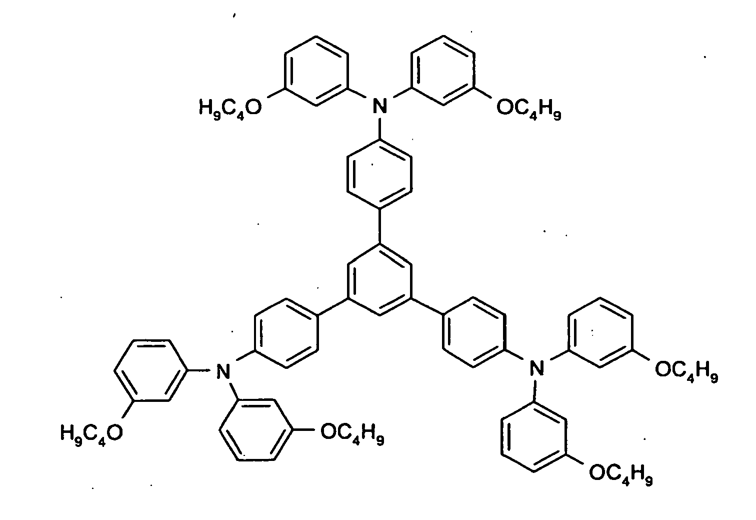

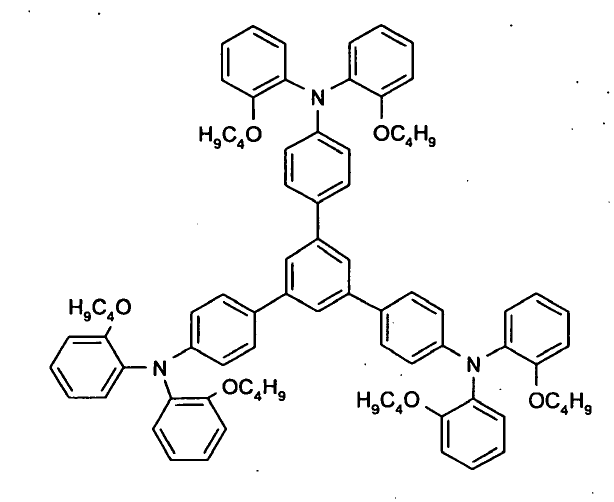

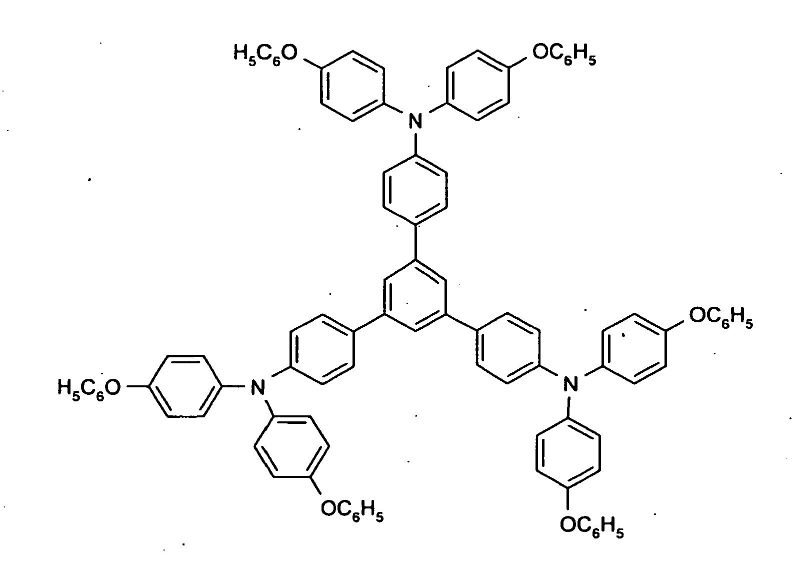

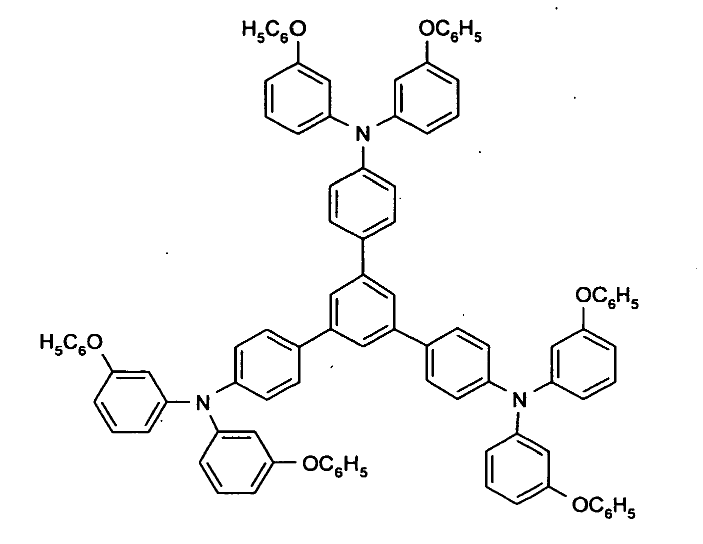

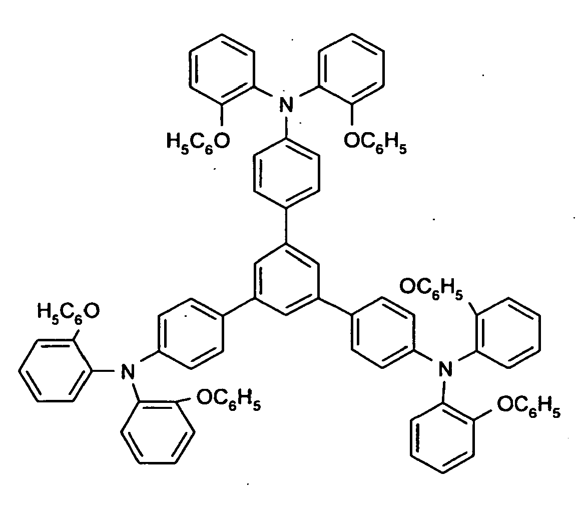

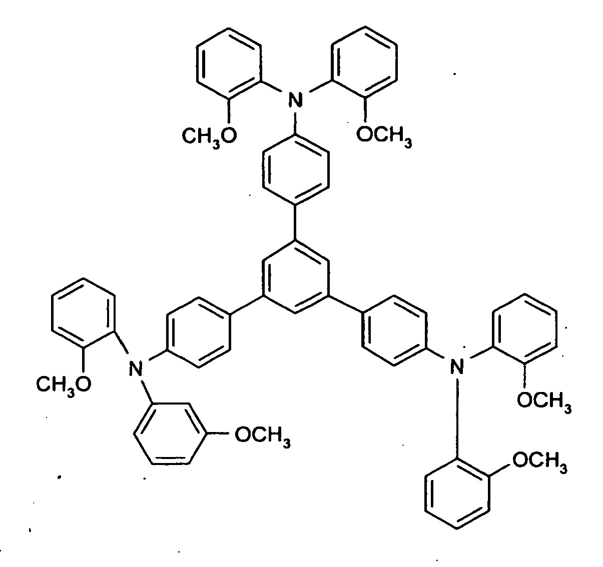

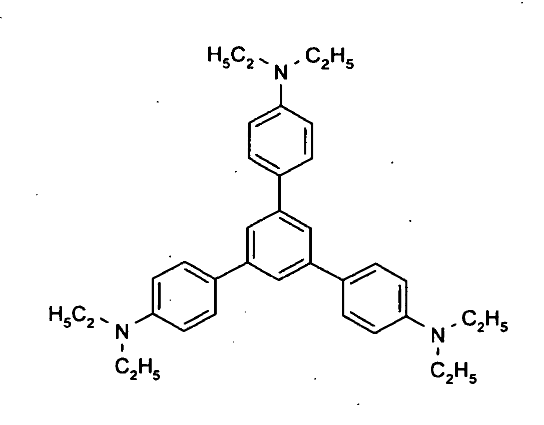

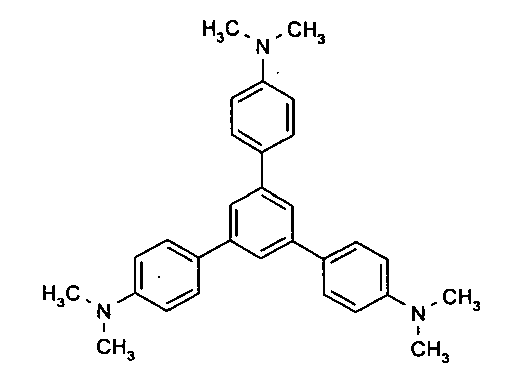

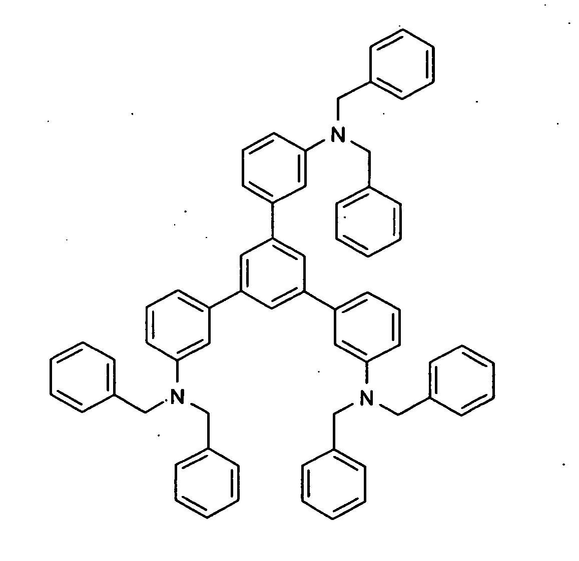

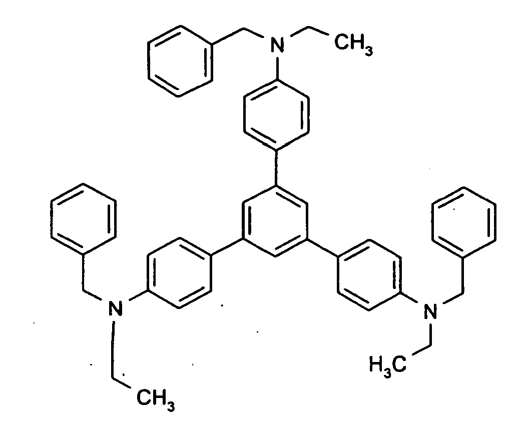

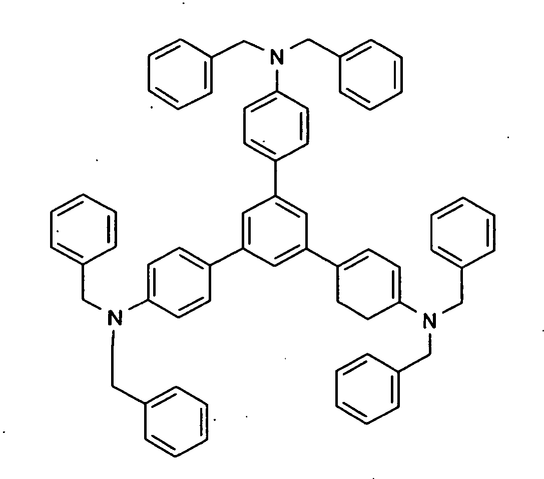

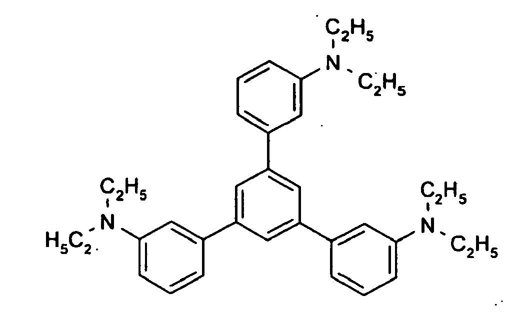

5. Elektrolumineszierende Anordnungen gemäß Anspruch 1, **dadurch gekennzeichnet dass** weitere Lochleiter, die von der aromatischen Aminverbindung A verschieden sind, aromatische Aminverbindungen ausgewählt aus den allgemeinen Formeln 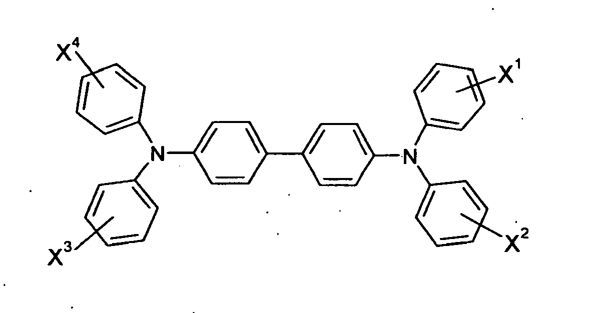 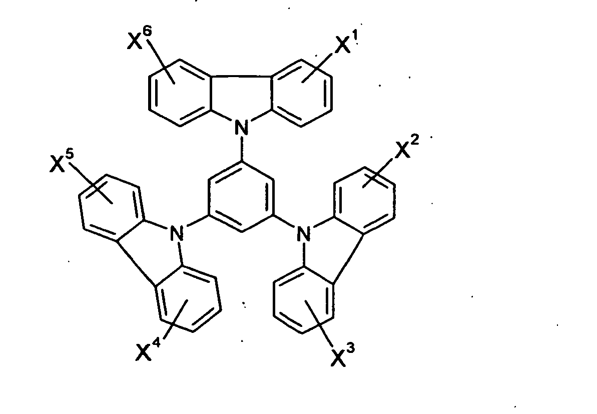 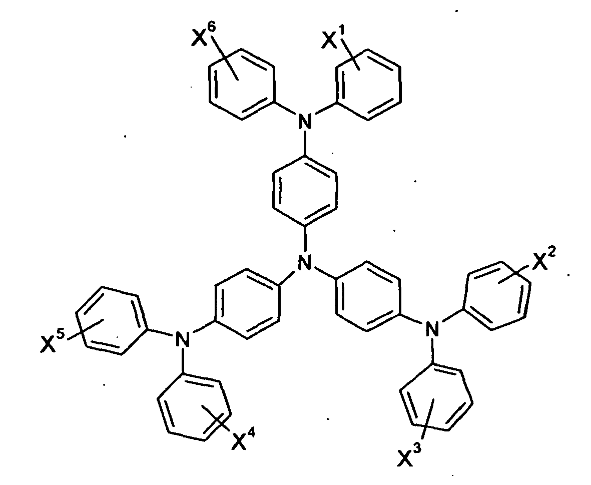 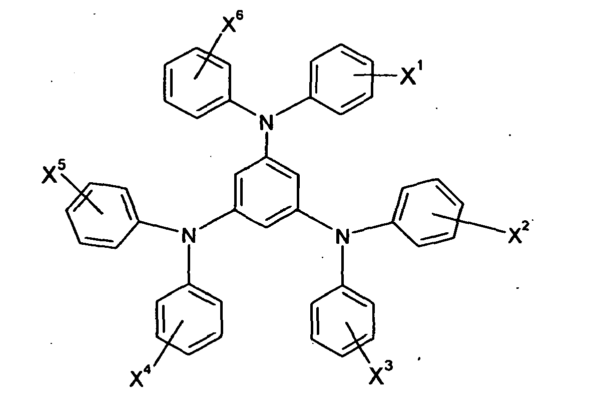 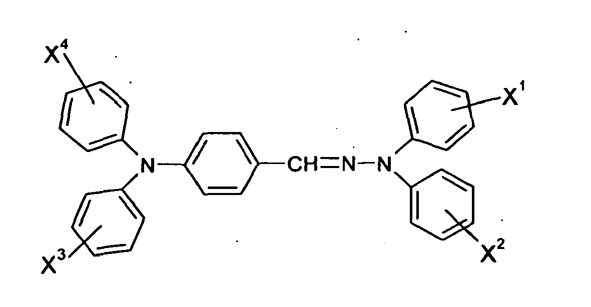 worin X¹ bis X⁶ unabhängig voneinander für H, Halogen, Alkyl, Aryl, Alkoxy, Aryloxy, vorzugsweise für Wasserstoff, Fluor, Chlor, Brom, (C₁-C₁₀)-, insbesondere für (C₁-C₄)-Alkyl oder -Alkoxy, Phenyl, Naphthyl, Phenoxy und Naphthyloxy stehen.

6. Elektrolumineszierende. Anordnungen gemäß Anspruch 5, **dadurch gekennzeichnet, dass** die weiteren Lochleiter ausgewählt werden aus folgenden Verbindungen:

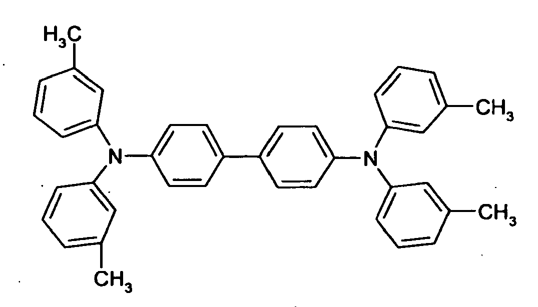

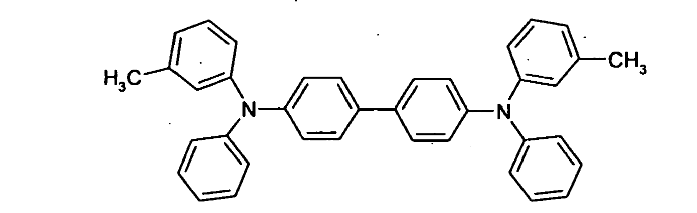

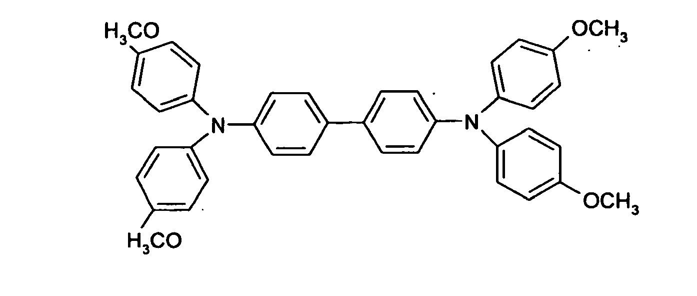

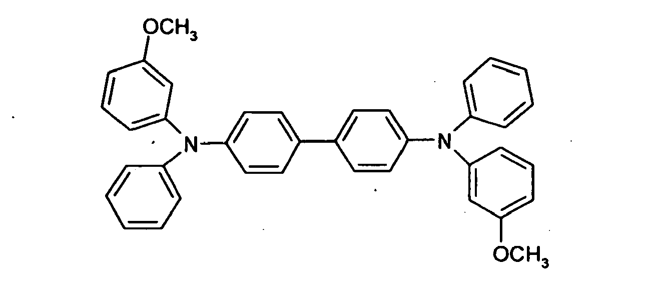

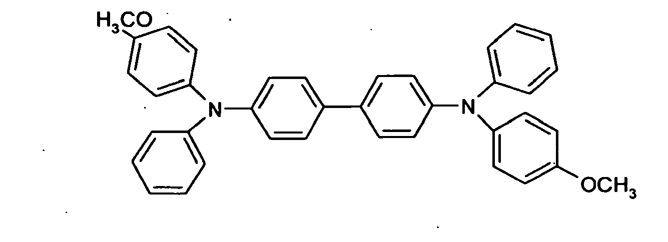

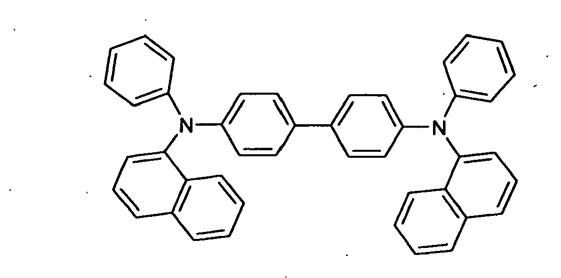

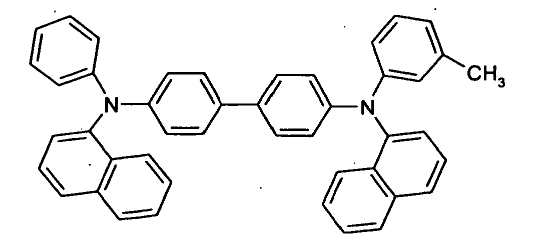

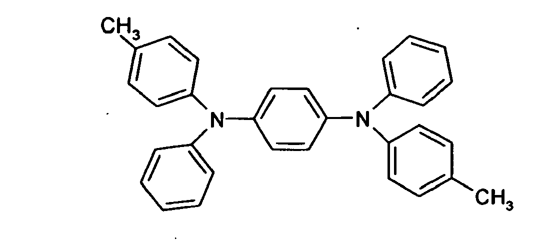

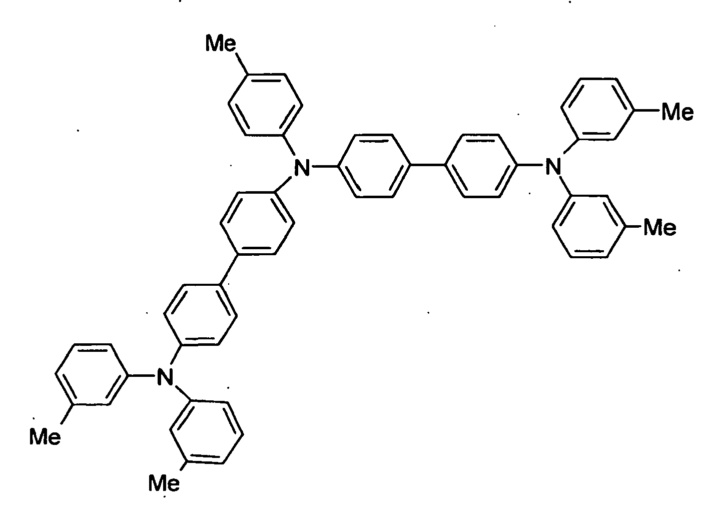

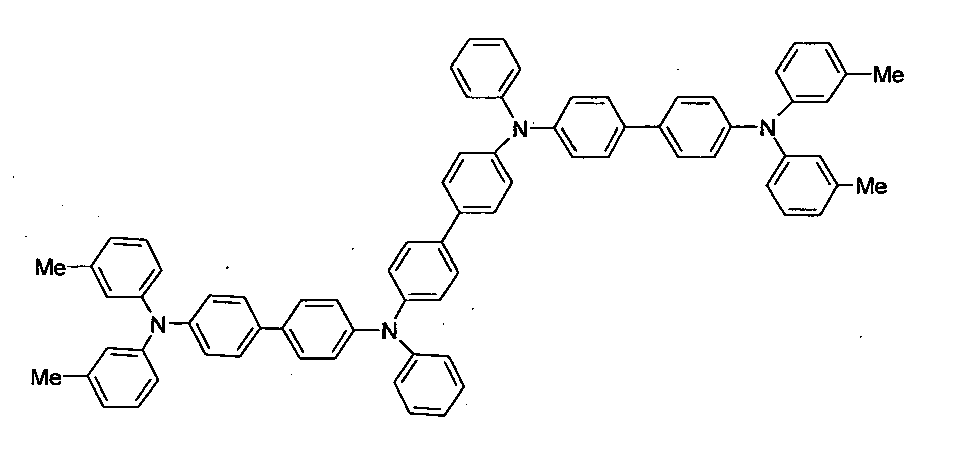

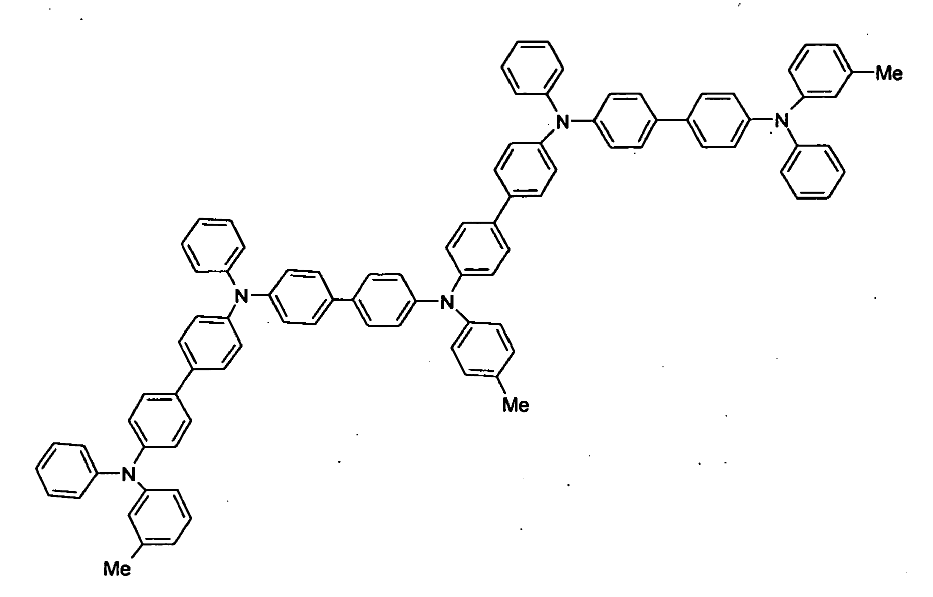

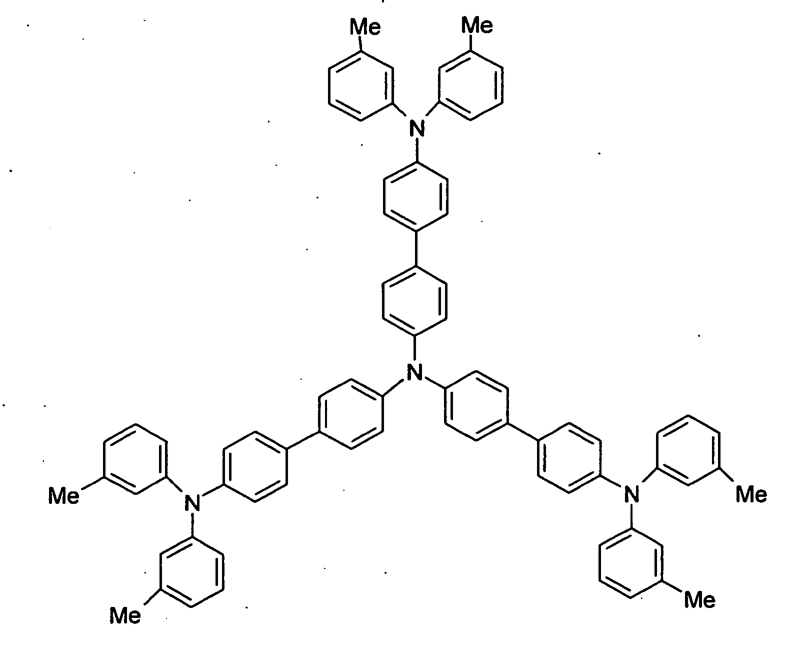

7. Elektrolumineszierende Anordnungen gemäß Anspruch 1, **dadurch gekennzeichnet, daß** Polyanionen, vorzugsweise die Anionen von polymeren Carbonsäuren und/oder polymeren Sulfonsäuren enthalten sind.

8. Elektrolumineszierende Anordnungen gemäß Anspruch 7, **dadurch gekennzeichnet, daß** Polystyrolsulfonsäure oder ein Erdalkalisalz davon als Polyanion enthalten ist.

9. Elektrolumineszierende Anordnungen gemäß Anspruch 1, **dadurch gekennzeichnet, daß** ein photolumineszierendes Material (Komponente C) enthalten ist.

10. Elektrolumineszierende Anordnungen gemäß Anspruch 1, **dadurch gekennzeichnet, daß** Substanzen die Photolumineszenz zeigen, Metallkomplexe, Chelate oder anorganische Nanoteilchen enthalten sind.

11. Elektrolumineszierende Anordnungen gemäß Anspruch 1, **dadurch gekennzeichnet, daß** mindestens eine Verbindung ausgewählt aus der Gruppe von Stilbene, Distilbene, Methinfarbstoffe, Cumarine, Naphthalimide, Perylene, Rubren, Chinacridone, Phenanthrene, Anthracene, Phthalocyamine, Metallkomplexe von ein-, zwei- oder dreiwertigen Metallen, welche Chelate bilden, anorganische Nanoteilchen enthält.

12. Elektrolumineszierende Anordnungen gemäß Anspruch 11, **dadurch gekennzeichnet, daß** das Metall ausgewählt ist aus der Gruppe von Lithium, Natrium, Kalium, Magnesium, Calcium, Bor, Aluminium, Gallium, Indium, Seltende Erden und die anorganischen Nanoteilchen ausgewählt sind aus der Gruppe von CdS, CdSe, ZnS oder ZnO.

13. Elektrolumineszierende Anordnungen gemäß Anspruch 1, **dadurch gekennzeichnet, daß** ein Oxin-Komplex (8-Hydroxychinolin-Komplex) von Al³⁺, Mg²⁺, In³⁺, Ga³⁺, Zn²⁺, Be²⁺, Li⁺, Ca²⁺, Na⁺ oder Aluminiumtris(5-methyloxin), Galliumtris(5-chloro-chinolin) oder Seltenenerd-Metallen enthalten ist.

14. Elektrolumineszierende Anordnungen gemäß Anspruch 1, **dadurch gekennzeichnet, daß** ein Metallkomplex ausgewählt aus
Alq₃ q = 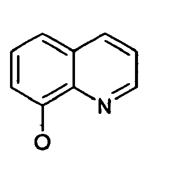 Inq₃, Gaq₃, Znq₂, Beq₂, Mgq₂,
oder Al(qa)₃, Ga(qa)₃, In(qa)₃, Zn(qa)₂, Be(qa)₂, Mg(qa)₂ enthalten ist, wobei
(qa) für 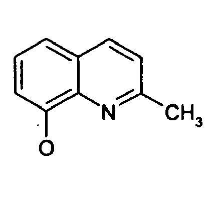 steht.

## Revendications

1. Dispositifs électroluminescents, contenant un substrat, une anode, un élément électroluminescent et une cathode, au moins une des deux électrodes étant transparente ou semi-transparente dans la plage spectrale visible et l'élément électroluminescent contenant une zone d'injection de trous, une zone de transport de trous, une zone électroluminescente et éventuellement une zone de transport d'électrons et/ou une zone d'injection d'électrons dans l'ordre indiqué, chacune des zones présentes pouvant également adopter la fonction des autres zones, **caractérisés en ce que** la zone d'injection de trous contient un polythiophène neutre ou cationique des formules (Ia) et/ou (Ib), 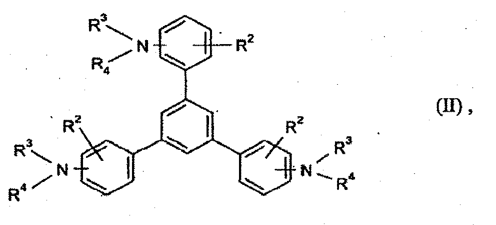 dans laquelle
R² représente l'hydrogène, un alkyle éventuellement substitué ou un halogène,
R³ et R⁴ représentent indépendamment l'un de l'autre un alkyle en C₁-C₁₀ éventuellement substitué, un alkyle en C₁-C₁₀ substitué par un alcoxycarbonyle, un aryle, un aralkyle ou un cycloalkyle, à chaque fois éventuellement substitué.

2. Dispositifs électroluminescents selon la revendication 1, **caractérisés en ce qu'**ils contiennent un ou plusieurs liants polymères transparents B.

3. Dispositifs électroluminescents selon la revendication 1, **caractérisés en ce que** dans la formule (II)
R³ et R⁴ représentent indépendamment l'un de l'autre un alkyle en C₁-C₆, un (C₁-C₄)-alcoxy-carbonyl-(C₁-C₆)-alkyle, un phényl-(C₁-C₄)-alkyle, naphtyl-(C₁-C₄)-alkyle, cyclopentyle, cyclohexyle, phényle, naphtyle ou anthracyle, à chaque fois éventuellement substitué par un alkyle en C₁-C₄ et/ou un alcoxy en C₁-C₉ et
R² représente l'hydrogène, un alkyle en C₁-C₆ ou un halogène.

4. Dispositifs électroluminescents selon la revendication 3, **caractérisés en ce que** le composé aromatique d'amine A est choisi parmi les composés 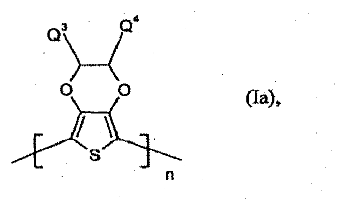 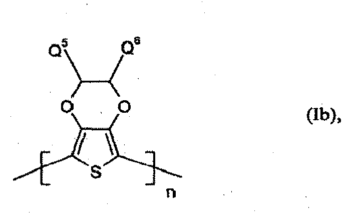 dans lesquelles
Q³ et Q⁴ représentent indépendamment l'un de l'autre l' hydrogène, un alkyle en C₁-C₁₈, un alcényle en C₂-C₁₂, un cycloalkyle en C₃-C₇, un aralkyle en C₇-C₁₅, un aryle en C₆-C₁₀, un alcoxy en C₁-C₁₈ ou un alkyloxyester en C₂-C₁₈, éventuellement substitué, et
Q⁵ et Q⁶ représentent indépendamment l'un de l'autre l'hydrogène, toutefois pas les deux en même temps, un alkyle en C₁-C₁₈, un alcényle en C₂-C₁₂, un cycloalkyle en C₃-C₇, un aralkyle en C₇-C₁₅, un aryle en C₆-C₁₀, un alcoxy en C₁-C₁₈ ou un alkyloxyester en C₂-C₁₈, à chaque fois substitué par au moins un groupe sulfonate,
n représente un nombre entier de 2 à 10 000,
et la couche conductrice de trous adjacente à la couche d'injection de trous contient plusieurs composés aromatiques d'amine, au moins un composé d'amine A choisi à partir de la formule générale (II) étant contenu en tant que composé aromatique d'amine suivants :

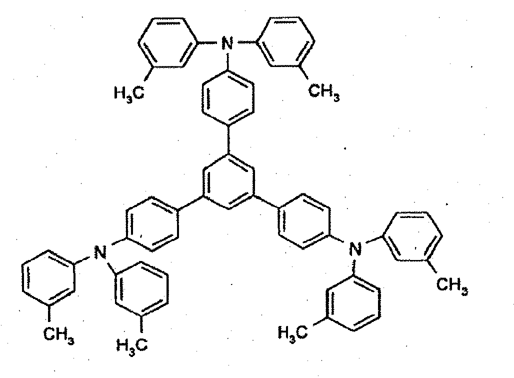

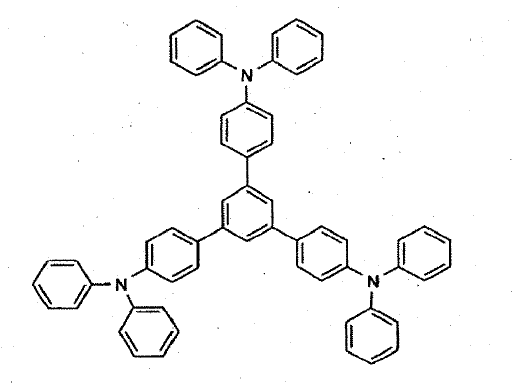

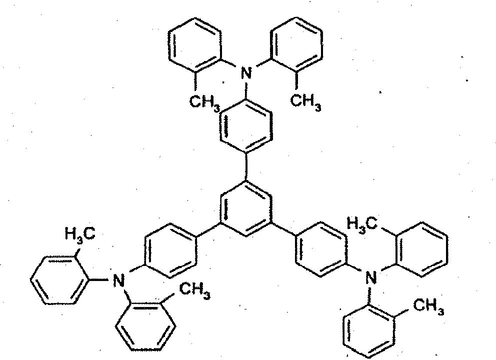

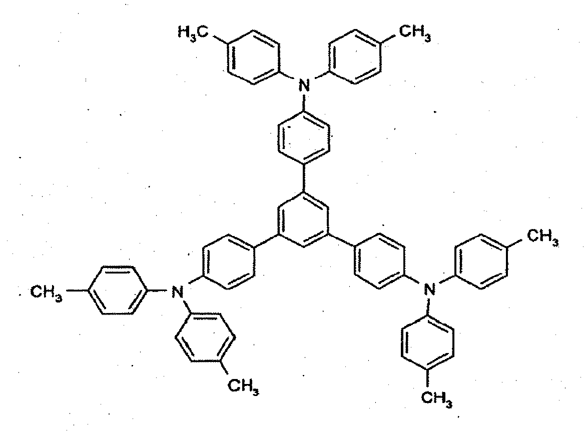

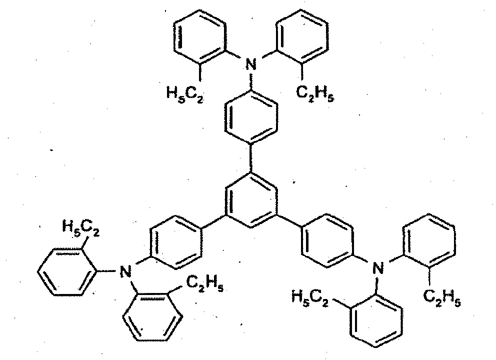

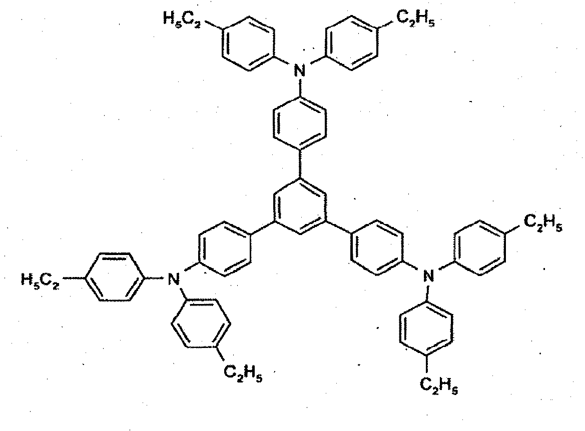

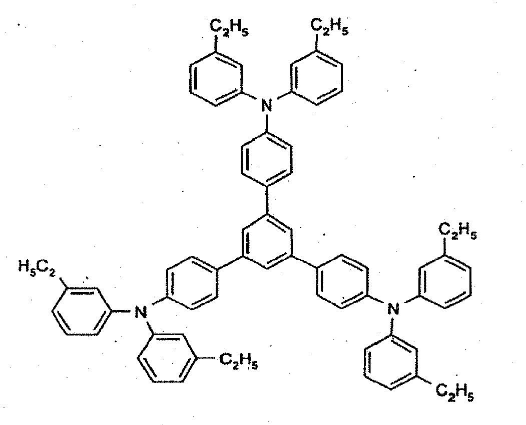

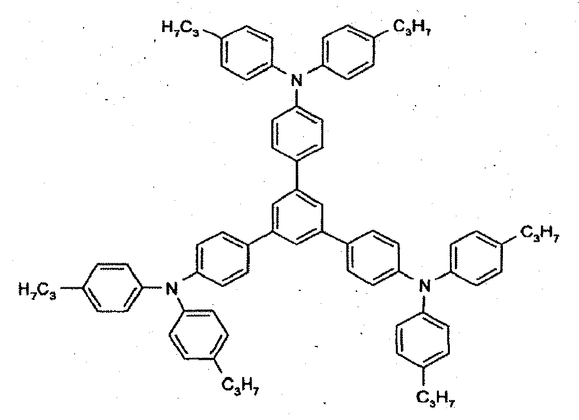

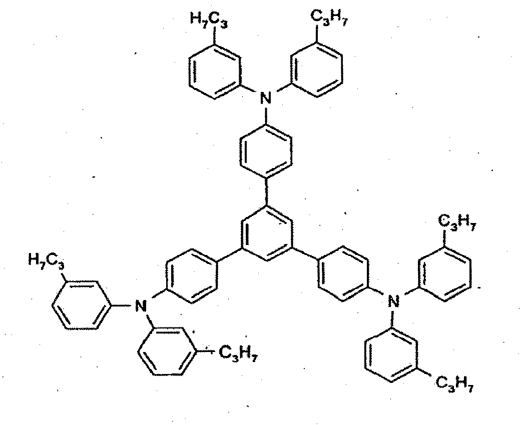

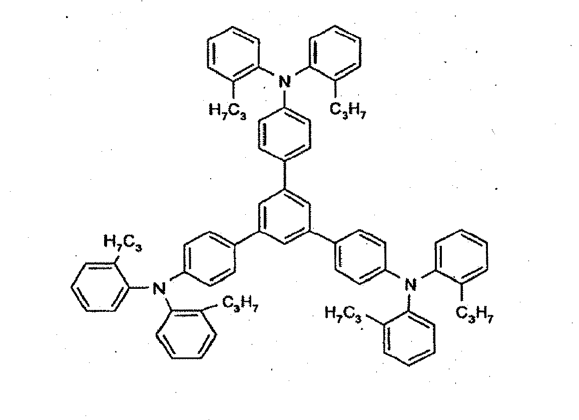

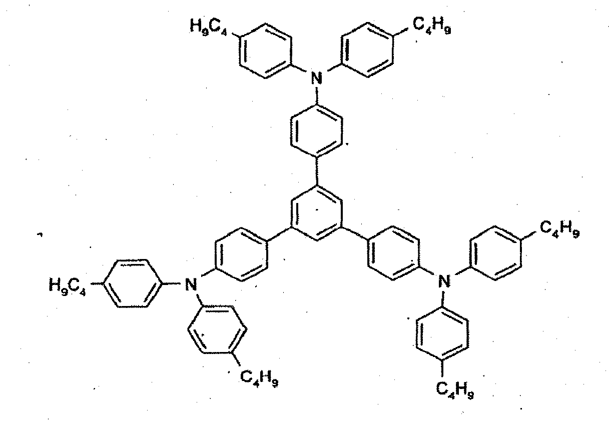

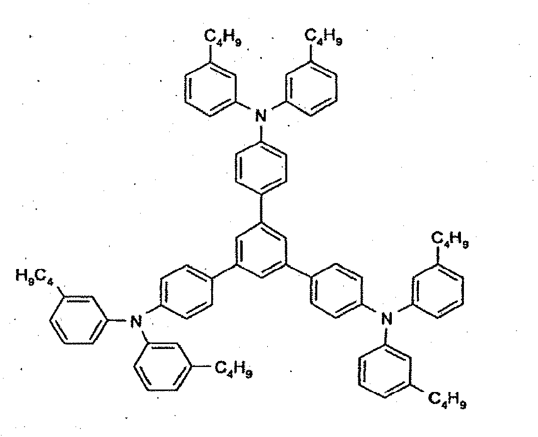

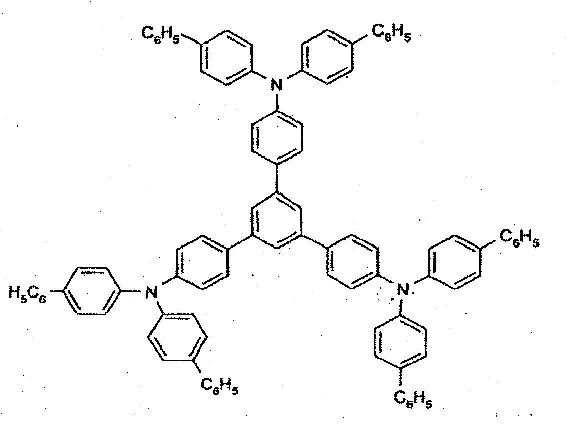

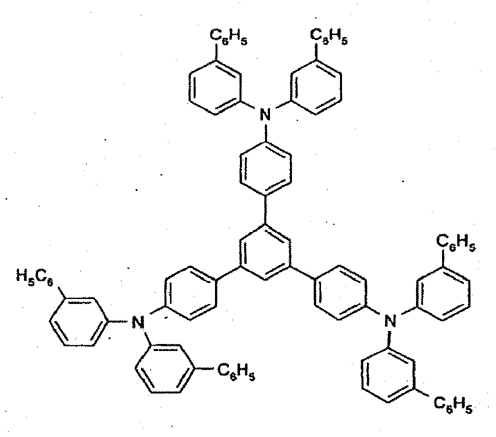

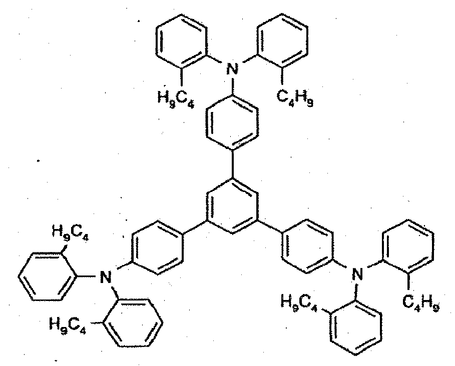

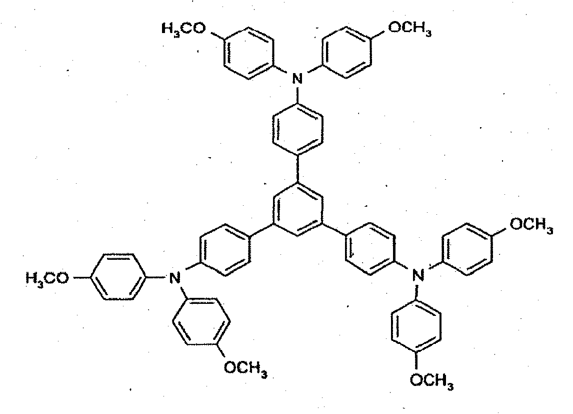

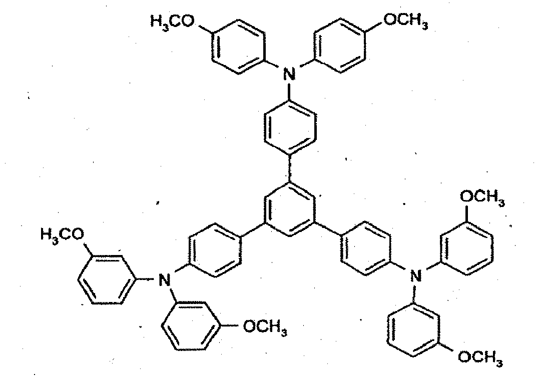

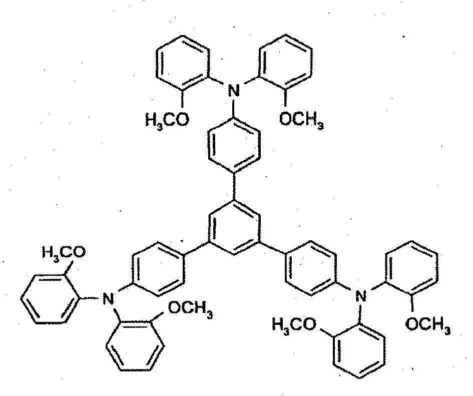

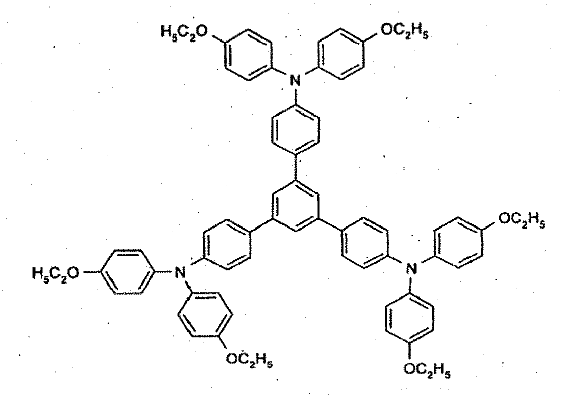

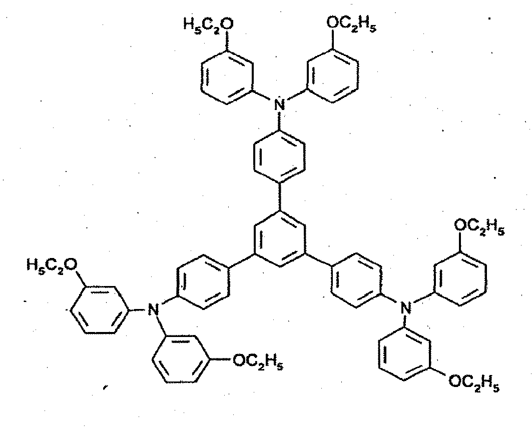

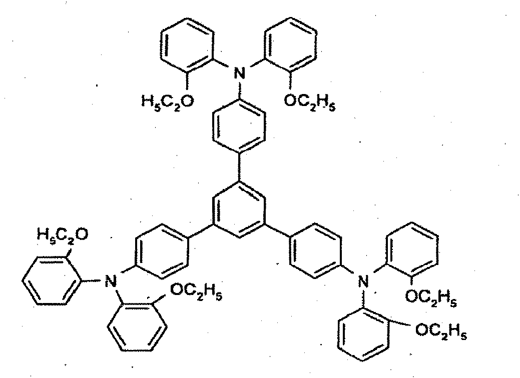

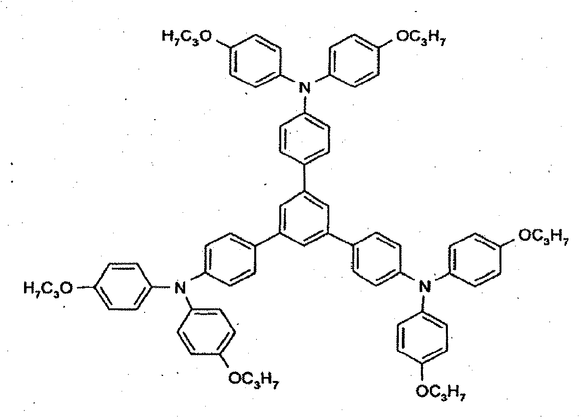

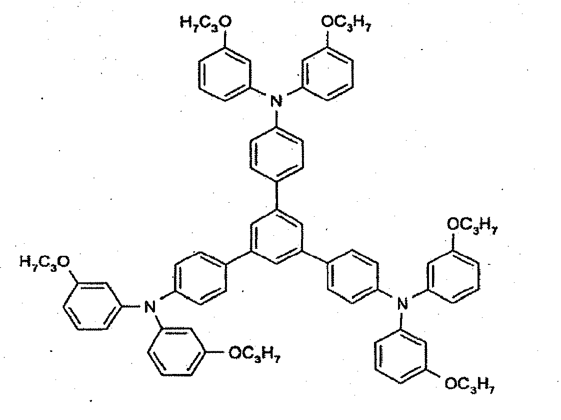

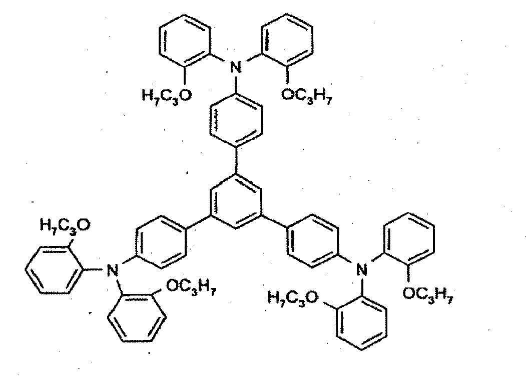

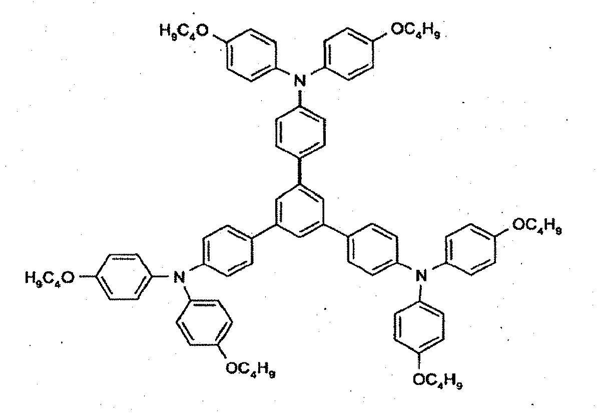

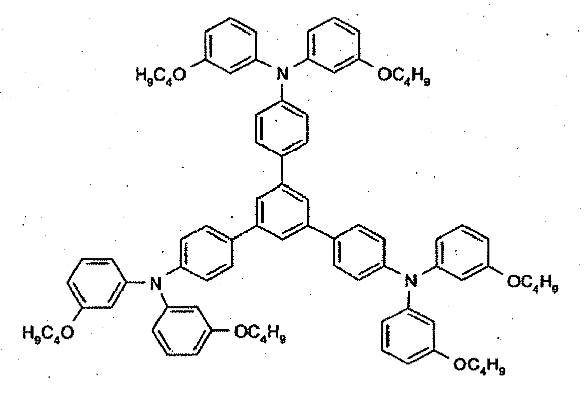

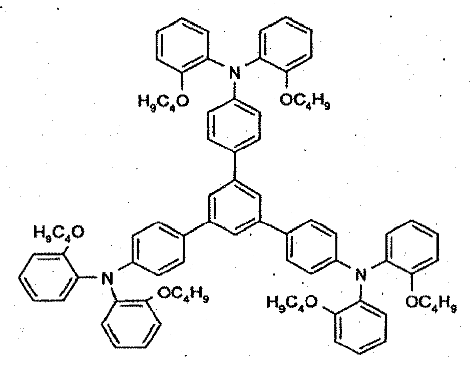

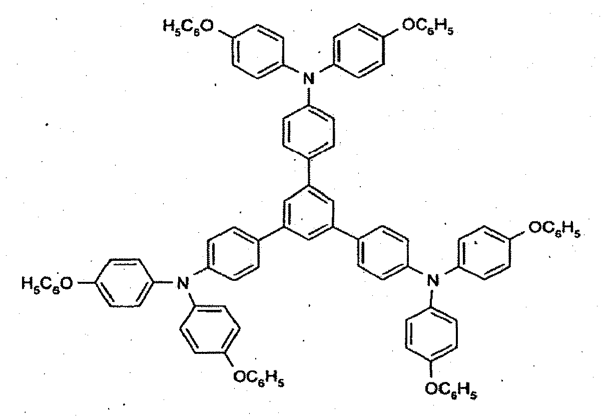

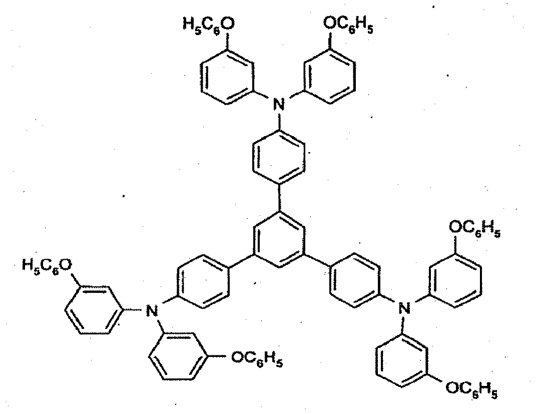

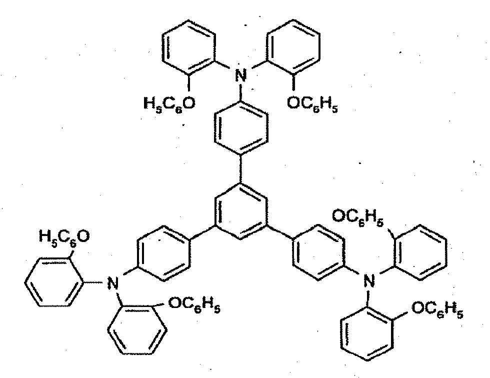

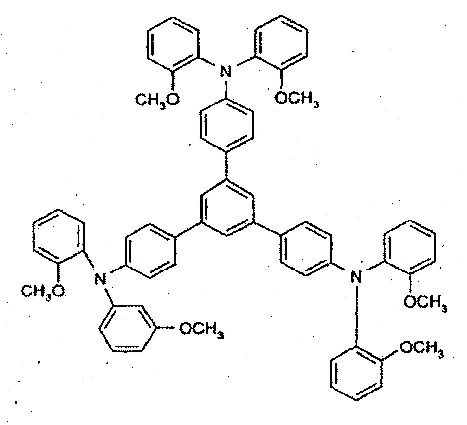

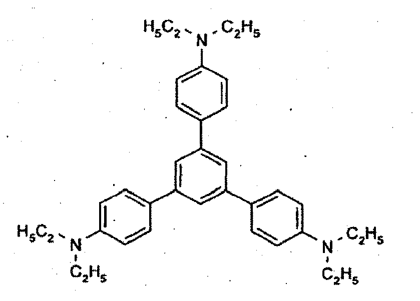

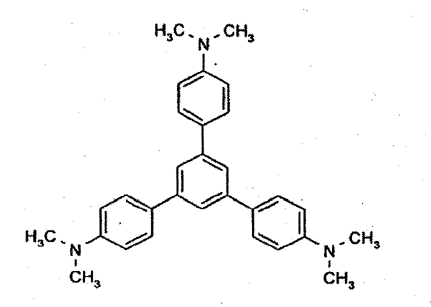

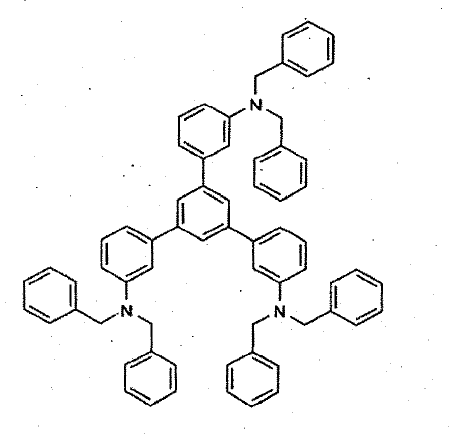

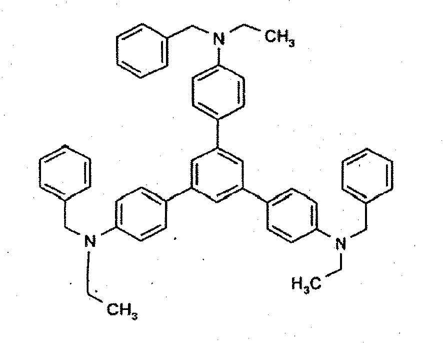

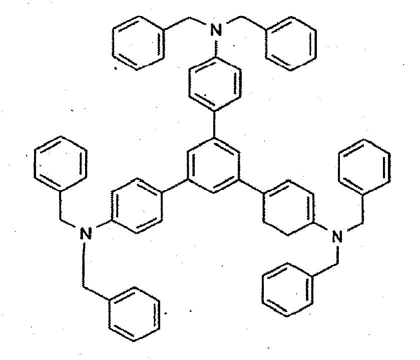

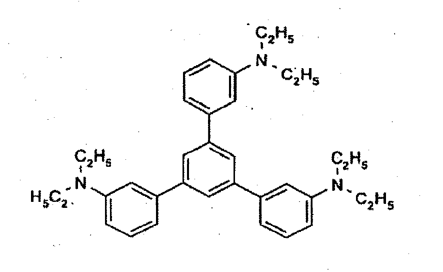

5. Dispositifs électroluminescents selon la revendication 1, **caractérisés en ce qu'**ils contiennent des conducteurs de trous supplémentaires, différents du composé aromatique d'amine A, qui sont des composés aromatiques d'amine choisis parmi les formules générales 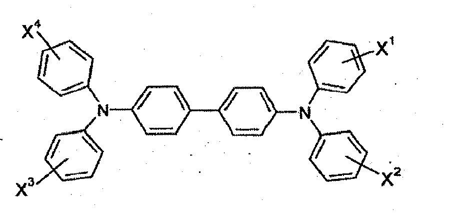 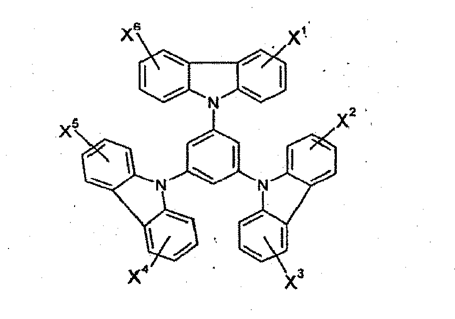 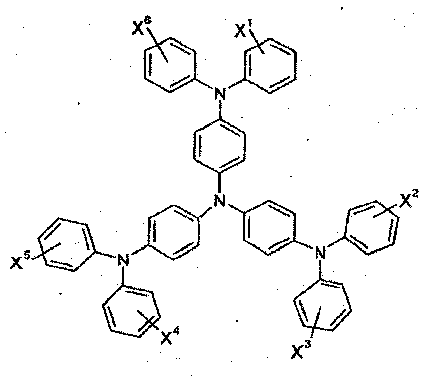 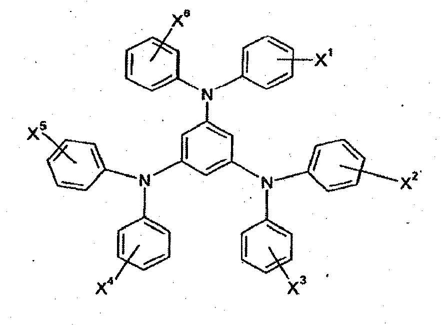 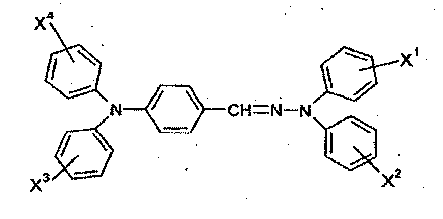 dans lesquelles X¹ à X⁶ représentent indépendamment les uns des autres H, halogène, allyle, aryle, alcoxy, aryloxy, de préférence hydrogène, fluor, chlore, brome, alkyle ou alcoxy en C₁-C₁₀, notamment en C₁-C₄, phényle, naphtyle, phénoxy et naphtyloxy.

6. Dispositifs électroluminescents selon la revendication 5, **caractérisés en ce que** les conducteurs de trous supplémentaires sont choisis parmi les composés suivants :

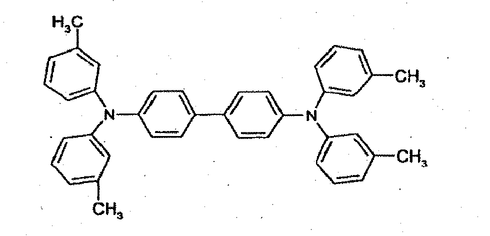

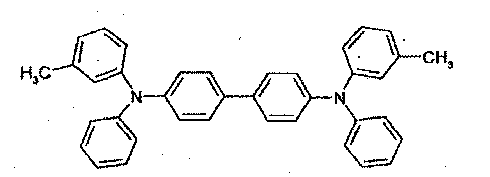

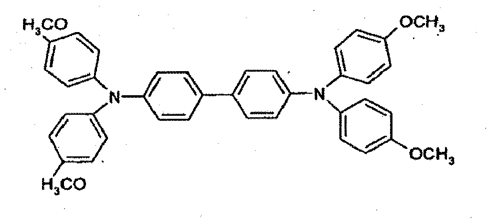

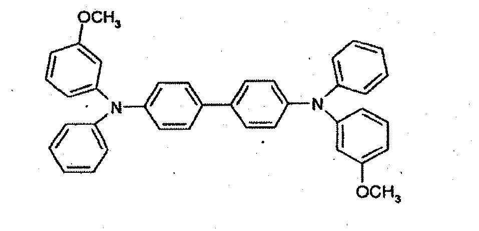

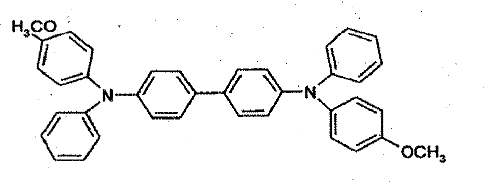

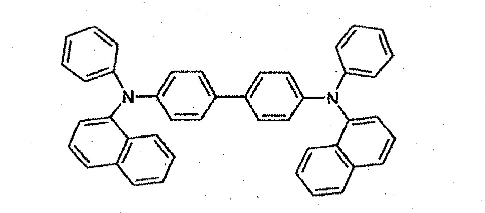

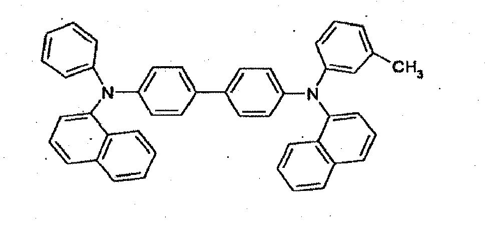

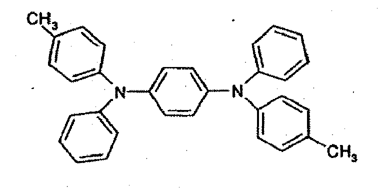

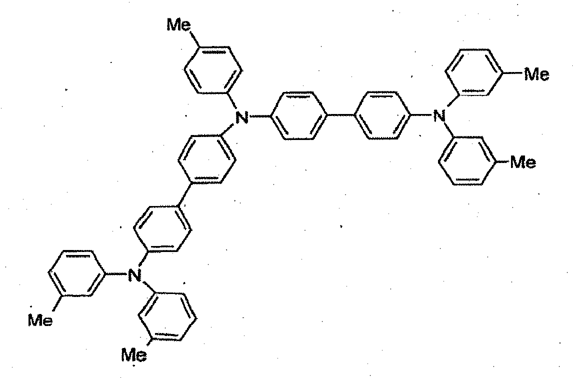

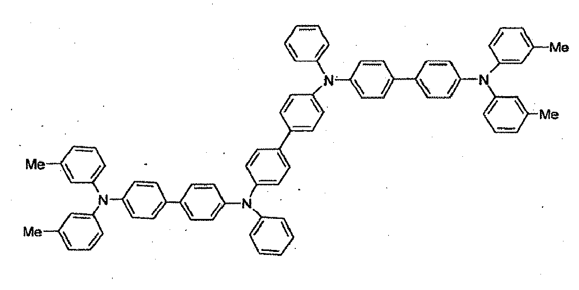

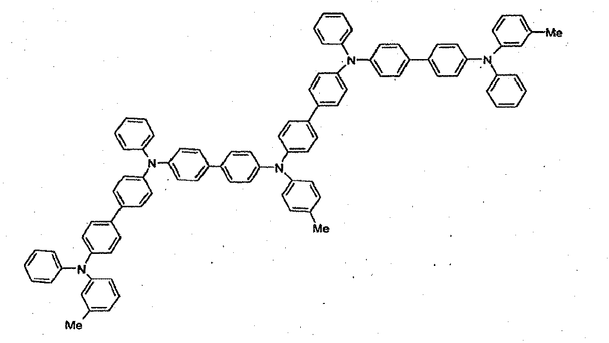

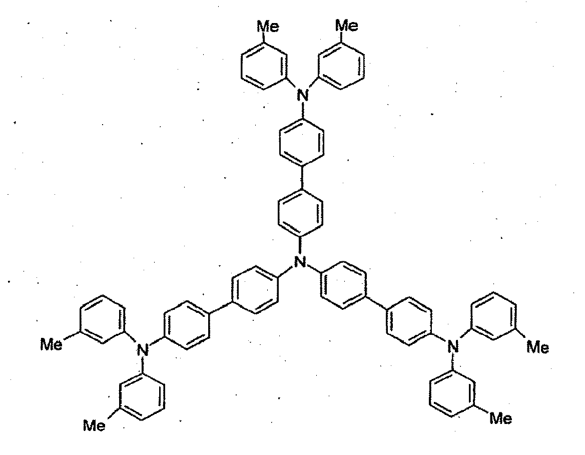

7. Dispositifs électroluminescents selon la revendication 1, **caractérisés en ce qu'**ils contiennent des polyanions, de préférence les anions d'acides carboxyliques polymères et/ou d'acides sulfoniques polymères.

8. Dispositifs électroluminescents selon la revendication 7, **caractérisés en ce qu'**ils contiennent de l'acide polystyrène-sulfonique ou un de ses sels alcalino-terreux en tant que polyanion.

9. Dispositifs électroluminescents selon la revendication 1, **caractérisés en ce qu'**ils contiennent un matériau photoluminescent (composant C).

10. Dispositifs électroluminescents selon la revendication 1, **caractérisés en ce qu'**ils contiennent des substances qui présentent une photoluminescence, des complexes métalliques, des chélates ou des nanoparticules inorganiques.

11. Dispositifs électroluminescents selon la revendication 1, **caractérisés en ce qu'**ils contiennent au moins un composé choisi dans le groupe des stilbènes, des distilbènes, des colorants méthine, des coumarines, des naphtalimides, des pérylènes, du rubrène, des quinacridones, des phénanthrènes, des anthracènes, des phtalocyamines, des complexes métalliques de métaux mono-, bi- ou trivalents, qui forment des chélates, des nanoparticules inorganique.

12. Dispositifs électroluminescents selon la revendication 11, **caractérisés en ce que** le métal est choisi dans le groupe du lithium, du sodium, du potassium, du magnésium, du calcium, du bore, de l'aluminium, du gallium, de l'indium, des terres rares et les nanoparticules inorganiques sont choisies dans le groupe de CdS, CdSe, ZnS ou ZnO.

13. Dispositifs électroluminescents selon la revendication 1, **caractérisés en ce qu'**ils contiennent un complexe d'oxine (complexe de 8-hydroxyquinoline) de Al³⁺, Mg²⁺, In³⁺, Ga³⁺, Zn²⁺, Be²⁺, Li⁺, Ca²⁺, Na⁺ ou d'aluminiumtris (5-méthyloxine), de galliumtris (5-chloro-quinoline) ou de métaux de terres rares.

14. Dispositifs électroluminescents selon la revendication 1, **caractérisés en ce qu'**ils Contiennent un complexe métallique choisi parmi
Alq₃ q = 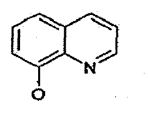 Inq₃, Gaq₃, Znq₂, Beq₂, Mgq₂,
ou Al(qa)₃, Ga(qa)₃, In(qa)₃, Zn(qa)₂, Be(qa)₂, Mg(qa)₂,
(qa) représentant

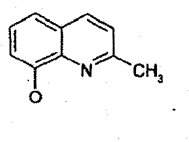

## Claims

1. Electroluminescent assemblies containing a substrate, an anode, an electroluminescent element and a cathode, where at least one of the two electrodes is transparent or semitransparent in the visible spectral region and the electroluminescent element contains, in the specified order, a hole injection zone, a hole transport zone, an electroluminescent zone, and optionally an electron transport zone and/or an electron injection zone, where each of the zones present may also take over the function of the other zones, **characterized in that** the hole injection zone contains an uncharged or cationic polythiophene of the formula (Ia) and/or (Ib), 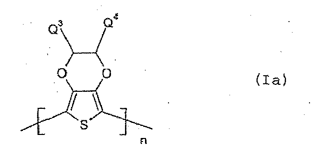 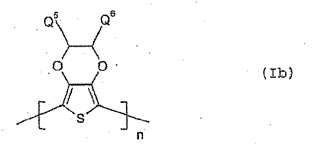 where
Q³ and Q⁴ represent, independently of one another, hydrogen, substituted or unsubstituted (C₁-C₁₈)-alkyl, (C₂-C₁₂) -alkenyl, (C₃-C₇) -cycloalkyl, (C₇-C₁₅)-aralkyl, (C₆-C₁₀) -aryl, (C₁-C₁₈) -alkoxy or (C₂-C₁₈) -alkyloxy ester and
Q⁵ and Q⁶ represent, independently of one another, hydrogen, but not both simultaneously, (C₁-C₁₈) -alkyl, (C₂-C₁₂)-alkenyl, (C₃-C₇) -cycloalkyl, (C₇-C₁₅) -aralkyl, (C₆-C₁₀) -aryl, (C₁-C₁₈) -alkoxy or (C₂-C₁₈) -alkyloxy ester substituted in each case by at least one sulphonate group,
n is an integer from 2 to 10 000,
and the hole conductor zone adjoining the hole injection zone contains a plurality of aromatic amine compounds, where at least one amine compound A selected from among compounds of the general formula (II) 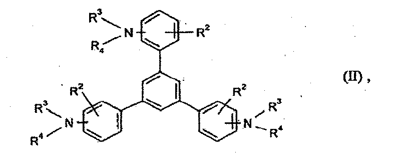 where
R² represents hydrogen, substituted or unsubstituted alkyl or halogen,
R³ and R⁴ represent, independently of one another, substituted or unsubstituted (C₁-C₁₀)-alkyl, alkoxycarbonyl-substituted (C₁-C₁₀)-alkyl, in each case substituted or unsubstituted aryl, aralkyl or cycloalkyl, is present as aromatic amine compound.

2. Electroluminescent assemblies according to Claim 1, **characterized in that** one or more transparent polymeric binders B are present.

3. Electroluminescent assemblies according to Claim 1, **characterized in that**, in formula (II),
R³ and R⁴ represent, independently of one another, (C₁-C₆)-alkyl, (C₁-C₄)-alkoxycarbonyl-(C₁-C₆)-alkyl, in each case unsubstituted or (C₁-C₄)-alkyl- and/or (C₁-C₄)-alkoxy-substituted phenyl-(C₁-C₄)-alkyl, naphthyl-(C₁-C₄)-alkyl, cyclopentyl, cyclohexyl, phenyl, naphthyl or anthracyl and
R² represents hydrogen, (C₁-C₆)-alkyl or halogen.

4. Electroluminescent assemblies according to Claim 3, **characterized in that** the aromatic amine compound A is selected from among the following compounds:

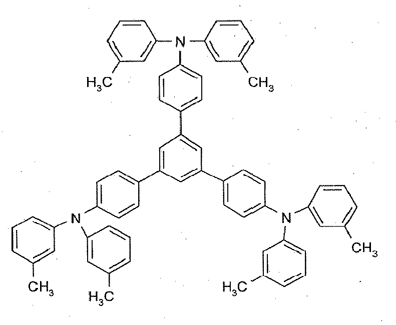

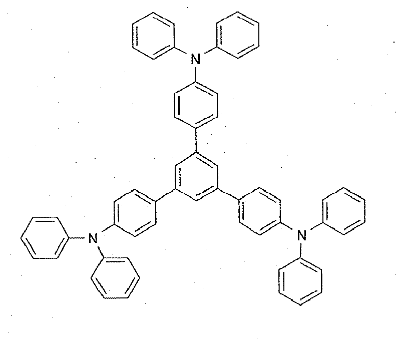

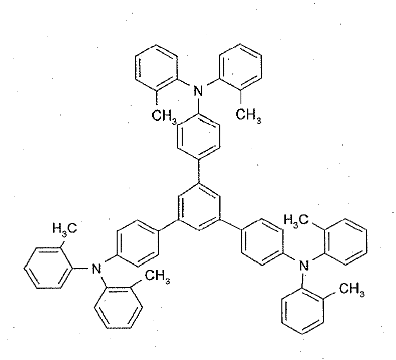

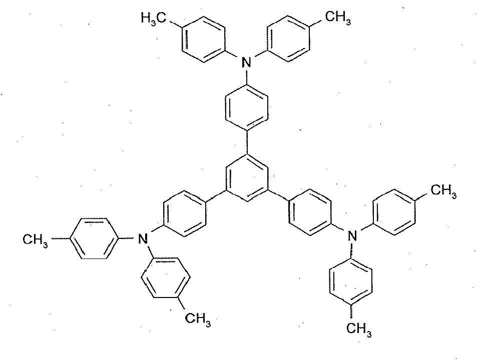

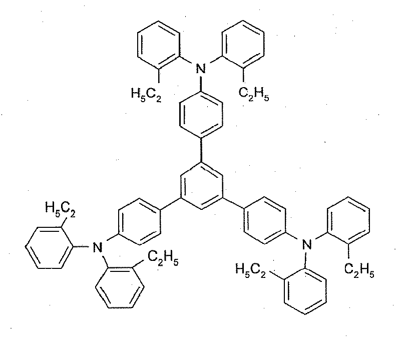

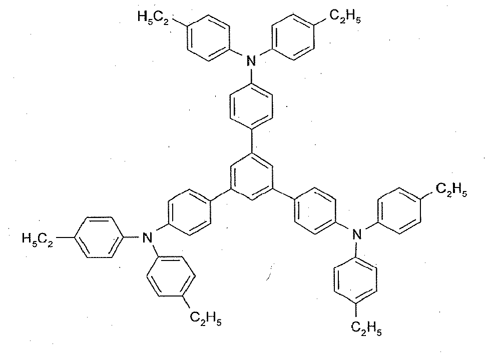

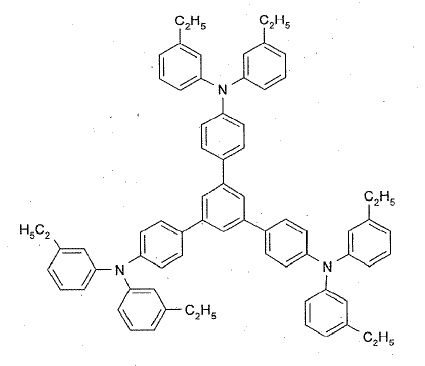

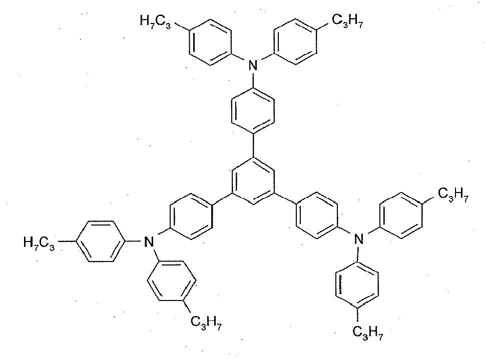

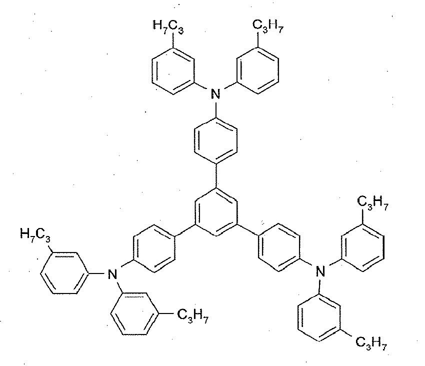

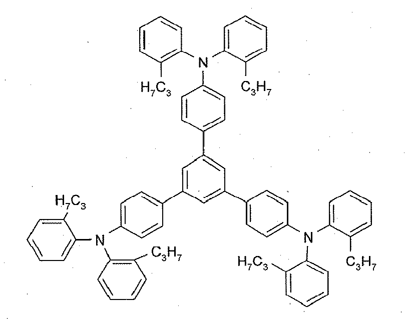

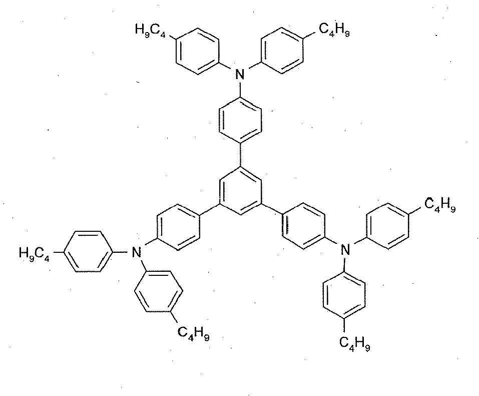

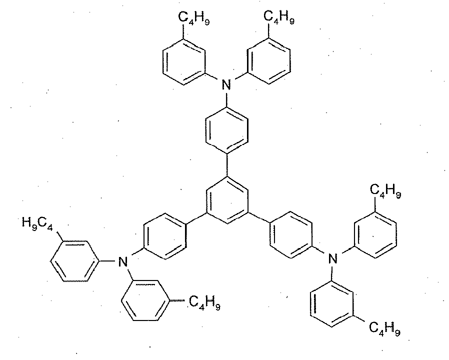

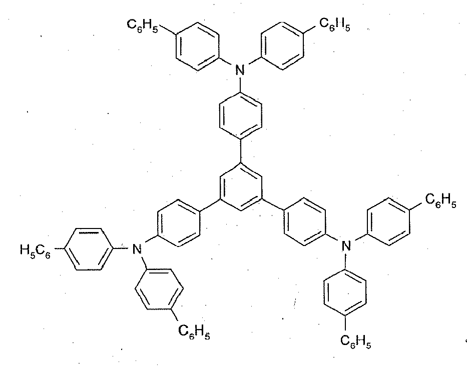

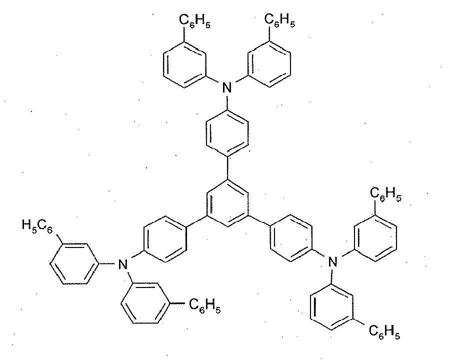

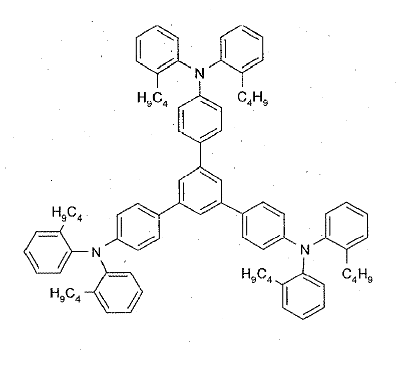

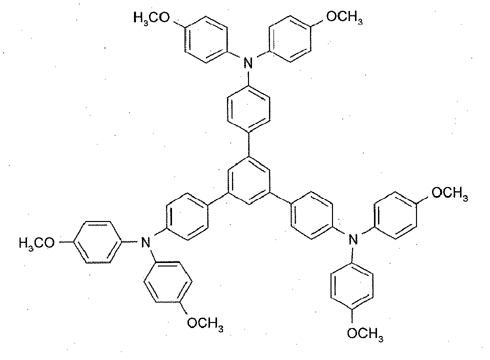

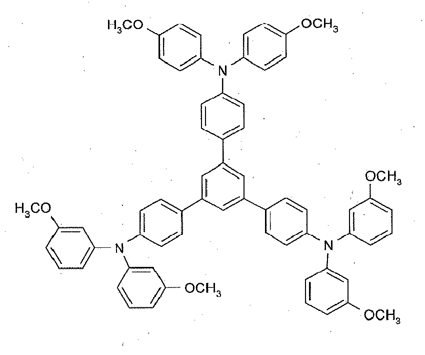

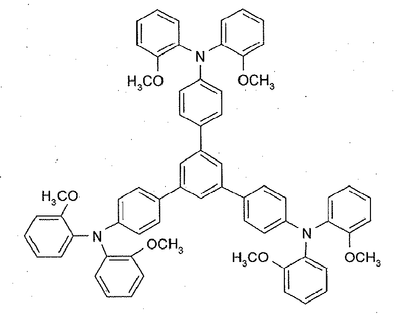

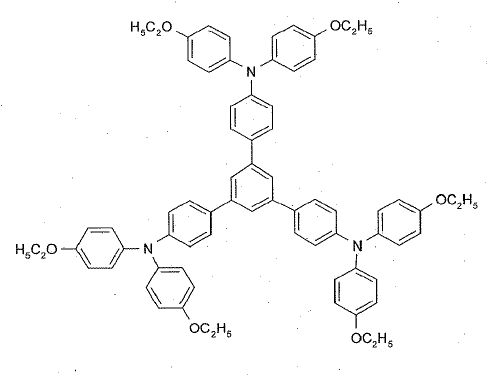

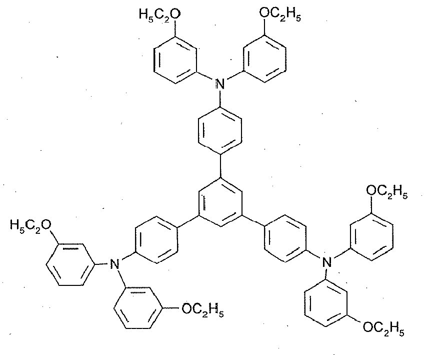

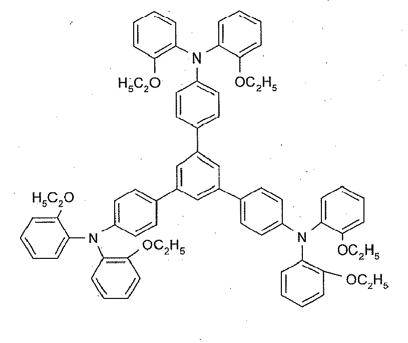

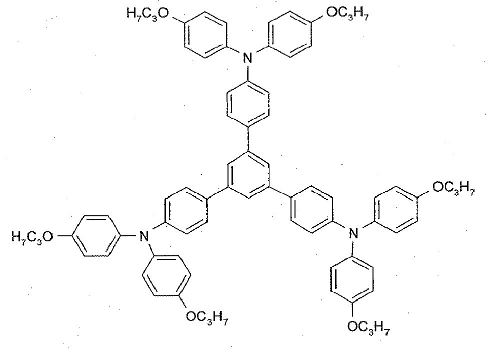

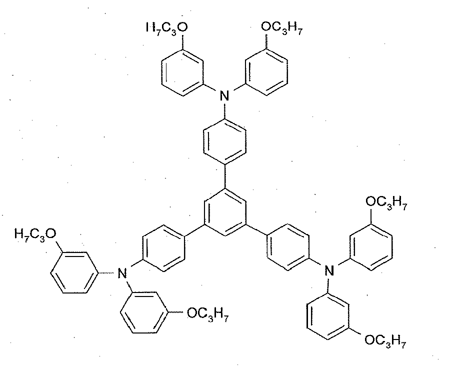

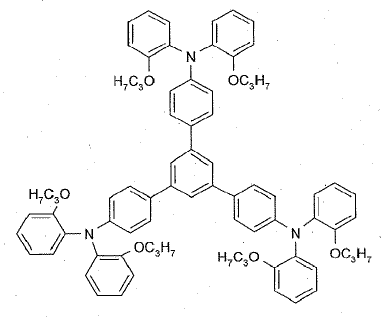

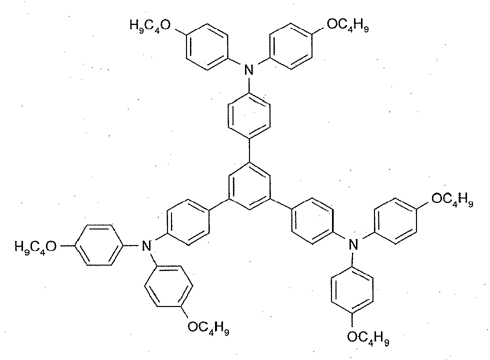

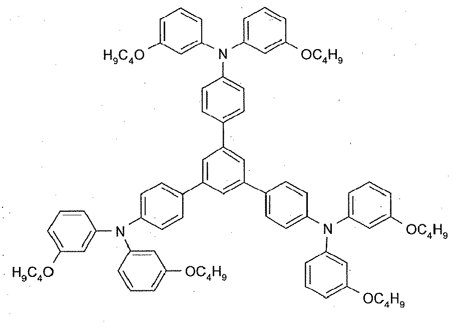

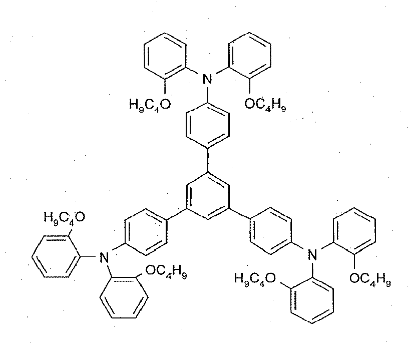

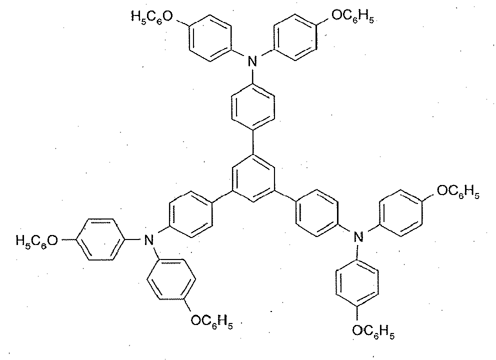

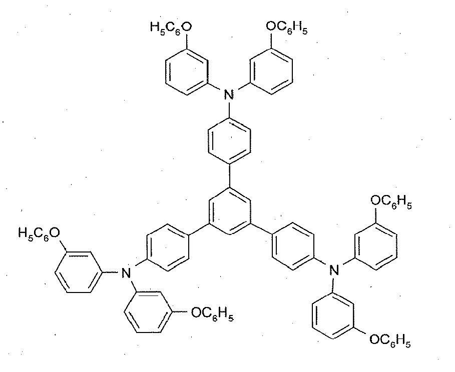

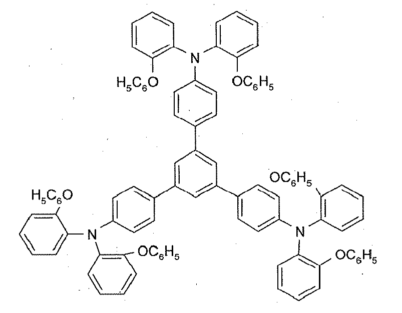

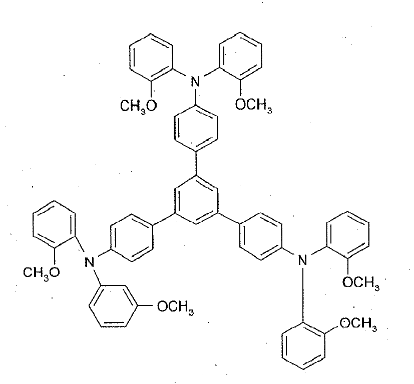

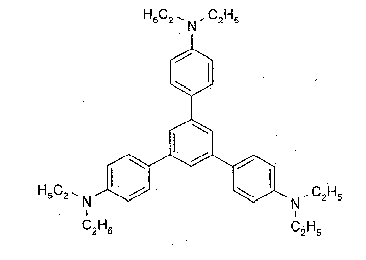

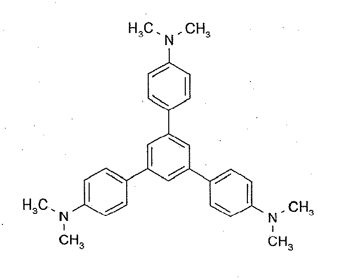

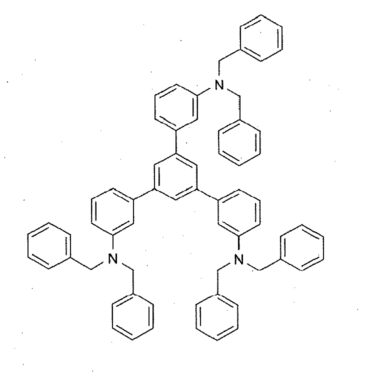

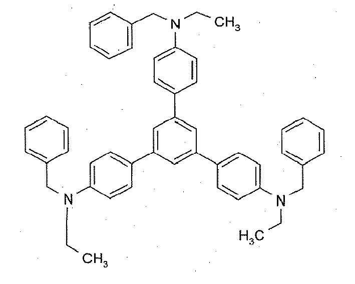

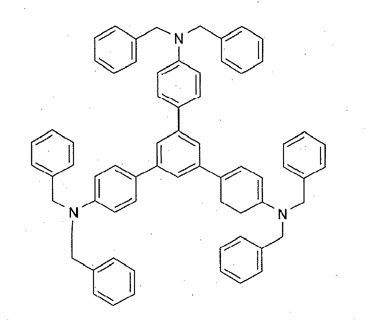

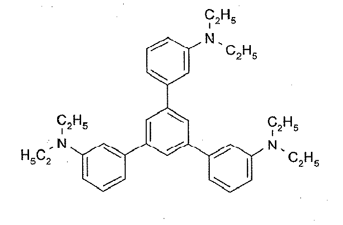

5. Electroluminescent assemblies according to Claim 1, **characterized in that** further hole conductors which are different from the aromatic amine compound A, aromatic amine compounds selected from among compounds of the general formulae 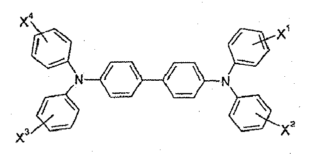 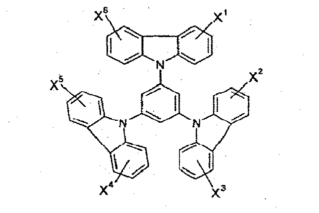 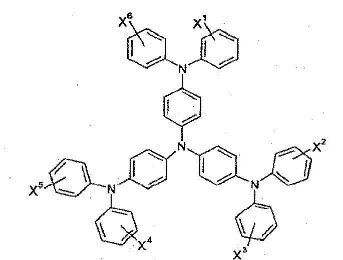 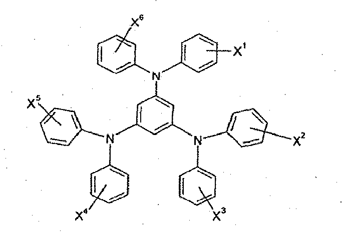 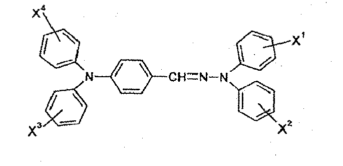 where X¹ to X⁶ represent, independently of one another, H, halogen, alkyl, aryl, alkoxy, aryloxy, preferably hydrogen, fluorine, chlorine, bromine, (C₁-C₁₀)-, in particular (C₁-C₄)-alkyl or -alkoxy, phenyl, naphthyl, phenoxy and naphthyloxy.

6. Electroluminescent assemblies according to Claim 5, **characterized in that** the further hole conductors are selected from among the following compounds:

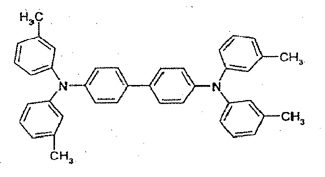

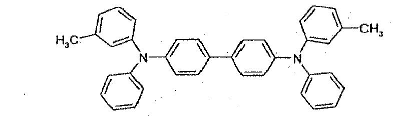

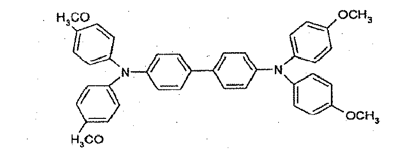

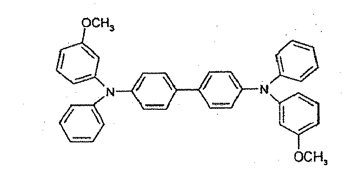

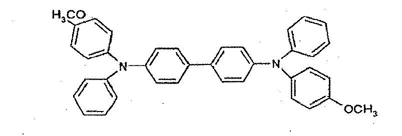

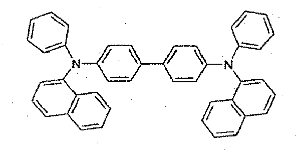

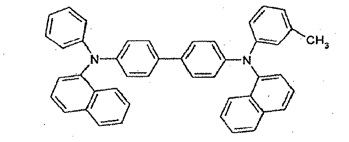

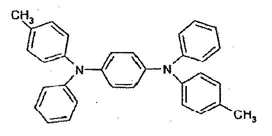

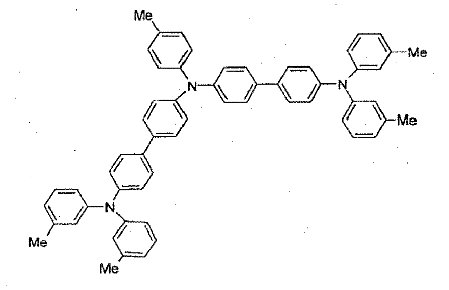

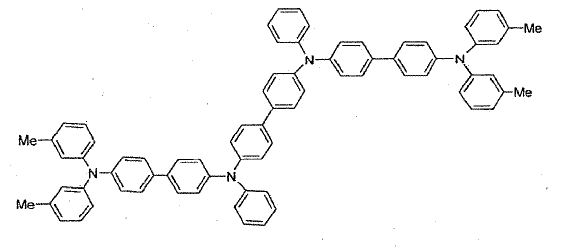

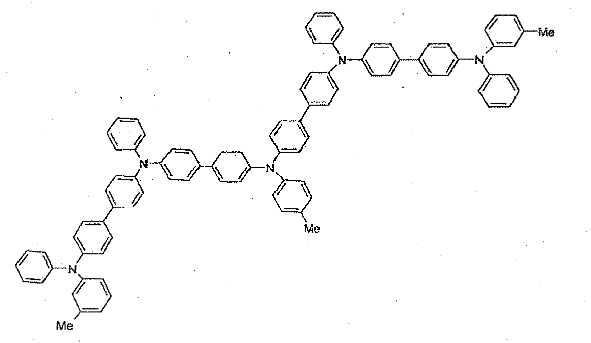

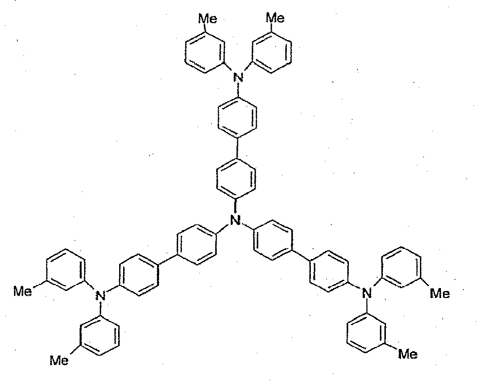

7. Electroluminescent assemblies according to Claim 1, **characterized in that** polyanions, preferably the anions of polymeric carboxylic acids and/or polymeric sulphonic acids, are present.

8. Electroluminescent assemblies according to Claim 7, **characterized in that** polystyrenesulphonic acid or an alkaline earth metal salt thereof is present as polyanion.

9. Electroluminescent assemblies according to Claim 1, **characterized in that** a photoluminescent material (component C) is present.

10. Electroluminescent assemblies according to Claim 1, **characterized in that** substances which display photoluminescence, metal complexes, chelates or inorganic nanosize particles are present.

11. Electroluminescent assemblies according to Claim 1, **characterized in that** at least one compound selected from the group consisting of stilbenes, distilbenes, methine dyes, coumarins, naphthalimides, perylenes, rubrene, quinacridones, phenanthrenes, anthracenes, phthalocyanines, metal complexes of monovalent, divalent or trivalent metals which form chelates and inorganic nanosize particles is present.

12. Electroluminescent assemblies according to Claim 11, **characterized in that** the metal is selected from the group consisting of lithium, sodium, potassium, magnesium, calcium, boron, aluminum, gallium, indium, rare earths and the inorganic nanosize particles are selected from the group consisting of CdS, CdSe, ZnS and ZnO.

13. Electroluminescent assemblies according to Claim 1, **characterized in that** an oxine complex (8-hydroxyquinoline complex) of Al³⁺, Mg²⁺, In³⁺, Ga³⁺, Zn²⁺, Be²⁺, Li⁺, Ca²⁺, Na⁺ or aluminium tris (5-methyloxine), gallium tris(5-chloroquinoline) or rare earth metals is present.

14. Electroluminescent assemblies according to Claim 1, **characterized in that** a metal complex selected from among
Alq₃ q = 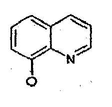 Inq₃, Gaq₃, Znq₂, Beq₂, Mgq₂,
and Al (qa)₃, Ga (qa)₃, In (qa)₃, Zn (qa)₂, Be (qa)₂, Mg (qa)₂, where
(qa) represents 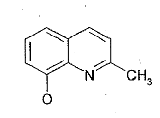 is present.
